# EUROPEAN PATENT APPLICATION

(11) **EP 3 708 565 A1**
(43) Date of publication of application: **16.09.2020**
(21) Application number: 20161066.4
(22) Date of filing: 04.03.2020
(51) Int. Cl.: C07D 239/34, A01N 43/54, C07D 239/52, C07D 239/56

(54) **PYRIMIDINYLOXYPHENYLAMIDINES AND THE USE THEREOF AS FUNGICIDES**

(71) Applicant: Bayer AG, 51373 Leverkusen (DE)
(72) Inventor: Winter, Philipp, 40627 Düsseldorf (DE); Montagne, Cyril, 69009 Lyon (FR); Es-Sayed, Mazen, 40764 Langenfeld (DE); Thomas, Vicent, 69003 Lyon (FR)
(74) Representative: BIP Patents

(57) **Abstract**

The present invention relates to compounds of the formula (I), in particular to pyrimidinyloxyphenylamidines of the formula (I), to the use of pyrimidinyloxyphenylamidines of the formula (I) according to the invention for controlling unwanted microorganisms, in particular phytopathogenic fungi and also to a composition for this purpose, comprising the pyrimidinyloxyphenylamidines of the formula (I) according to the invention. Furthermore, the invention relates to a method for controlling unwanted microorganisms, in particular phytopathogenic fungi, characterized in that the compounds of the formula (I) are applied to the microorganisms, in particular to the phytopathogenic fungi and/or in their habitat.

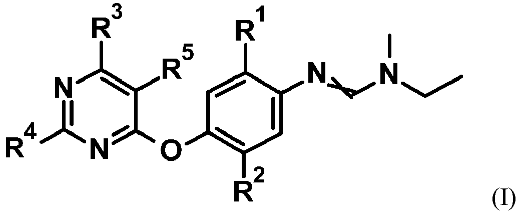

## Description

The present invention relates to compounds of the formula (I), in particular to pyrimidinyloxyphenylamidines of the formula (I), to the use of pyrimidinyloxyphenylamidines of the formula (I) according to the invention for controlling unwanted microorganisms, in particular phytopathogenic fungi and also to a composition for this purpose, comprising the pyrimidinyloxyphenylamidines of the formula (I) according to the invention. Furthermore, the invention relates to a method for controlling unwanted microorganisms, in particular phytopathogenic fungi, characterized in that the compounds of the formula (I) are applied to the microorganisms, in particular to the phytopathogenic fungi and/or in their habitat.

WO2000/046184 discloses the use of amidines, including N-methyl-N-methyl-N'-[(4-phenoxy)-2,5-xylyl]-formamidine, as fungicides.

WO2003/093224, WO2007/031512, WO2007/031513, WO2007/031523, WO2007/031524, WO2007/031526, WO2007/031527, WO2007/061966, WO2008/101682, WO2008/110279, WO2008/110280, WO2008/110281, WO2008/110312, WO2008/110313, WO2008/110314, WO2008/110315, WO2008/128639, WO2009/156098, WO2009/156074, WO2010/086118, WO2012/025450, WO2012/090969, WO2014/157596, WO2016/202688, WO2016/202742, WO2017/005710, WO2018/108992, WO2018/108998 disclose the use of arylamidine derivatives as fungicides.

WO2007/031508 and WO2007/093227 disclose the use of arylamidine derivatives as fungicides and insecticides.

WO2003/024219 discloses fungicide compositions comprising at least one N2-phenylamidine derivative in combination with a further selected known active compound.

WO2004/037239 discloses antifungicidal medicaments based on N2-phenylamidine derivatives.

WO2005/089547, WO2005/120234, WO2012/146125, WO2013/136275, WO2014/037314, and WO2018/108977 disclose fungicide mixtures comprising at least one arylamidine derivative and a further selected known fungicide.

WO2007/031507 discloses fungicide mixtures comprising at least one arylamidine derivative and two other selected known fungicides.

The effectiveness of the phenylamidines described in the prior art as fungicides is good but in many cases the spectrum of action for example in view of the fungicidal efficacy and/or the used application rate needs to be improved. In particular, the fungicidal efficacy needs to be improved.

Accordingly, it is an object of the present invention to provide phenylamidines having an improved fungicidal efficacy. Good fungicidal efficacy and a broad spectrum of action with respect to the phytopathogenic fungi to be controlled was observed for the inventive compounds of formula (I), i.e. inventive compounds of formula (I) act as fungicides.

Accordingly, the present invention provides pyrimidinyloxyphenylamidines of the formula (I) 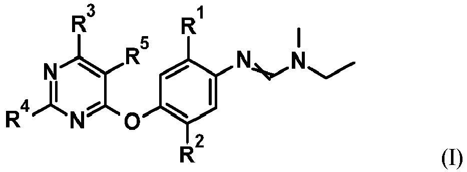 in which
- R¹ and R²: are independently selected from the group consisting of
cyano, halogen, -Si(R⁶)(R⁷)(R⁸), and
C₁-C₈-Alkyl, C₁-C₈-Alkoxy, C₃-C₈-Cycloalkyl, C₁-C₈-Haloalkyl, C₂-C₈-Alkenyl, C₂-C₈-Alkynyl, C₁-C₈-Alkylsulfanyl, C₁-C₈-Alkylsulfinyl, C₁-C₈-Alkylsulfonyl, -C(O)-C₁-C₈-Alkyl, -C(O)-C₃-C₈-Cycloalkyl, -C(O)NH-C₁-C₈-Alkyl, -C(O)N-di-C₁-C₈-Alkyl, -C(O)O-C₁-C₈-Alkyl, -NH-C₁-C₈-Alkyl, -N-di-C₁-C₈-Alkyl, each of which may be independently non-substituted or substituted with one or more substituent(s) selected from the group consisting of C₁-C₈-Alkoxy and halogen,
- R³ and R⁴: are independently selected from the group consisting of hydrogen,
C₁-C₈-Alkyl, C₂-C₈-Alkenyl, C₁-C₈-Alkoxy, C₃-C₈-Cycloalkyl, C₃-C₈-Cycloalkoxy, each of which may be independently non-substituted or substituted with one or more substituent(s) selected from the group consisting of C₁-C₈-Alkyl, C₁-C₈-Haloalkyl, C₁-C₈-Alkoxy, C₆-C₁₄-Aryl, halogen, and -Si(R⁶)(R⁷)(R⁸), and
-S(O)ₙ-R'
wherein
n represents 0, 1, or 2, and
R' is selected from the group consisting of C₁-C₈-Alkyl, C₃-C₈-Cycloalkyl, C₃-C₈-Cycloalkyl-C₁-C₈-Alkyl, C₃-C₈-Cycloalkoxy, C₆-C₁₄-Aryl, Aryl-C₁-C₈-Alkyl, Heteroaryl, each of which may be independently non-substituted or substituted with one or more substituent(s) selected from the group consisting of C₁-C₈-Alkyl, C₁-C₈-Haloalkyl, C₁-C₈-Alkoxy, C₆-C₁₄-Aryl, halogen, and-Si(R⁶)(R⁷)(R⁸), and
- R⁵: is selected from the group consisting of hydrogen, halogen, cyano, C₁-C₈-Alkyl, C₂-C₈-Alkenyl, C₂-C₈-Alkynyl, C₃-C₆-Cycloalkyl, C₃-C₆-Cycloalkoxy, C₁-C₈-Alkylsulfanyl, C₁-C₈-Alkylsulfinyl, C₁-C₈-Alkylsulfonyl, and wherein
- R⁶, R⁷ and R⁸: are independently from each other selected from C₁-C₈-Alkyl and phenyl,
or their salts, N-oxides, metal complexes and their stereoisomers.

The radical definitions specified above can be combined with one another as desired.

The "crossed line" representation of the N-C double bond in formula (I) reflects the possible cis/trans stereochemistry of this bond.

According to the type of substituents defined above, the compounds of the formula (I) have basic properties and can form salts, possibly also internal salts or adducts, with inorganic or organic acids or with metal ions. The compounds of the formula (I) carry amidine groups which induce basic properties. Thus, these compounds can be reacted with acids to give salts, or they are obtained directly as salts by the synthesis.

The salts obtainable in this way likewise have fungicidal properties.

Optionally substituted groups may be mono- or polysubstituted, where the substituents in the case of polysubstitutions may be the same or different.

Another subject matter of the invention is the use of the pyrimidinyloxyphenylamidines of the formula (I) according to the invention or of agrochemical formulations comprising these for controlling unwanted microorganisms, in particular for controlling phytopathogenic fungi. of a composition according to claim 7 for controlling phytopathogenic fungi.

Another subject matter of the present invention is an agrochemical formulation for controlling unwanted microorganisms, in particular for controlling phytopathogenic fungi, comprising at least one pyrimidinyloxyphenylamidines of the formula (I) according to the present invention.

A further subject matter of the invention relates to a method for controlling unwanted microorganisms, in particular for controlling phytopathogenic fungi, characterized in that the pyrimidinyloxyphenylamidines of the formula (I) according to the invention or agrochemical formulations comprising these are applied to the microorganisms and/or their habitat, in particular the phytopathogenic fungi and/or their habitat.

Moreover, the invention further relates to seed coated with at least one compound of the formula (I).

The invention finally provides a method for protecting seed against unwanted microorganisms, in particular against phytopathogenic fungi, by using seed treated with at least one compound of the formula (I).

### Definitions

In the definitions of the symbols given in the above formulae, collective terms were used, which are generally representative of the following substituents:
**Halogen:** fluorine, chlorine, bromine or iodine, preferably fluorine, chlorine or bromine, and more preferably fluorine or chlorine.
**Alkyl:** saturated, straight-chain or branched hydrocarbyl radical having 1 to 8, preferably 1 to 6, and more preferably 1 to 4 carbon atoms, for example (but not limited to) C₁-C₆-alkyl such as methyl, ethyl, propyl (n-propyl), 1-methylethyl (iso-propyl), butyl (n-butyl), 1-methylpropyl (sec-butyl), 2-methylpropyl (iso-butyl), 1,1-dimethylethyl (tert-butyl), pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Particularly, said group is a C₁-C₄-alkyl group, e.g. a methyl, ethyl, propyl, 1-methylethyl (isopropyl), butyl, 1-methylpropyl (sec-butyl), 2-methylpropyl (iso-butyl) or 1,1-dimethylethyl (tert-butyl) group. This definition also applies to alkyl as part of a composite substituent, for example cycloalkylalkyl or hydroxyalkyl, unless defined elsewhere like, for example, alkylsulfanyl, alkylsulfinyl, alkylsulfonyl, haloalkyl or haloalkylsulfanyl.
**Alkenyl:** unsaturated, straight-chain or branched hydrocarbyl radicals having 2 to 8, preferably 2 to 6, and more preferably 2 to 4 carbon atoms and one double bond in any position, for example (but not limited to) C₂-C₆-alkenyl such as vinyl, allyl, (E)-2-methylvinyl, (Z)-2-methylvinyl, isopropenyl, homoallyl, (E)-but-2-enyl, (Z)-but-2-enyl, (E)-but-1-enyl, (Z)-but-1-enyl, 2-methylprop-2-enyl, 1-methylprop-2-enyl, 2-methylprop-1-enyl, (E)-1-methylprop-1-enyl, (Z)-1-methylprop-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, 3- methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1- methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylvinyl, 1- isopropylvinyl, (E)-3,3-dimethylprop-1-enyl, (Z)-3,3-dimethylprop-1-enyl, hex-5-enyl, (E)-hex-4- enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, 4-methylpent-4-enyl, 3-methylpent-4-enyl, 2-methylpent-4-enyl, 1- methylpent-4-enyl, 4-methylpent-3-enyl, (E)-3-methylpent-3-enyl, (Z)-3-methylpent-3-enyl, (E)-2-methylpent-3-enyl, (Z)-2-methylpent-3-enyl, (E)-1-methylpent-3-enyl, (Z)-1-methylpent-3-enyl, (E)-4-methylpent-2-enyl, (Z)-4-methylpent-2-enyl, (E)-3-methylpent-2-enyl, (Z)-3-methylpent-2-enyl, (E)-2- methylpent-2-enyl, (Z)-2-methylpent-2-enyl, (E)-1-methylpent-2-enyl, (Z)-1-methylpent-2-enyl, (E)-4-methylpent-1-enyl, (Z)-4-methylpent-1-enyl, (E)-3-methylpent-1-enyl, (Z)-3-methylpent-1 -enyl, (E)-2-methylpent-1 -enyl, (Z)-2-methylpent-1-enyl, (E)-1-methylpent-1-enyl, (Z)-1-methylpent-1-enyl, 3-ethylbut- 3-enyl, 2-ethylbut-3-enyl, 1-ethylbut-3-enyl, (E)-3-ethylbut-2-enyl, (Z)-3-ethylbut-2-enyl, (E)-2-ethylbut-2-enyl, (Z)-2-ethylbut-2-enyl, (E)-1-ethylbut-2-enyl, (Z)-1-ethylbut-2-enyl, (E)-3-ethylbut-1-enyl, (Z)-3-ethylbut-1-enyl, 2-ethylbut-1-enyl, (E)-1-ethylbut-1-enyl, (Z)-1-ethylbut-1-enyl, 2-propylprop-2-enyl, 1-propylprop-2- enyl, 2-isopropylprop-2-enyl, 1-isopropylprop-2-enyl, (E)-2-propylprop-1-enyl, (Z)-2-propylprop-1-enyl, (E)-1-propylprop-1-enyl, (Z)-1-propylprop-1-enyl, (E)-2-isopropylprop-1-enyl, (Z)-2-isopropylprop-1-enyl, (E)-1-isopropylprop-1-enyl, (Z)-1-isopropylprop-1-enyl, 1-(1,1-dimethylethyl)ethenyl, buta-1,3-dienyl, penta-1,4-dienyl, hexa-1,5-dienyl or methylhexadienyl. Particularly, said group is vinyl or allyl. This definition also applies to alkenyl as part of a composite substituent, for example haloalkenyl, unless defined elsewhere.
Alkynyl: straight-chain or branched hydrocarbyl groups having 2 to 8, preferably 2 to 6, and more preferably 2 to 4 carbon atoms and one triple bond in any position, for example (but not limited to) C₂-C₆-alkynyl, such as ethynyl, prop-1-ynyl, prop-2-ynyl, but-1-ynyl, but-2-ynyl, but-3-ynyl, 1-methylprop-2-ynyl, pent-1-ynyl, pent-2-ynyl, pent-3-ynyl, pent-4-ynyl, 2-methylbut-3-ynyl, 1 -methylbut-3-ynyl, 1-methylbut-2-ynyl, 3-methylbut-1-ynyl, 1-ethylprop-2-ynyl, hex-1-ynyl, hex-2-ynyl, hex-3-ynyl, hex-4-ynyl, hex-5-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl, or 3,3-dimethylbut-1-ynyl group. Particularly, said alkynyl group is ethynyl, prop-1-ynyl, or prop-2-ynyl. This definition also applies to alkynyl as part of a composite substituent, for example haloalkynyl, unless defined elsewhere.
**Alkoxy:** saturated, straight-chain or branched alkoxy radicals having 1 to 8, preferably 1 to 6 and more preferably 1 to 4 carbon atoms, for example (but not limited to) C₁-C₆-alkoxy such as methoxy, ethoxy, propoxy, 1-methylethoxy, butoxy, 1-methylpropoxy, 2-methylpropoxy, 1,1-dimethylethoxy, pentoxy, 1-methylbutoxy, 2-methylbutoxy, 3-methylbutoxy, 2,2-dimethylpropoxy, 1-ethylpropoxy, 1,1-dimethylpropoxy, 1,2-dimethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy. This definition also applies to alkoxy as part of a composite substituent, for example haloalkoxy, alkynylalkoxy, unless defined elsewhere.
**Alkoxycarbonyl:** an alkoxy group which has 1 to 8, preferably 1 to 6 and more preferably 1 to 4 carbon atoms (as specified above) and is bonded to the skeleton via a carbonyl group (-C(=O)-). This definition also applies to alkoxycarbonyl as part of a composite substituent, for example cycloalkylalkoxycarbonyl, unless defined elsewhere.
**Alkylsulfanyl:** saturated, straight-chain or branched alkylsulfanyl radicals having 1 to 8, preferably 1 to 6 and more preferably 1 to 4 carbon atoms, for example (but not limited to) C₁-C₆-alkylsulfanyl such as methylsulfanyl, ethylsulfanyl, propylsulfanyl, 1-methylethylsulfanyl, butylsulfanyl, 1-methylpropylsulfanyl, 2-methylpropylsulfanyl, 1,1-dimethylethylsulfanyl, pentylsulfanyl, 1-methylbutylsulfanyl, 2-methylbutylsulfanyl, 3-methylbutylsulfanyl, 2,2-dimethylpropylsulfanyl, 1-ethylpropylsulfanyl, 1,1-dimethylpropylsulfanyl, 1,2-dimethylpropylsulfanyl, hexylsulfanyl, 1-methylpentylsulfanyl, 2-methylpentylsulfanyl, 3-methylpentylsulfanyl, 4-methylpentylsulfanyl, 1,1-dimethylbutylsulfanyl, 1,2-dimethylbutylsulfanyl, 1,3-dimethylbutylsulfanyl, 2,2-dimethylbutylsulfanyl, 2,3-dimethylbutylsulfanyl, 3,3-dimethylbutylsulfanyl, 1-ethylbutylsulfanyl, 2-ethylbutylsulfanyl, 1,1,2-trimethylpropylsulfanyl, 1,2,2-trimethylpropylsulfanyl, 1-ethyl-1-methylpropylsulfanyl and 1-ethyl-2-methylpropylsulfanyl. This definition also applies to alkylsulfanyl as part of a composite substituent, for example haloalkylsulfanyl, unless defined elsewhere.
**Alkylsulfinyl:** saturated, straight-chain or branched alkylsulfinyl radicals having 1 to 8, preferably 1 to 6 and more preferably 1 to 4 carbon atoms, for example (but not limited to) C₁-C₆-alkylsulfinyl such as methylsulfinyl, ethylsulfinyl, propylsulfinyl, 1-methylethylsulfinyl, butylsulfinyl, 1-methylpropylsulfinyl, 2-methylpropylsulfinyl, 1,1-dimethylethylsulfinyl, pentylsulfinyl, 1-methylbutylsulfinyl, 2-methylbutylsulfinyl, 3-methylbutylsulfinyl, 2,2-dimethylpropylsulfinyl, 1-ethylpropylsulfinyl, 1,1-dimethylpropylsulfinyl, 1,2-dimethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl and 1-ethyl-2-methylpropylsulfinyl. This definition also applies to alkylsulfinyl as part of a composite substituent, for example haloalkylsulfinyl, unless defined elsewhere.
**Alkylsulfonyl:** saturated, straight-chain or branched alkylsulfonyl radicals having 1 to 8, preferably 1 to 6 and more preferably 1 to 4 carbon atoms, for example (but not limited to) C₁-C₆-alkylsulfonyl such as methylsulfonyl, ethylsulfonyl, propylsulfonyl, 1-methylethylsulfonyl, butylsulfonyl, 1-methylpropylsulfonyl, 2-methylpropylsulfonyl, 1,1-dimethylethylsulfonyl, pentylsulfonyl, 1-methylbutylsulfonyl, 2-methylbutylsulfonyl, 3-methylbutylsulfonyl, 2,2-dimethylpropylsulfonyl, 1-ethylpropylsulfonyl, 1,1-dimethylpropylsulfonyl, 1,2-dimethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl and 1-ethyl-2-methylpropylsulfonyl. This definition also applies to alkylsulfonyl as part of a composite substituent, for example alkylsulfonylalkyl, unless defined elsewhere.
**Monoalkylamino** represents an amino radical having one alkyl residue with 1 to 4 carbon atoms attached to the nitrogen atom. Non-limiting examples include methylamino, ethylamino, n-propylamino, isopropyl-amino, n-butylamino and tert-butylamino.
**Dialkylamino** represents an amino radical having two independently selected alkyl residues with 1 to 4 carbon atoms each attached to the nitrogen atom. Non-limiting examples include *N,N-*dimethylamino, *N,N*-diethylamino, *N,N*-diisopropylamino, *N*-ethyl-*N*-methylamino, *N*-methyl-*N*-n-propylamino, *N-*iso-propyl-*N*-n-propylamino and *N*-tert-butyl-*N*-methylamino.
**Cycloalkyl:** monocyclic, saturated hydrocarbyl groups having 3 to 10, preferably 3 to 8 and more preferably 3 to 6 carbon ring members, for example (but not limited to) cyclopropyl, cyclopentyl and cyclohexyl. This definition also applies to cycloalkyl as part of a composite substituent, for example cycloalkylalkyl, unless defined elsewhere.
**Cycloalkenyl:** monocyclic, partially unsaturated hydrocarbyl groups having 3 to 10, preferably 3 to 8 and more preferably 3 to 6 carbon ring members, for example (but not limited to) cyclopropenyl, cyclopentenyl and cyclohexenyl. This definition also applies to cycloalkenyl as part of a composite substituent, for example cycloalkenylalkyl, unless defined elsewhere.
**Cycloalkoxy:** monocyclic, saturated cycloalkyloxy radicals having 3 to 10, preferably 3 to 8 and more preferably 3 to 6 carbon ring members, for example (but not limited to) cyclopropyloxy, cyclopentyloxy and cyclohexyloxy. This definition also applies to cycloalkoxy as part of a composite substituent, for example cycloalkoxyalkyl, unless defined elsewhere.
**Cycloalkylsulfanyl:** monocyclic, saturated cycloalkylsulfanyl radicals having 3 to 10, preferably 3 to 8 and more preferably 3 to 6 carbon ring members, for example (but not limited to) cyclopropylsulfanyl, cyclopentylsulfanyl and cyclohexylsulfanyl. This definition also applies to cycloalkylsulfanyl as part of a composite substituent, for example cycloalkylsulfanylalkyl, unless defined elsewhere.
**Haloalkyl:** straight-chain or branched alkyl groups having 1 to 8, preferably 1 to 6 and more preferably 1 to 4 carbon atoms (as specified above), where some or all of the hydrogen atoms in these groups are replaced by halogen atoms as specified above, for example (but not limited to) C₁-C₃-haloalkyl such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl and 1,1,1-trifluoroprop-2-yl. This definition also applies to haloalkyl as part of a composite substituent, for example haloalkylaminoalkyl, unless defined elsewhere.
   Haloalkenyl and haloalkynyl are defined analogously to haloalkyl except that, instead of alkyl groups, alkenyl and alkynyl groups are present as part of the substituent.
**Haloalkoxy:** straight-chain or branched alkoxy groups having 1 to 8, preferably 1 to 6 and more preferably 1 to 4 carbon atoms (as specified above), where some or all of the hydrogen atoms in these groups are replaced by halogen atoms as specified above, for example (but not limited to) C₁-C₃-haloalkoxy such as chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, pentafluoroethoxy and 1,1,1-trifluoroprop-2-oxy. This definition also applies to haloalkoxy as part of a composite substituent, for example haloalkoxyalkyl, unless defined elsewhere.
**Haloalkylsulfanyl:** straight-chain or branched alkylsulfanyl groups having 1 to 8, preferably 1 to 6 and more preferably 1 to 4 carbon atoms (as specified above), where some or all of the hydrogen atoms in these groups are replaced by halogen atoms as specified above, for example (but not limited to) C₁-C₃-haloalkylsulfanyl such as chloromethylsulfanyl, bromomethylsulfanyl, dichloromethylsulfanyl, trichloromethylsulfanyl, fluoromethylsulfanyl, difluoromethylsulfanyl, trifluoromethylsulfanyl, chlorofluoromethylsulfanyl, dichlorofluoromethylsulfanyl, chlorodifluoromethylsulfanyl, 1-chloro-ethylsulfanyl, 1-bromoethylsulfanyl, 1-fluoroethylsulfanyl, 2-fluoroethylsulfanyl, 2,2-difluoroethyl-sulfanyl, 2,2,2-trifluoroethylsulfanyl, 2-chloro-2-fluoroethylsulfanyl, 2-chloro-2,2-difluoroethylsulfanyl, 2,2-dichloro-2-fluoroethylsulfanyl, 2,2,2-trichloroethylsulfanyl, pentafluoroethylsulfanyl, 3,3,3-trifluoropropylthio and 1,1,1-trifluoroprop-2-ylsulfanyl. This definition also applies to haloalkylsulfanyl as part of a composite substituent, for example haloalkylsulfanylalkyl, unless defined elsewhere.
**Aryl:** mono-, bi- or tricyclic aromatic or partially aromatic group having 6 to 14 carbon atoms, for example (but not limited to) phenyl, naphthyl, tetrahydronapthyl, indenyl and indanyl. The binding to the superordinate general structure can be carried out via any possible ring member of the aryl residue. Aryl is preferably selected from phenyl, 1-naphthyl and 2-naphthyl. Phenyl is particularly preferred.
**Heteroaryl:** 5 or 6-membered cyclic aromatic group containing at least 1, if appropriate also 2, 3, 4 or 5 heteroatoms, wherein the heteroatoms are each selected independently of one another from the group S, N and O, and which group can also be part of a bi- or tricyclic system having up to 14 ring members, wherein the ring system can be formed with one or two further cycloalkyl, cycloalkenyl, heterocyclyl, aryl and/or heteroaryl residues and wherein benzofused 5 or 6-membered heteroaryl groups are preferred. The binding to the superordinate general structure can be carried out via any possible ring member of the heteroaryl residue. Examples of **5-membered heteroaryl groups which are attached to the skeleton via one of the carbon ring members** are fur-2-yl, fur-3-yl, thien-2-yl, thien-3-yl, pyrrol-2-yl, pyrrol-3-yl, isoxazol-3-yl, isoxazol-4-yl, isoxazol-5-yl, isothiazol-3-yl, isothiazol-4-yl, isothiazol-5-yl, pyrazol-3-yl, pyrazol-4-yl, pyrazol-5-yl, oxazol-2-yl, oxazol-4-yl, oxazol-5-yl, thiazol-2-yl, thiazol-4-yl, thiazol-5-yl, imidazol-2-yl, imidazole-4-yl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl. Examples of **5-membered heteroaryl groups which are attached to the skeleton via a nitrogen ring member** are pyrrol-1-yl, pyrazol-1-yl, 1,2,4-triazol-1-yl, imidazol-1-yl, 1,2,3-triazol-1-yl and 1,3,4-triazol-1-yl. Examples of **6-membered heteroaryl groups** are pyridine-2-yl, pyridine-3-yl, pyridine-4-yl, pyridazin-3-yl, pyridazin-4-yl, pyrimidin-2-yl, pyrimidin-4-yl, pyrimidin-5-yl, pyrazine-2-yl, 1,3,5-triazin-2-yl, 1,2,4-triazin-3-yl and 1,2,4,5-tetrazin-3-yl. Examples of **benzofused 5-membered heteroaryl groups** are indol-1-yl, indol-2-yl, indol-3-yl, indol-4-yl, indol-5-yl, indol-6-yl, indol-7-yl, benzimidazol-1-yl, benzimidazol-2-yl, benzimidazol-4-yl, benzimidazol-5-yl, indazol-1-yl, indazol-3-yl, indazol-4-yl, indazol-5-yl, indazol-6-yl, indazol-7-yl, indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1,3-benzothiazol-2-yl, 1,3-benzothiazol-4-yl, 1,3-benzothiazol-5-yl, 1,3-benzothiazol-6-yl, 1,3-benzothiazol-7-yl, 1,3-benzoxazol-2-yl, 1,3-benzoxazol-4-yl, 1,3-benzoxazol-5-yl, 1,3-benzoxazol-6-yl and 1,3-benzoxazol-7-yl. Examples of **benzofused 6-membered heteroaryl groups are** quinolin-2-yl, quinolin-3-yl, quinolin-4-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl and isoquinolin-8-yl. Further examples of 5- or 6-membered heteroaryls which are part of a bicyclic ring system are 1,2,3,4-tetrahydroquinolin-1-yl, 1,2,3,4-tetrahydroquinolin-2-yl, 1,2,3,4-tetrahydroquinolin-7-yl, 1,2,3,4-tetrahydroquinolin-8-yl, 1,2,3,4-tetrahydroisoquinolin-1-yl, 1,2,3,4-tetrahydroisoquinolin-2-yl, 1,2,3,4-tetrahydroisoquinolin-5-yl, 1,2,3,4-tetrahydroisoquinolin-6-yl and 1,2,3,4-tetrahydroisoquinolin-7-yl. This definition also applies to heteroaryl as part of a composite substituent, for example heteroarylalkyl, unless defined elsewhere.
**Heterocyclyl:** three- to seven-membered, saturated or partially unsaturated heterocyclic group containing at least one, if appropriate up to four heteroatoms and/or heterogroups independently selected from the group consisting of N, O, S, S(=O), S(=O)₂ and di-(C₁-C₄)alkylsilyl, which group can be benzofused. The binding to the superordinate general structure can be carried out via a ring carbon atom or, if possible, via a ring nitrogen atom of the heterocyclic group. **Saturated heterocyclic groups** in this sense are for example (but not limited to) oxiranyl, aziridinyl, tetrahydrofuran-2-yl, tetrahydrofuran-3-yl, tetrahydrothien-2-yl, tetrahydrothien-3-yl, pyrrolidin-2-yl, pyrrolidin-3-yl, isoxazolidin-3-yl, isoxazolidin-4-yl, isoxazolidin-5-yl, isothiazolidin-3-yl, isothiazolidin-4-yl, isothiazolidin-5-yl, pyrazolidin-3-yl, pyrazolidin-4-yl, pyrazolidin-5-yl, oxazolidin-2-yl, oxazolidin-4-yl, oxazolidin-5-yl, thiazolidin-2-yl, thiazolidin-4-yl, thiazolidin-5-yl, imidazolidin-2-yl, imidazolidin-4-yl, 1,2,4-oxadiazolidin-3-yl, 1,2,4-oxadiazolidin-5-yl, 1,3,4-oxadiazolidin-2-yl, 1,2,4-thiadiazolidin-3-yl, 1,2,4-thiadiazolidin-5-yl, 1,3,4-thiadiazolidin-2-yl, 1,2,4-triazolidin-3-yl, 1,3,4-triazolidin-2-yl, piperidin-2-yl, piperidin-3-yl, piperidin-4-yl, 1,3-dioxan-5-yl, tetrahydropyran-2-yl, tetrahydropyran-4-yl, tetrahydrothien-2-yl, hexahydropyridazin-3-yl, hexahydropyridazin-4-yl, hexahydropyrimidin-2-yl, hexahydropyrimidin-4-yl, hexahydropyrimidin-5-yl, piperazin-2-yl, 1,3,5-hexahydrotriazin-2-yl and 1,2,4-hexahydrotriazin-3-yl. **Partially unsaturated heterocyclic groups** in this sense are for example (but not limited to) 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl. Examples of **benzofused heterocyclic groups** are indolin-1-yl, indolin-2-yl, indolin-3-yl, isoindolin-1-yl, isoindolin-2-yl, 2,3-dihydrobenzofuran-2-yl and 2,3-dihydrobenzofuran-3-yl. This definition also applies to heterocyclyl as part of a composite substituent, for example heterocyclylalkyl, unless defined elsewhere.
**Oxo** represents a doubly bonded oxygen atom.
**Thiooxo** represents a doubly bonded sulfur atom.

Optionally substituted groups may be mono- or polysubstituted, where the substituents in the case of polysubstitutions may be identical or different.

Not included are combinations which are against natural laws and which the person skilled in the art would therefore exclude based on his/her expert knowledge. Ring structures having three or more adjacent oxygen atoms, for example, are excluded.

### Isomers

Depending on the nature of the substituents, the compound of the invention may be present in the form of different stereoisomers. These stereoisomers are, for example, enantiomers, diastereomers, atropisomers or geometric isomers. Accordingly, the invention encompasses both pure stereoisomers and any mixture of these isomers. Where a compound can be present in two or more tautomer forms in equilibrium, reference to the compound by means of one tautomeric description is to be considered to include all tautomer forms.

### cis (= Z-) or trans (=E) isomers

Any of the compounds of the present invention can also exist in one or more geometric isomer forms depending on the number of double bonds in the compound. Geometric isomers by nature of substituents about a double bond or a ring may be present in *cis* (= *Z*-) or *trans* (= *E*-) form. The invention thus relates equally to all geometric isomers and to all possible mixtures, in all proportions.

### Salts / N-Oxides

Depending on the nature of the substituents, the compound of the invention may be present in the form of the free compound or an agrochemically active salt or N-oxide thereof.

Agrochemically active salts include acid addition salts of inorganic and organic acids well as salts of customary bases. Examples of inorganic acids are hydrohalic acids, such as hydrogen fluoride, hydrogen chloride, hydrogen bromide and hydrogen iodide, sulfuric acid, phosphoric acid and nitric acid, and acidic salts, such as sodium bisulfate and potassium bisulfate. Useful organic acids include, for example, formic acid, carbonic acid and alkanoic acids such as acetic acid, trifluoroacetic acid, trichloroacetic acid and propionic acid, and also glycolic acid, thiocyanic acid, lactic acid, succinic acid, citric acid, benzoic acid, cinnamic acid, oxalic acid, saturated or mono- or diunsaturated fatty acids having 6 to 20 carbon atoms, alkylsulphuric monoesters, alkylsulphonic acids (sulphonic acids having straight-chain or branched alkyl radicals having 1 to 20 carbon atoms), arylsulphonic acids or aryldisulphonic acids (aromatic radicals, such as phenyl and naphthyl, which bear one or two sulphonic acid groups), alkylphosphonic acids (phosphonic acids having straight-chain or branched alkyl radicals having 1 to 20 carbon atoms), arylphosphonic acids or aryldiphosphonic acids (aromatic radicals, such as phenyl and naphthyl, which bear one or two phosphonic acid radicals), where the alkyl and aryl radicals may bear further substituents, for example p-toluenesulphonic acid, salicylic acid, p-aminosalicylic acid, 2-phenoxybenzoic acid, 2-acetoxybenzoic acid.

N-oxides can be obtained in a simple manner by customary processes, for example by N-oxidation with hydrogen peroxide (H₂O₂), peracids, for example peroxy sulfuric acid or peroxy carboxylic acids, such as meta-chloroperoxybenzoic acid or peroxymonosulfuric acid (Caro's acid).

E.g. the corresponding N-oxides may be prepared starting from the respective compounds using conventional oxidation methods, e.g. by treating the compounds with an organic peracid such as metachloroperbenzoic acid (e.g. WO-A 2003/64572 or J. Med. Chem. 38 (11), 1892-1903, 1995); or with inorganic oxidizing agents such as hydrogen peroxide (e.g. J. Heterocyc. Chem. 18 (7), 1305-1308, 1981) or oxone (e.g. J. Am. Chem. Soc. 123 (25), 5962-5973, 2001). The oxidation may lead to pure mono-N-oxides or to a mixture of different N-oxides, which can be separated by conventional methods such as chromatography.

### Crystalline Form

The compound of the invention may exist in multiple crystalline and/or amorphous forms. Crystalline forms include unsolvated crystalline forms, solvates and hydrates.

Solvates of the compounds of the invention or their salts are stoichiometric compositions of the compounds with solvents.

In the formula (I), the groups have the meanings as defined above or, for further embodiments, below. The given definitions also apply to all intermediates.

In a further embodiment, the present invention provides compounds of formula (I) wherein
- R¹ and R²: are independently selected from the group consisting of C₁-C₈-Alkyl, C₁-C₈-Alkoxy, C₃-C₈-Cycloalkyl, C₁-C₈-Haloalkyl, C₂-C₈-Alkenyl, C₂-C₈-Alkynyl, C₁-C₈-Alkylsulfanyl, C₁-C₈-Alkylsulfinyl, C₁-C₈-Alkylsulfonyl, cyano, and halogen,
- R³: is selected from the group consisting of
hydrogen, C₁-C₈-Alkyl, C₂-C₈-Alkenyl, C₁-C₈-Haloalkyl, C₁-C₈-Alkoxy, C₃-C₈-Cycloalkyl, C₃-C₈-Cycloalkoxy, C₁-C₈-Alkylsulfanyl, C₁-C₈-Alkylsulfinyl, C₁-C₈-Alkylsulfonyl, C₃-C₈-Cycloalkylsulfanyl, C₃-C₈-Cycloalkyl-S(O)-, C₃-C₈-Cycloalkyl-SO₂-, C₃-C₈-Cycloalkyl-C₁-C₈-Alkylsulfanyl, C₆-C₁₄-Aryl-S-, C₆-C₁₄-Aryl-S- having one or more halogen substituents, C₆-C₁₄-Aryl-S(O)-, C₆-C₁₄-Aryl-S(O)- having one or more halogen substituents, C₆-C₁₄-Aryl-SO₂-, C₆-C₁₄-Aryl-SO₂- having one or more halogen substituents, Aryl-C₁-C₈-Alkyl-S-, C₁-C₈-Haloalkylsulfanyl, C₁-C₈-Alkylsulfanyl substituted with -Si(R⁶)(R⁷)(R⁸), C₁-C₈-Alkylsulfinyl substituted with -Si(R⁶)(R⁷)(R⁸), C₁-C₈-Alkylsulfonyl substituted with -Si(R⁶)(R⁷)(R⁸), Heteroaryl-S-, Heteroaryl-S(O)-, Heteroaryl-SO₂-,
- R⁴: is selected from the group consisting of
C₁-C₈-Alkyl, C₂-C₈-Alkenyl, C₁-C₈-Haloalkyl, C₃-C₈-Cycloalkyl, C₃-C₈-Cycloalkoxy, C₁-C₈-Alkylsulfanyl, C₁-C₈-Alkylsulfinyl, C₁-C₈-Alkylsulfonyl, C₃-C₈-Cycloalkylsulfanyl, C₃-C₈-Cycloalkyl-S(O)-, C₃-C₈-Cycloalkyl-SO₂-, C₃-C₈-Cycloalkyl-C₁-C₈-Alkylsulfanyl, C₆-C₁₄-Aryl-S-, C₆-C₁₄-Aryl-S- having one or more halogen substituents, C₆-C₁₄-Aryl-S(O)-, C₆-C₁₄-Aryl-S(O)- having one or more halogen substituents, C₆-C₁₄-Aryl-SO₂-, C₆-C₁₄-Aryl-SO₂- having one or more halogen substituents, Aryl-C₁-C₈-Alkyl-S-, C₁-C₈-Haloalkylsulfanyl, C₁-C₈-Alkylsulfanyl substituted with -Si(R⁶)(R⁷)(R⁸), C₁-C₈-Alkylsulfinyl substituted with-Si(R⁶)(R⁷)(R⁸), C₁-C₈-Alkylsulfonyl substituted with -Si(R⁶)(R⁷)(R⁸), Heteroaryl-S-, Heteroaryl-S(O)-, Heteroaryl-SO₂-,
wherein R⁶, R⁷ and R⁸ are in each case independently from each other selected from C₁-C₈-Alkyl,
and
- R⁵: is selected from the group consisting of hydrogen, halogen, cyano, C₁-C₈-Alkyl, C₂-C₈-Alkenyl, C₂-C₈-Alkynyl, C₃-C₆-Cycloalkyl, C₃-C₆-Cycloalkoxy, C₁-C₈-Alkylsulfanyl, C₁-C₈-Alkylsulfinyl, C₁-C₈-Alkylsulfonyl,
and salts, N-oxides, metal complexes and stereoisomers thereof.

In another embodiment, the present invention provides compounds of formula (I) wherein
- R¹ and R²: are independently selected from the group consisting of C₁-C₆-Alkyl, C₁-C₆-Haloalkyl, cyano, and halogen,
- R³: is selected from the group consisting of
hydrogen, C₁-C₆-Alkyl, C₂-C₆-Alkenyl, C₁-C₆-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S- substituted with 1 to 5 halogen, C₆-C₁₄-Aryl-C₁-C₆-Alkyl-S-, C₁-C₃-Haloalkylsulfanyl, C₁-C₆-Alkylsulfanyl substituted with -Si(R⁶)(R⁷)(R⁸), Heteroaryl-S-,
- R⁴: is selected from the group consisting of
C₁-C₆-Alkyl, C₂-C₆-Alkenyl, C₁-C₆-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S-substituted with 1 to 5 halogen, C₆-C₁₄-Aryl-C₁-C₆-Alkyl-S-, C₁-C₃-Haloalkylsulfanyl, C₁-C₆-Alkylsulfanyl substituted with -Si(R⁶)(R⁷)(R⁸), Heteroaryl-S-,
wherein R⁶, R⁷ and R⁸ are in each case independently from each other selected from C₁-C₆-Alkyl, and
- R⁵: is selected from the group consisting of hydrogen and C₁-C₆-Alkyl,
and salts, N-oxides, metal complexes and stereoisomers thereof.

In another embodiment, the present invention provides compounds of formula (I) wherein
- R¹: is selected from the group consisting of C₁-C₆-Alkyl and halogen,
- R²: is selected from the group consisting of C₁-C₆-Alkyl, C₁-C₆-Haloalkyl, cyano, and halogen,
- R³: is selected from the group consisting of hydrogen, C₁-C₆-Alkyl, and C₁-C₆-Alkylsulfanyl,
- R⁴: is selected from the group consisting of
C₁-C₆-Alkyl, C₂-C₆-Alkenyl, C₁-C₆-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S-substituted with 1 to 5 halogen, C₆-C₁₄-Aryl-C₁-C₆-Alkyl-S-, C₁-C₈-Haloalkylsulfanyl, C₁-C₆-Alkylsulfanyl substituted with -Si(R⁶)(R⁷)(R⁸), Heteroaryl-S-,
wherein R⁶, R⁷ and R⁸ are independently from each other selected from C₁-C₆-Alkyl, and
- R⁵: is selected from the group consisting of hydrogen and C₁-C₆-Alkyl,
and salts, N-oxides, metal complexes and stereoisomers thereof.

In another embodiment, the present invention provides compounds of formula (I) wherein
- R¹: is selected from the group consisting of C₁-C₆-Alkyl and halogen,
- R²: is selected from the group consisting of C₁-C₆-Haloalkyl, cyano, and halogen,
- R³: is selected from the group consisting of C₁-C₆-Alkyl and C₁-C₆-Alkylsulfanyl,
- R⁴: is selected from the group consisting of
C₁-C₆-Alkyl, C₂-C₆-Alkenyl, C₁-C₆-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S-substituted with 1 to 5 halogen, C₆-C₁₄-Aryl-C₁-C₆-Alkyl-S-, C₁-C₈-Haloalkylsulfanyl, C₁-C₆-Alkylsulfanyl substituted with -Si(R⁶)(R⁷)(R⁸), Heteroaryl-S-,
wherein R⁶, R⁷ and R⁸ are independently from each other selected from C₁-C₆-Alkyl, and
- R⁵: is selected from the group consisting of hydrogen and C₁-C₆-Alkyl,
or wherein
- R¹: is selected from the group consisting of C₁-C₆-Alkyl and halogen,
- R²: is C₁-C₆-Alkyl,
- R³: is selected from the group consisting of C₁-C₆-Alkyl, and C₁-C₆-Alkylsulfanyl,
- R⁴: is selected from the group consisting of
C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S- substituted with 1 to 5 halogen, C₆-C₁₄-Aryl-C₁-C₆-Alkyl-S-, C₁-C₃-Haloalkylsulfanyl, C₁-C₆-Alkylsulfanyl substituted with -Si(R⁶)(R⁷)(R⁸), Heteroaryl-S-,
wherein R⁶, R⁷ and R⁸ are independently from each other selected from C₁-C₆-Alkyl, and
- R⁵: is selected from the group consisting of hydrogen and C₁-C₆-Alkyl,

and salts, N-oxides, metal complexes and stereoisomers thereof.

In another embodiment, the present invention provides compounds of formula (I) wherein
- R¹: is selected from the group consisting of C₁-C₆-Alkyl and halogen,
- R²: is selected from the group consisting of C₁-C₆-Alkyl, C₁-C₆-Haloalkyl, cyano, and halogen,
- R³: is selected from the group consisting of hydrogen, C₁-C₆-Alkyl, and C₁-C₆-Alkylsulfanyl,
- R⁴: is selected from the group consisting of
C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S- substituted with 1 to 5 halogen, C₆-C₁₄-Aryl-C₁-C₆-Alkyl-S-, C₁-C₃-Haloalkylsulfanyl, C₁-C₆-Alkylsulfanyl substituted with -Si(R⁶)(R⁷)(R⁸), Heteroaryl-S-,
wherein R⁶, R⁷ and R⁸ are independently from each other selected from C₁-C₆-Alkyl, and
- R⁵: is selected from the group consisting of hydrogen and C₁-C₆-Alkyl,
and salts, N-oxides, metal complexes and stereoisomers thereof.

In another embodiment, the present invention provides compounds of formula (I) wherein
- R¹ and R²: are independently selected from the group consisting of C₁-C₃-Alkyl, C₁-C₂-Haloalkyl, cyano, and halogen,
- R³: is selected from the group consisting of hydrogen, C₁-C₆-Alkyl, C₂-C₆-Alkenyl, C₁-C₂-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₃-Alkylsulfanyl, C₆-Aryl-S-, C₆-Aryl-S- substituted with 1 to 3 halogen, C₆-Aryl-C₁-C₃-Alkyl-S-, C₁-C₃-Haloalkylsulfanyl, C₁-C₆-Alkylsulfanyl substituted with -Si(R⁶)(R⁷)(R⁸), Heteroaryl-S-,
- R⁴: is selected from the group consisting of
C₁-C₆-Alkyl, C₂-C₆-Alkenyl, C₁-C₂-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₃-Alkylsulfanyl, C₆-Aryl-S-, C₆-Aryl-S- substituted with 1 to 3 halogen, C₆-Aryl-C₁-C₃-Alkyl-S-, C₁-C₃-Haloalkylsulfanyl, C₁-C₆-Alkylsulfanyl substituted with -Si(R⁶)(R⁷)(R⁸), Heteroaryl-S-,
wherein R⁶, R⁷ and R⁸ are in each case independently from each other selected from C₁-C₆-Alkyl, and
- R⁵: is selected from the group consisting of hydrogen and C₁-C₆-Alkyl,
and salts, N-oxides, metal complexes and stereoisomers thereof.

In another embodiment, the present invention provides compounds of formula (I) wherein
- R¹: is selected from the group consisting of C₁-C₃-Alkyl, fluorine and chlorine,
- R²: is selected from the group consisting of C₁-C₃-Alkyl, C₁-C₂-Haloalkyl, cyano, and halogen,
- R³: is selected from the group consisting of hydrogen, C₁-C₃-Alkyl, and C₁-C₆-Alkylsulfanyl,
- R⁴: is selected from the group consisting of
C₁-C₆-Alkyl, C₂-C₆-Alkenyl, C₁-C₂-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₃-Alkylsulfanyl, C₆-Aryl-S-, C₆-Aryl-S- substituted with 1 to 3 halogen, C₆-Aryl-C₁-C₃-Alkyl-S-, C₁-C₃-Haloalkylsulfanyl, C₁-C₆-Alkylsulfanyl substituted with -Si(R⁶)(R⁷)(R⁸), Heteroaryl-S-,
wherein R⁶, R⁷ and R⁸ are independently from each other selected from C₁-C₆-Alkyl, and
- R⁵: is selected from the group consisting of hydrogen and C₁-C₆-Alkyl,
and salts, N-oxides, metal complexes and stereoisomers thereof.

In another embodiment, the present invention provides compounds of formula (I) wherein
- R¹ and R²: are independently selected from the group consisting of methyl, difluoromethyl, trifluoromethyl, cyano, chlorine, and bromine,
- R³: is selected from the group consisting of
hydrogen, methyl, 3,3-dimethylbutyl, tert-butyl,
(E)-3,3-dimethylbut-1-en-1-yl,
trifluoromethyl,
cyclopentyloxy,
methylsulfanyl, ethylsulfanyl, propylthio, isopropylthio, butan-2-ylthio, 2-methylpropylthio, 3-methylbutylthio, tert-butylthio, cyclopropylmethylthio, phenylsulfanyl, benzylsulfinyl, 2,2,2-trifluoroethylthio, 3,3,3-trifluoropropylthio, 2-trimethylsilylethylthio, cyclohexylthio, cyclopentylthio, (2-fluorophenyl)thio, (3-fluorophenyl)thio, (4-fluorophenyl)thio, pyridin-4-ylthio,
- R⁴: is selected from the group consisting of
methyl, 3,3-dimethylbutyl, tert-butyl,
(E)-3,3-dimethylbut-1-en-1-yl,
trifluoromethyl,
cyclopentyloxy,
methylsulfanyl, ethylsulfanyl, propylthio, isopropylthio, butan-2-ylthio, 2-methylpropylthio, 3-methylbutylthio, tert-butylthio, cyclopropylmethylthio, phenylsulfanyl, benzylsulfinyl, 2,2,2-trifluoroethylthio, 3,3,3-trifluoropropylthio, 2-trimethylsilylethylthio, cyclohexylthio, cyclopentylthio, (2-fluorophenyl)thio, (3-fluorophenyl)thio, (4-fluorophenyl)thio, pyridin-4-ylthio, and
- R⁵: is selected from the group consisting of hydrogen and methyl,
and salts, N-oxides, metal complexes and stereoisomers thereof.

In another embodiment, the present invention provides compounds of formula (I) wherein
- R¹: is selected from the group consisting of methyl and chlorine,
- R²: is selected from the group consisting of methyl, difluoromethyl, trifluoromethyl, cyano, and bromine,
- R³: is selected from the group consisting of hydrogen, methyl, butan-2-ylthio, and tert-butylthio,
- R⁴: is selected from the group consisting of
methyl, 3,3-dimethylbutyl, tert-butyl,
(E)-3,3-dimethylbut-1-en-1-yl,
trifluoromethyl,
cyclopentyloxy,
methylsulfanyl, ethylsulfanyl, propylthio, isopropylthio, butan-2-ylthio, 2-methylpropylthio, 3-methylbutylthio, tert-butylthio, cyclopropylmethylthio, phenylsulfanyl, benzylsulfinyl, 2,2,2-trifluoroethylthio, 3,3,3-trifluoropropylthio, 2-trimethylsilylethylthio, cyclohexylthio, cyclopentylthio, (2-fluorophenyl)thio, (3-fluorophenyl)thio, (4-fluorophenyl)thio, pyridin-4-ylthio, and
- R⁵: is selected from the group consisting of hydrogen and methyl,
and salts, N-oxides, metal complexes and stereoisomers thereof.

In another embodiment, the present invention provides compounds of formula (I) wherein
- R¹: is selected from the group consisting of methyl and chlorine,
- R²: is selected from the group consisting of difluoromethyl, trifluoromethyl, cyano, and bromine,
- R³: is selected from the group consisting of hydrogen, methyl, butan-2-ylthio, and tert-butylthio,
- R⁴: is selected from the group consisting of
methyl, 3,3-dimethylbutyl, tert-butyl,
(E)-3,3-dimethylbut-1-en-1-yl,
trifluoromethyl,
cyclopentyloxy, methylsulfanyl, ethylsulfanyl, propylthio, isopropylthio, butan-2-ylthio, 2-methylpropylthio, 3-methylbutylthio, tert-butylthio, cyclopropylmethylthio, phenylsulfanyl, benzylsulfinyl, 2,2,2-trifluoroethylthio, 3,3,3-trifluoropropylthio, 2-trimethylsilylethylthio, cyclohexylthio, cyclopentylthio, (2-fluorophenyl)thio, (3-fluorophenyl)thio, (4-fluorophenyl)thio, pyridin-4-ylthio, and
- R⁵: is selected from the group consisting of hydrogen and methyl,
or wherein
- R¹: is selected from the group consisting of methyl and chlorine,
- R²: is methyl,
- R³: is selected from the group consisting of hydrogen, methyl, butan-2-ylthio, and tert-butylthio,
- R⁴: is selected from the group consisting of
methyl, 3,3-dimethylbutyl,
(E)-3,3-dimethylbut-1-en-1-yl,
methylsulfanyl, ethylsulfanyl, propylthio, isopropylthio, butan-2-ylthio, 2-methylpropylthio, 3-methylbutylthio, tert-butylthio, cyclopropylmethylthio, phenylsulfanyl, benzylsulfinyl, 2,2,2-trifluoroethylthio, 3,3,3-trifluoropropylthio, 2-trimethylsilylethylthio, cyclohexylthio, cyclopentylthio, (2-fluorophenyl)thio, (3-fluorophenyl)thio, (4-fluorophenyl)thio, pyridin-4-ylthio, and
- R⁵: is selected from the group consisting of hydrogen and methyl,
and salts, N-oxides, metal complexes and stereoisomers thereof.

In another embodiment, the present invention provides compounds of formula (I), wherein said compounds are selected from the group consisting of
(I.001) N'-[5-bromo-4-(2-tert-butylsulfanylpyrimidin-4-yl)oxy-2-methylphenyl]-N-ethyl-N-methyl-methanimidamide,
(I.002) N'-[2,5-dimethyl-4-[2-(3-methylbutylsulfanyl)pyrimidin-4-yl]oxyphenyl]-N-ethyl-N-methyl-methanimidamide,
(I.003) N'-[4-[2-(3,3-dimethylbutyl)pyrimidin-4-yl]oxy-2,5-dimethylphenyl]-N-ethyl-N-methylmethan-imidamide,
(I.004) N'-[2,5-dimethyl-4-[2-[rac-(2R)-butan-2-yl]sulfanylpyrimidin-4-yl]oxyphenyl]-N-ethyl-N-methylmethanimidamide,
(I.005) N'-[5-bromo-2-methyl-4-[2-(2,2,2-trifluoroethylsulfanyl)pyrimidin-4-yl]oxyphenyl]-N-ethyl-N-methylmethanimidamide,
(I.006) N'-[2-chloro-5-methyl-4-[2-(2,2,2-trifluoroethylsulfanyl)pyrimidin-4-yl]oxyphenyl]-N-ethyl-N-methylmethanimidamide,
(I.007) N'-[5-bromo-2-methyl-4-[2-(2-methylpropylsulfanyl)pyrimidin-4-yl]oxyphenyl]-N-ethyl-N-methylmethanimidamide,
(I.008) N-ethyl-N'-[4-[2-(2-fluorophenyl)sulfanylpyrimidin-4-yl]oxy-2,5-dimethylphenyl]-N-methyl-methanimidamide,
(I.009) N-ethyl-N'-[4-[2-(3-fluorophenyl)sulfanylpyrimidin-4-yl]oxy-2,5-dimethylphenyl]-N-methyl-methanimidamide,
(I.010) N-ethyl-N'-[4-[2-(4-fluorophenyl)sulfanylpyrimidin-4-yl]oxy-2,5-dimethylphenyl]-N-methyl-methanimidamide,
(I.011) N'-[4-(2-cyclohexylsulfanylpyrimidin-4-yl)oxy-2,5-dimethylphenyl]-N-ethyl-N-methylmethan-imidamide,
(I.012) N'-[4-(2-cyclopentylsulfanylpyrimidin-4-yl)oxy-2,5-dimethylphenyl]-N-ethyl-N-methylmethan-imidamide,
(I.013) N'-[2,5-dimethyl-4-[2-(3,3,3-trifluoropropylsulfanyl)pyrimidin-4-yl]oxyphenyl]-N-ethyl-N-methylmethanimidamide,
(I.014) N'-[5-bromo-2-methyl-4-(2-propan-2-ylsulfanylpyrimidin-4-yl)oxyphenyl]-N-ethyl-N-methyl-methanimidamide,
(I.015) N'-[5-bromo-2-methyl-4-(2-propylsulfanylpyrimidin-4-yl)oxyphenyl]-N-ethyl-N-methylmethan-imidamide,
(I.016) N'-[5-bromo-4-(2-ethylsulfanylpyrimidin-4-yl)oxy-2-methylphenyl]-N-ethyl-N-methylmethan-imidamide,
(I.017) N'-[5-bromo-2-methyl-4-[2-(trifluoromethyl)pyrimidin-4-yl]oxyphenyl]-N-ethyl-N-methyl-methanimidamide,
(I.018) N'-[5-bromo-4-(6-tert-butylsulfanyl-2-methylpyrimidin-4-yl)oxy-2-methylphenyl]-N-ethyl-N-methylmethanimidamide,
(I.019) N-ethyl-N'-[4-(2-ethylsulfanyl-6-methylpyrimidin-4-yl)oxy-2,5-dimethylphenyl]-N-methyl-methanimidamide,
(I.020) N'-[2,5-dimethyl-4-(6-methyl-2-propylsulfanylpyrimidin-4-yl)oxyphenyl]-N-ethyl-N-methyl-methanimidamide,
(I.021) N'-[2,5-dimethyl-4-[6-methyl-2-(2-methylpropylsulfanyl)pyrimidin-4-yl]oxyphenyl]-N-ethyl-N-methylmethanimidamide,
(I.022) N'-[2,5-dimethyl-4-(6-methyl-2-propan-2-ylsulfanylpyrimidin-4-yl)oxyphenyl]-N-ethyl-N-methylmethanimidamide,
(I.023) N'-[4-(6-tert-butylsulfanyl-2-methylpyrimidin-4-yl)oxy-2,5-dimethylphenyl]-N-ethyl-N-methyl-methanimidamide,
(I.024) N'-[4-(6-butan-2-ylsulfanyl-2-methylpyrimidin-4-yl)oxy-2,5-dimethylphenyl]-N-ethyl-N-methyl-methanimidamide,
(I.025) N'-[5-(difluoromethyl)-2-methyl-4-[2-(2-methylpropylsulfanyl)pyrimidin-4-yl]oxyphenyl]-N-ethyl-N-methylmethanimidamide,
(I.026) N'-[2,5-dimethyl-4-[2-(2-methylpropylsulfanyl)pyrimidin-4-yl]oxyphenyl]-N-ethyl-N-methyl-methanimidamide,
(I.027) N-ethyl-N'-[4-(2-ethylsulfanylpyrimidin-4-yl)oxy-2,5-dimethylphenyl]-N-methylmethanimid-amide,
(I.028) N'-[2,5-dimethyl-4-(2-propan-2-ylsulfanylpyrimidin-4-yl)oxyphenyl]-N-ethyl-N-methylmethan-imidamide,
(I.029) N'-[2,5-dimethyl-4-(2-propylsulfanylpyrimidin-4-yl)oxyphenyl]-N-ethyl-N-methylmethanimid-amide,
(I.030) N'-[2,5-dimethyl-4-[2-(trifluoromethyl)pyrimidin-4-yl]oxyphenyl]-N-ethyl-N-methylmethan-imidamide,
(I.031) N'-[2,5-dimethyl-4-[2-(2,2,2-trifluoroethylsulfanyl)pyrimidin-4-yl]oxyphenyl]-N-ethyl-N-methylmethanimidamide,
(I.032) N'-[4-(2-tert-butylpyrimidin-4-yl)oxy-2,5-dimethylphenyl]-N-ethyl-N-methylmethanimidamide,
(I.033) N'-[2,5-dimethyl-4-(6-methyl-2-methylsulfanylpyrimidin-4-yl)oxyphenyl]-N-ethyl-N-methyl-methanimidamide,
(I.034) N'-[4-[2-[(E)-3,3-dimethylbut-1-enyl]pyrimidin-4-yl]oxy-2,5-dimethylphenyl]-N-ethyl-N-methylmethanimidamide,
(I.035) N-ethyl-N-methyl-N'-[2-methyl-4-[2-(2-methylpropylsulfanyl)pyrimidin-4-yl]oxy-5-(trifluoromethyl)phenyl]methanimidamide,
(I.036) N'-[5-(difluoromethyl)-2-methyl-4-(2-propylsulfanylpyrimidin-4-yl)oxyphenyl]-N-ethyl-N-methylmethanimidamide,
(I.037) N'-[5-bromo-2-methyl-4-(2-phenylsulfanylpyrimidin-4-yl)oxyphenyl]-N-ethyl-N-methylmethan-imidamide,
(I.038) N'-[5-bromo-4-(2-tert-butylpyrimidin-4-yl)oxy-2-methylphenyl]-N-ethyl-N-methylmethanimid-amide,
(I.039) N'-[2,5-dimethyl-4-(2-phenylsulfanylpyrimidin-4-yl)oxyphenyl]-N-ethyl-N-methylmethanimid-amide,
(I.040) N'-[4-[2-(cyclopropylmethylsulfanyl)pyrimidin-4-yl]oxy-2,5-dimethylphenyl]-N-ethyl-N-methylmethanimidamide,
(I.041) N'-[4-(2-cyclopentyloxypyrimidin-4-yl)oxy-2,5-dimethylphenyl]-N-ethyl-N-methylmethanimid-amide,
(I.042) N'-[4-(2-benzylsulfanylpyrimidin-4-yl)oxy-2,5-dimethylphenyl]-N-ethyl-N-methylmethanimid-amide,
(I.043) N'-[2,5-dimethyl-4-[2-(2-trimethylsilylethylsulfanyl)pyrimidin-4-yl]oxyphenyl]-N-ethyl-N-methylmethanimidamide,
(I.044) N'-[4-(2-tert-butylpyrimidin-4-yl)oxy-5-cyano-2-methylphenyl]-N-ethyl-N-methylmethanimid-amide,
(I.045) N'-[4-(2-tert-butylsulfanylpyrimidin-4-yl)oxy-2-chloro-5-methylphenyl]-N-ethyl-N-methyl-methanimidamide,
(I.046) N'-[2-chloro-5-methyl-4-(2-propan-2-ylsulfanylpyrimidin-4-yl)oxyphenyl]-N-ethyl-N-methyl-methanimidamide,
(I.047) N'-[2,5-dimethyl-4-(2-pyridin-4-ylsulfanylpyrimidin-4-yl)oxyphenyl]-N-ethyl-N-methylmethan-imidamide,
(I.048) N'-[2-chloro-5-methyl-4-[2-(2-methylpropylsulfanyl)pyrimidin-4-yl]oxyphenyl]-N-ethyl-N-methylmethanimidamide,
(I.049) N'-[5-cyano-2-methyl-4-[2-(2-methylpropylsulfanyl)pyrimidin-4-yl]oxyphenyl]-N-ethyl-N-methylmethanimidamide,
(I.050) N'-[5-cyano-2-methyl-4-(2-propylsulfanylpyrimidin-4-yl)oxyphenyl]-N-ethyl-N-methylmethan-imidamide, and
(I.051) N'-[5-(difluoromethyl)-2-methyl-4-[5-methyl-2-(2-methylpropylsulfanyl)pyrimidin-4-yl]oxyphenyl]-N-ethyl-N-methylmethanimidamide.

Further embodiments of the compounds of formula (I) of the present invention:
In a further embodiment, the present invention provides compounds of formula (I), *supra,* in which:
- R¹: is selected from the group consisting of C₁-C₈-Alkyl, C₁-C₈-Alkoxy, C₃-C₈-Cycloalkyl, C₁-C₈-Haloalkyl, C₂-C₈-Alkenyl, C₂-C₈-Alkynyl, C₁-C₈-Alkylsulfanyl, C₁-C₈-Alkylsulfinyl, C₁-C₈-Alkylsulfonyl, cyano, and halogen,
and salts, N-oxides, metal complexes and stereoisomers thereof.

In a further embodiment, the present invention provides compounds of formula (I), *supra,* in which:
- R¹: is selected from the group consisting of C₁-C₆-Alkyl, C₁-C₆-Haloalkyl, cyano, and halogen,
and salts, N-oxides, metal complexes and stereoisomers thereof.

In a further embodiment, the present invention provides compounds of formula (I), *supra,* in which:
- R¹: is selected from the group consisting of C₁-C₆-Alkyl and halogen,
and salts, N-oxides, metal complexes and stereoisomers thereof.

In a further embodiment, the present invention provides compounds of formula (I), *supra,* in which:
- R¹: is selected from the group consisting of C₁-C₃-Alkyl, C₁-C₂-Haloalkyl, cyano, and halogen,
and salts, N-oxides, metal complexes and stereoisomers thereof.

In a further embodiment, the present invention provides compounds of formula (I), *supra,* in which:
- R¹: is selected from the group consisting of methyl, difluoromethyl, trifluoromethyl, cyano, chlorine, and bromine,
and salts, N-oxides, metal complexes and stereoisomers thereof.

In a further embodiment, the present invention provides compounds of formula (I), *supra,* in which:
- R¹: is selected from the group consisting of methyl and chlorine,
and salts, N-oxides, metal complexes and stereoisomers thereof.

In a further embodiment, the present invention provides compounds of formula (I), *supra,* in which:
- R²: is selected from the group consisting of C₁-C₈-Alkyl, C₁-C₈-Alkoxy, C₃-C₈-Cycloalkyl, C₁-C₈-Haloalkyl, C₂-C₈-Alkenyl, C₂-C₈-Alkynyl, C₁-C₈-Alkylsulfanyl, C₁-C₈-Alkylsulfinyl, C₁-C₈-Alkylsulfonyl, cyano, and halogen,
and salts, N-oxides, metal complexes and stereoisomers thereof.

In a further embodiment, the present invention provides compounds of formula (I), *supra,* in which:
- R²: is selected from the group consisting of C₁-C₆-Alkyl, C₁-C₆-Haloalkyl, cyano, and halogen,
and salts, N-oxides, metal complexes and stereoisomers thereof.

In a further embodiment, the present invention provides compounds of formula (I), *supra,* in which:
- R²: is selected from the group consisting of C₁-C₆-Haloalkyl, cyano, and halogen,
and salts, N-oxides, metal complexes and stereoisomers thereof.

In a further embodiment, the present invention provides compounds of formula (I), *supra,* in which:
- R²: is selected from the group consisting of C₁-C₆-Alkyl,
and salts, N-oxides, metal complexes and stereoisomers thereof.

In a further embodiment, the present invention provides compounds of formula (I), *supra,* in which:
- R²: is selected from the group consisting of C₁-C₃-Alkyl, C₁-C₂-Haloalkyl, cyano, and halogen,
and salts, N-oxides, metal complexes and stereoisomers thereof.

In a further embodiment, the present invention provides compounds of formula (I), *supra,* in which:
- R²: is selected from the group consisting of methyl, difluoromethyl, trifluoromethyl, cyano, and bromine,
and salts, N-oxides, metal complexes and stereoisomers thereof.

In a further embodiment, the present invention provides compounds of formula (I), *supra,* in which:
- R²: is selected from the group consisting of difluoromethyl, trifluoromethyl, cyano, and bromine,
and salts, N-oxides, metal complexes and stereoisomers thereof.

In a further embodiment, the present invention provides compounds of formula (I), *supra,* in which:
- R²: is methyl,
and salts, N-oxides, metal complexes and stereoisomers thereof.

In a further embodiment, the present invention provides compounds of formula (I), *supra,* in which:
- R³: is selected from the group consisting of hydrogen, C₁-C₈-Alkyl, C₂-C₈-Alkenyl, C₁-C₈-Haloalkyl, C₁-C₈-Alkoxy, C₃-C₈-Cycloalkyl, C₃-C₈-Cycloalkoxy, C₁-C₈-Alkylsulfanyl, C₁-C₈-Alkylsulfinyl, C₁-C₈-Alkylsulfonyl, C₃-C₈-Cycloalkylsulfanyl, C₃-C₈-Cycloalkyl-S(O)-, C₃-C₈-Cycloalkyl-SO₂-, C₃-C₈-Cycloalkyl-C₁-C₈-Alkylsulfanyl, C₆-C₁₄-Aryl-S-, C₆-C₁₄-Aryl-S- having one or more halogen substituents, C₆-C₁₄-Aryl-S(O)-, C₆-C₁₄-Aryl-S(O)- having one or more halogen substituents, C₆-C₁₄-Aryl-SO₂-, C₆-C₁₄-Aryl-SO₂- having one or more halogen substituents, Aryl-C₁-C₈-Alkyl-S-, C₁-C₈-Haloalkylsulfanyl, C₁-C₈-Alkylsulfanyl substituted with -Si(R⁶)(R⁷)(R⁸), C₁-C₈-Alkylsulfinyl substituted with -Si(R⁶)(R⁷)(R⁸), C₁-C₈-Alkylsulfonyl substituted with-Si(R⁶)(R⁷)(R⁸), Heteroaryl-S-, Heteroaryl-S(O)-, Heteroaryl-SO₂-,
wherein R⁶, R⁷ and R⁸ are independently from each other selected from C₁-C₈-Alkyl,
and salts, N-oxides, metal complexes and stereoisomers thereof.

In a further embodiment, the present invention provides compounds of formula (I), *supra,* in which:
- R³: is selected from the group consisting of hydrogen, C₁-C₆-Alkyl, C₂-C₆-Alkenyl, C₁-C₆-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S- substituted with 1 to 5 halogen, C₆-C₁₄-Aryl-C₁-C₆-Alkyl-S-, C₁-C₃-Haloalkylsulfanyl, C₁-C₆-Alkylsulfanyl substituted with -Si(R⁶)(R⁷)(R⁸), Heteroaryl-S-,
wherein R⁶, R⁷ and R⁸ are independently from each other selected from C₁-C₆-Alkyl,
and salts, N-oxides, metal complexes and stereoisomers thereof.

In a further embodiment, the present invention provides compounds of formula (I), *supra,* in which:
- R³: is selected from the group consisting of hydrogen, C₁-C₆-Alkyl, and C₁-C₆-Alkylsulfanyl,
and salts, N-oxides, metal complexes and stereoisomers thereof.

In a further embodiment, the present invention provides compounds of formula (I), *supra,* in which:
- R³: is selected from the group consisting of hydrogen, methyl, 3,3-dimethylbutyl, tert-butyl,
(E)-3,3-dimethylbut-1-en-1-yl,
trifluoromethyl,
cyclopentyloxy,
methylsulfanyl, ethylsulfanyl, propylthio, isopropylthio, butan-2-ylthio, 2-methylpropylthio, 3-methylbutylthio, tert-butylthio, cyclopropylmethylthio, phenylsulfanyl, benzylsulfinyl, 2,2,2-trifluoroethylthio, 3,3,3-trifluoropropylthio, 2-trimethylsilylethylthio, cyclohexylthio, cyclopentylthio, (2-fluorophenyl)thio, (3-fluorophenyl)thio, (4-fluorophenyl)thio, pyridin-4-ylthio,
and salts, N-oxides, metal complexes and stereoisomers thereof.

In a further embodiment, the present invention provides compounds of formula (I), *supra,* in which:
- R³: is selected from the group consisting of hydrogen, methyl, butan-2-ylthio, and tert-butylthio,
and salts, N-oxides, metal complexes and stereoisomers thereof.

In a further embodiment, the present invention provides compounds of formula (I), *supra,* in which:
- R⁴: is selected from the group consisting of C₁-C₈-Alkyl, C₂-C₈-Alkenyl, C₁-C₈-Haloalkyl, C₃-C₈-Cycloalkyl, C₃-C₈-Cycloalkoxy, C₁-C₈-Alkylsulfanyl, C₁-C₈-Alkylsulfinyl, C₁-C₈-Alkylsulfonyl, C₃-C₈-Cycloalkylsulfanyl, C₃-C₈-Cycloalkyl-S(O)-, C₃-C₈-Cycloalkyl-SO₂-, C₃-C₈-Cycloalkyl-C₁-C₈-Alkylsulfanyl, C₆-C₁₄-Aryl-S-, C₆-C₁₄-Aryl-S- having one or more halogen substituents, C₆-C₁₄-Aryl-S(O)-, C₆-C₁₄-Aryl-S(O)- having one or more halogen substituents, C₆-C₁₄-Aryl-SO₂-, C₆-C₁₄-Aryl-SO₂- having one or more halogen substituents, Aryl-C₁-C₈-Alkyl-S-, C₁-C₈-Haloalkylsulfanyl, C₁-C₈-Alkylsulfanyl substituted with -Si(R⁶)(R⁷)(R⁸), C₁-C₈-Alkylsulfinyl substituted with-Si(R⁶)(R⁷)(R⁸), C₁-C₈-Alkylsulfonyl substituted with -Si(R⁶)(R⁷)(R⁸), Heteroaryl-S-, Heteroaryl-S(O)-, Heteroaryl-SO₂-,
wherein R⁶, R⁷ and R⁸ are independently from each other selected from C₁-C₈-Alkyl,
and salts, N-oxides, metal complexes and stereoisomers thereof.

In a further embodiment, the present invention provides compounds of formula (I), *supra,* in which:
- R⁴: is selected from the group consisting of C₁-C₆-Alkyl, C₂-C₆-Alkenyl, C₁-C₆-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S- substituted with 1 to 5 halogen, C₆-C₁₄-Aryl-C₁-C₆-Alkyl-S-, C₁-C₈-Haloalkylsulfanyl, C₁-C₆-Alkylsulfanyl substituted with -Si(R⁶)(R⁷)(R⁸), Heteroaryl-S-,
wherein R⁶, R⁷ and R⁸ are independently from each other selected from C₁-C₆-Alkyl,
and salts, N-oxides, metal complexes and stereoisomers thereof.

In a further embodiment, the present invention provides compounds of formula (I), *supra,* in which:
- R⁴: is selected from the group consisting of C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S- substituted with 1 to 5 halogen, C₆-C₁₄-Aryl-C₁-C₆-Alkyl-S-, C₁-C₈-Haloalkylsulfanyl, C₁-C₆-Alkylsulfanyl substituted with-Si(R⁶)(R⁷)(R⁸), Heteroaryl-S-,
wherein R⁶, R⁷ and R⁸ are independently from each other selected from C₁-C₆-Alkyl,
and salts, N-oxides, metal complexes and stereoisomers thereof.

In a further embodiment, the present invention provides compounds of formula (I), *supra,* in which:
- R⁴: is selected from the group consisting of
methyl, 3,3-dimethylbutyl, tert-butyl,
(E)-3,3-dimethylbut-1-en-1-yl,
trifluoromethyl,
cyclopentyloxy,
methylsulfanyl, ethylsulfanyl, propylthio, isopropylthio, butan-2-ylthio, 2-methylpropylthio, 3-methylbutylthio, tert-butylthio, cyclopropylmethylthio, phenylsulfanyl, benzylsulfinyl, 2,2,2-trifluoroethylthio, 3,3,3-trifluoropropylthio, 2-trimethylsilylethylthio, cyclohexylthio, cyclopentylthio, (2-fluorophenyl)thio, (3-fluorophenyl)thio, (4-fluorophenyl)thio, pyridin-4-ylthio,
and salts, N-oxides, metal complexes and stereoisomers thereof.

In a further embodiment, the present invention provides compounds of formula (I), *supra,* in which:
- R⁴: is selected from the group consisting of
methyl, 3,3-dimethylbutyl,
(E)-3,3-dimethylbut-1-en-1-yl,
methylsulfanyl, ethylsulfanyl, propylthio, isopropylthio, butan-2-ylthio, 2-methylpropylthio, 3-methylbutylthio, tert-butylthio, cyclopropylmethylthio, phenylsulfanyl, benzylsulfinyl, 2,2,2-trifluoroethylthio, 3,3,3-trifluoropropylthio, 2-trimethylsilylethylthio, cyclohexylthio, cyclopentylthio, (2-fluorophenyl)thio, (3-fluorophenyl)thio, (4-fluorophenyl)thio, pyridin-4-ylthio,
and salts, N-oxides, metal complexes and stereoisomers thereof.

In a further embodiment, the present invention provides compounds of formula (I), *supra,* in which:
- R⁵: is selected from the group consisting of hydrogen, halogen, cyano, C₁-C₈-Alkyl, C₂-C₈-Alkenyl, C₂-C₈-Alkynyl, C₃-C₆-Cycloalkyl, C₃-C₆-Cycloalkoxy, C₁-C₈-Alkylsulfanyl, C₁-C₈-Alkylsulfinyl, C₁-C₈-Alkylsulfonyl,
and salts, N-oxides, metal complexes and stereoisomers thereof.

In a further embodiment, the present invention provides compounds of formula (I), *supra,* in which:
- R⁵: is selected from the group consisting of hydrogen and C₁-C₆-Alkyl,
and salts, N-oxides, metal complexes and stereoisomers thereof.

In a further embodiment, the present invention provides compounds of formula (I), *supra,* in which:
- R⁵: is selected from the group consisting of hydrogen and methyl,
and salts, N-oxides, metal complexes and stereoisomers thereof.

In a particular further embodiment of the first aspect, the present invention covers combinations of two or more of the above mentioned embodiments under the heading "further embodiments of the compounds of formula (I) the present invention".

The present invention covers any sub-combination within any embodiment or aspect of the present invention of compounds of general formula (I), *supra.*

Compounds in connection with the present invention are in another embodiment compounds of formula (I) selected from the group consisting of Table 1:

**Table 1: Pyrimidinyloxyphenylamidines according to the present invention; CN = cyano; OMe = methoxy;**

| **Ex N°** | **R1** | **R2** | **R3** | **R4** | **R5** |
|---|---|---|---|---|---|
| I.001 | CH₃ | Br | H | tert-butylthio | H |
| I.002 | CH₃ | CH₃ | H | 3-methylbutylthio | H |
| I.003 | CH₃ | CH₃ | H | 3,3-dimethylbutyl | H |
| I.004 | CH₃ | CH₃ | H | butan-2-ylthio | H |
| I.005 | CH₃ | Br | H | 2,2,2-trifluoroethylthio | H |
| I.006 | Cl | CH₃ | H | 2,2,2-trifluoroethylthio | H |
| I.007 | CH₃ | Br | H | 2-methylpropylthio | H |
| I.008 | CH₃ | CH₃ | H | (2-fluorophenyl)thio | H |
| I.009 | CH₃ | CH₃ | H | (3-fluorophenyl)thio | H |
| I.010 | CH₃ | CH₃ | H | (4-fluorophenyl)thio | H |
| I.011 | CH₃ | CH₃ | H | cyclohexylthio | H |
| I.012 | CH₃ | CH₃ | H | cyclopentylthio | H |
| I.013 | CH₃ | CH₃ | H | 3,3,3-trifluoropropylthio | H |
| I.014 | CH₃ | Br | H | isopropylthio | H |
| I.015 | CH₃ | Br | H | propylthio | H |
| I.016 | CH₃ | Br | H | ethylsulfanyl | H |
| I.017 | CH₃ | Br | H | CF₃ | H |
| I.018 | CH₃ | Br | tert-butylthio | CH₃ | H |
| I.019 | CH₃ | CH₃ | CH₃ | ethylsulfanyl | H |
| I.020 | CH₃ | CH₃ | CH₃ | propylthio | H |
| I.021 | CH₃ | CH₃ | CH₃ | 2-methylpropylthio | H |
| I.022 | CH₃ | CH₃ | CH₃ | isopropylthio | H |
| I.023 | CH₃ | CH₃ | tert-butylthio | CH₃ | H |
| I.024 | CH₃ | CH₃ | butan-2-ylthio | CH₃ | H |
| I.025 | CH₃ | CHF₂ | H | 2-methylpropylthio | H |
| I.026 | CH₃ | CH₃ | H | 2-methylpropylthio | H |
| I.027 | CH₃ | CH₃ | H | ethylsulfanyl | H |
| I.028 | CH₃ | CH₃ | H | isopropylthio | H |
| I.029 | CH₃ | CH₃ | H | propylthio | H |
| I.030 | CH₃ | CH₃ | H | CF₃ | H |
| I.031 | CH₃ | CH₃ | H | 2,2,2-trifluoroethylthio | H |
| I.032 | CH₃ | CH₃ | H | tert-butyl | H |
| I.033 | CH₃ | CH₃ | CH₃ | methylsulfanyl | H |
| I.034 | CH₃ | CH₃ | H | (E)-3,3-dimethylbut-1-en-1-yl | H |
| I.035 | CH₃ | CF₃ | H | 2-methylpropylthio | H |
| I.036 | CH₃ | CHF₂ | H | propylthio | H |
| I.037 | CH₃ | Br | H | phenylsulfanyl | H |
| I.038 | CH₃ | Br | H | tert-butyl | H |
| I.039 | CH₃ | CH₃ | H | phenylsulfanyl | H |
| I.040 | CH₃ | CH₃ | H | cyclopropylmethylthio | H |
| I.041 | CH₃ | CH₃ | H | cyclopentyloxy | H |
| I.042 | CH₃ | CH₃ | H | benzylsulfanyl | H |
| I.043 | CH₃ | CH₃ | H | 2-trimethylsilylethylthio | H |
| I.044 | CH₃ | CN | H | tert-butyl | H |
| I.045 | Cl | CH₃ | H | tert-butylthio | H |
| I.046 | Cl | CH₃ | H | isopropylthio | H |
| I.047 | CH₃ | CH₃ | H | pyridin-4-ylthio | H |
| I.048 | Cl | CH₃ | H | 2-methylpropylthio | H |
| I.049 | CH₃ | CN | H | 2-methylpropylthio | H |
| I.050 | CH₃ | CN | H | propylthio | H |
| I.051 | CH₃ | CHF₂ | H | 2-methylpropylthio | CH₃ |

The compounds of the formula (I) carry amidine groups which induce basic properties. Thus, these compounds can be reacted with acids to give salts.

In another embodiment, the present invention provides Pyrimidinyloxyphenylamidines of the formula (I) which are selected from the group consisting of Example Number (Ex N°) 1.001; I.002; I.003; I.004; I.005; I.006; I.007; I.008; I.009; I.010; I.011; I.012; I.013; I.014; I.015; I.016; I.017; I.018; I.019; I.020; I.021; I.022; I.023; I.024; I.025; I.026; I.027; I.028; I.029; I.030; I.031; I.032; I.033; I.034; I.035; I.036; I.037; I.038; I.039; I.040; I.041; I.042; I.043; I.044; I.045.

In another embodiment, the present invention provides Pyrimidinyloxyphenylamidines of the formula (I) which are selected from the group consisting of Example Number (Ex N°) 1.001; 1.002; 1.003; 1.004; 1.005; 1.006; 1.007; 1.008; 1.009; 1.010; 1.011; 1.012; 1.013; 1.014; 1.015; 1.016; 1.017; 1.018; 1.019; 1.020; 1.021; 1.022; 1.023; 1.024; 1.025; 1.026; 1.027; 1.028; 1.029; 1.031; 1.033; 1.034; 1.035; 1.036; 1.037; 1.038; 1.039; 1.040; 1.042; 1.043; 1.044; 1.045.

### Abbreviations and Symbols

- AcOH: acetic acid
- aq.: Aqueous
- BOC: tert-butoxycarbonyl
- DIPEA: diisopropylethylamine
- DMF: N,N-dimethylformamide
- DMSO: dimethylsulfoxide
- EtOAc: ethyl acetate
- HPLC: high performance liquid chromatography
- LCMS: liquid chromatography-mass spectrometry
- M: molarity
- mCPBA: *meta*-chloroperbenzoic acid
- MeCN: acetonitrile
- MeOH: methanol
- Ms: methane sulfonyl
- NMR: nuclear magnetic resonance
- RT: room temperature
- Rₜ: retention time
- sat.: saturated
- T: temperature
- THF: tetrahydrofuran
- Ts: *p*-toluenesulfonyl

### Preparation of the pyrimidinyloxyphenylamidines of the formula (I) according to the invention

The pyrimidinyloxyphenylamidines of the formula (I) according to the invention can be obtained by the processes shown in schemes (I-III) below. R¹, R², R³, R⁴ and R⁵ are as previously defined.

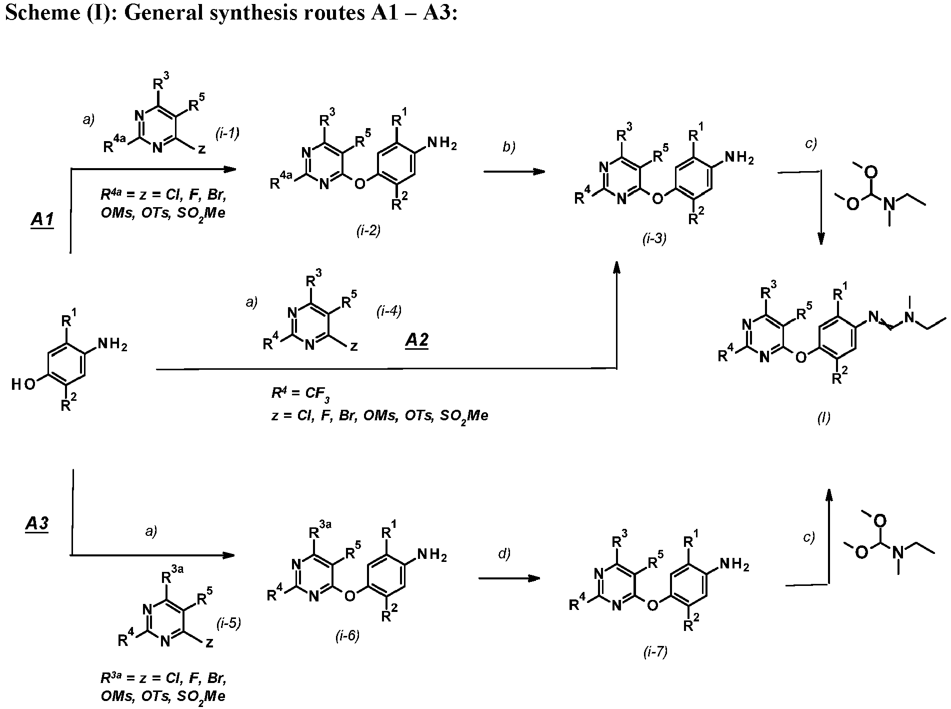

Conditions: a) 2N NaOH (aq.), acetone, RT; b) NaH, DMF, R⁴-Nucleophile, 0°C to 80°C; c) N-(dimethoxymethyl)-N-methyl-ethanamine, toluene, 80°C; d) NaH, DMF, R³-Nucleophile, 0°C to 80°C.
A1: An aminophenol is reacted with a pyrimidine (i-1) to form compounds of formula (i-2). For example, a mixture of a suitable base, such as NaOH in a suitable solvent, such as acetone are mixed at temperatures ranging from 0 to 100 °C to provide compounds of formula (i-2), which may then be isolated and, if necessary and desired, purified using techniques well known in the art, such as chromatography. The required aminophenols or pyrimidines (i-1) are commercially available or may be synthesized by methods known to the skilled artisan.
   Compounds of general formula (i-2) can be transformed into intermediates (i-3). For example, a suitable nucleophile R⁴, such as a thiol, is deprotonated with a suitable base, such as NaH, in a suitable solvent, such as DMF or MeCN at temperatures between 0°C and RT. After a suitable time between 5 and 60 min, intermediate (i-2) is added and the reaction can proceed at temperatures ranging from 0 to 120 °C. Compounds of general type (i-3) may then be isolated and, if necessary and desired, purified using techniques well known in the art, such as chromatography.
   A compound of general formula (i-3) can be transformed into pyrimidinyloxyphenylamidines of the formula (I). For example, a mixture of intermediate (i-3) and a suitable reagent, such as N-(dimethoxymethyl)-N-methyl-ethanamine, in a suitable solvent, such as toluene, is reacted at temperatures ranging from RT to reflux.
   The final compounds of general formula (I) may then be isolated and, if necessary and desired, purified using techniques well known in the art, such as chromatography.
A2: An aminophenol is reacted with a pyrimidine (i-4) to form compounds of formula (i-3). For this, both starting materials, a mixture of a suitable base, such as NaOH in a suitable solvent, such as acetone, are mixed at temperatures ranging from 0 to 100 °C to provide compounds of formula (i-3), which may then be isolated and, if necessary and desired, purified using techniques well known in the art, such as chromatography. The required aminophenols or pyrimidines (i-4) are commercially available or may be synthesized by methods known to the skilled artisan. The following step to achieve the desired compound of formula (I) can be performed analogously to step c) of route A1.
A3: An aminophenol is reacted with a pyrimidine (i-5) to form compounds of formula (i-6). For example, both starting materials, a mixture of a suitable base, such as NaOH in a suitable solvent, such as acetone, are mixed at temperatures ranging from 0 to 100 °C to provide compounds of formula (i-6), which may then be isolated and, if necessary and desired, purified using techniques well known in the art, such as chromatography. The required aminophenols or pyrimidines (i-5) are commercially available or may be synthesized by methods known to the skilled artisan.

Compounds of general formula (i-6) can be transformed into intermediates (i-7). For example, a suitable nucleophile R³, such as a thiol, is deprotonated with a suitable base, such as NaH, in a suitable solvent, such as DMF or MeCN, at temperatures between 0°C and RT. After a suitable time between 5 and 60 min, intermediate (i-6) is added and the reaction can proceed at temperatures ranging from 0 to 120 °C. Compounds of general formula (i-7) may then be isolated and, if necessary and desired, purified using techniques well known in the art, such as chromatography. The following step to achieve the desired compound of formula (I) can be performed analogously to step c) of route A1.

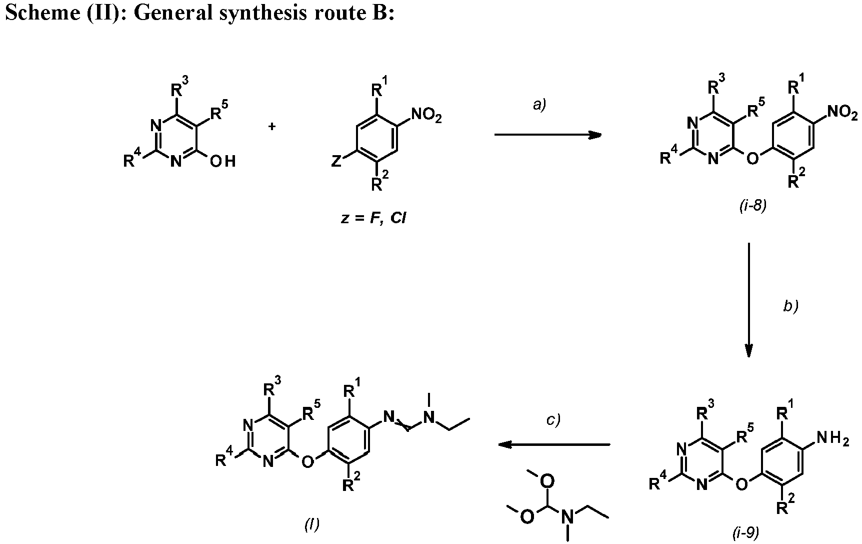

Conditions: a) Cs₂CO₃, DMF, RT; b) SnCl₂, EtOH, 90°C; c) N-(dimethoxymethyl)-N-methyl-ethanamine, toluene, 80°C.

A different access to pyrimidinyloxyphenylamidines of the formula (I) is described in Scheme (II). A pyrimidinol or tautomeric pyrimidinone is reacted with a nitroaromatic compound to yield intermediates of general formula (i-8). For example, the required starting materials, a suitable base, such as Cs₂CO₃, in a suitable solvent, such as DMF or MeCN, are mixed at temperatures ranging from 0 to 100 °C to provide compounds of formula (i-8), which may then be isolated and, if necessary and desired, purified using techniques well known in the art, such as chromatography. The required pyrimidinols or nitroaromatic compounds are commercially available or may be synthesized by methods known to the skilled artisan. The nitroaromatic intermediates of general formula (i-8) can be further reduced to compounds of the general formula (i-9). For this, a mixture of intermediate (i-8), a suitable reductant in a suitable solvent, such as SnCl₂ in EtOH, are reacted at temperatures ranging from 0 °C to reflux to provide compounds of formula (i-9), which may then be isolated and, if necessary and desired, purified using techniques well known in the art, such as chromatography. The last step c) can be performed according to conditions already described in and for Scheme (I).

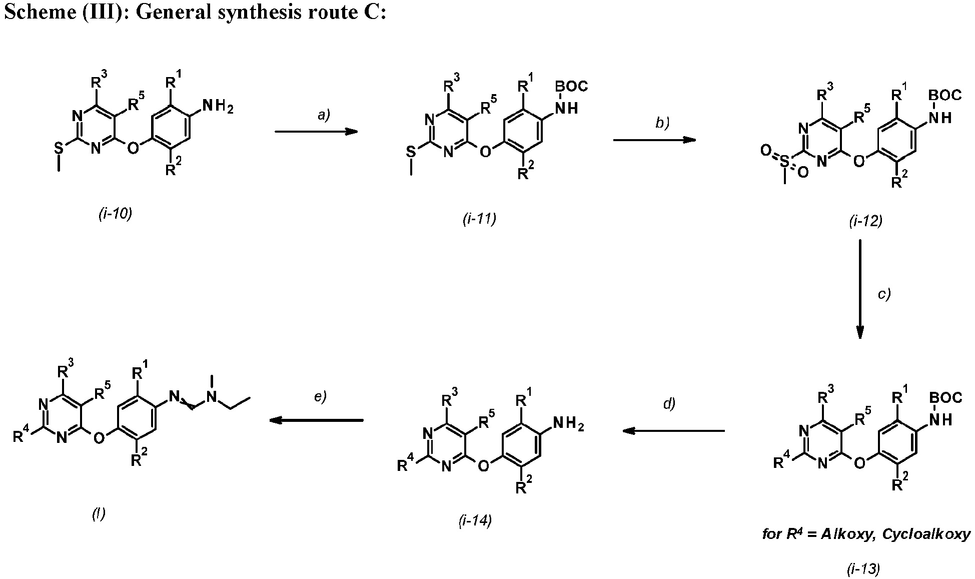

Conditions: a) Boc₂O, CH₂Cl₂, RT; b) *m*CPBA, CH₂Cl₂, RT; c) NaH, R₄-Nucleophile, DMF, RT; d) 4N HCl, 1,4-dioxane, RT; e) N-(dimethoxymethyl)-N-methyl-ethanamine, toluene, 80°C.

The general synthesis route of scheme (III) illustrates the preparation of pyrimidinyloxyphenylamidines of the general formula (I), wherein R⁴ is selected from the groups of C₁-C₆-Alkoxy and C₃-C₈-Cycloalkoxy, as shown e.g. in example 1-041. The synthesis starts with intermediates of the general formula (i-10) which can be prepared in the same way as the compounds of general formula (i-9) according to general synthesis route B (Scheme II). Firstly, a protecting group is inserted. For this, aniline (i-10) is protected as a Boc-carbamate represented by the compounds of general formula (i-11), using Boc₂O in CH₂Cl₂ at RT. Consequently, the methylsulfanyl-group is transformed into a leaving group using *m*CPBA as an oxidant in CH₂Cl₂ at RT to give the intermediate of general formula (i-12). This compound with the activated sulfoxide group is further converted into compounds of general formula (i-13). For example, an oxygen containing nucleophile R⁴ is deprotonated with a suitable base, such as NaH, in a suitable solvent, such as DMF or MeCN at temperatures between 0°C and RT. After a suitable time between 5 and 60 min, intermediate of the general formula (i-12) is added and the reaction can proceed at temperatures ranging from 0 to 60 °C to provide the compounds of the general formula (i-13). In the next step, the Boc-group is removed with HCl 4N in 1,4-dioxane providing the intermediate of the general formula (i-14), which in turn can be converted into the pyrimidinyloxyphenylamidines of general formula (I) by applying the conditions already described for step c) in Scheme (I). If necessary and desired, the example compound can be purified using techniques well known in the art, such as chromatography.

### Compositions/Formulations

The present invention further relates to compositions, in particular compositions for controlling unwanted microorganisms. The composition may be applied to the microorganisms and/or in their habitat.

The composition comprises at least one compound of the invention and at least one agriculturally suitable auxiliary, e.g. carrier(s) and/or surfactant(s).

A carrier is a solid or liquid, natural or synthetic, organic or inorganic substance that is generally inert. The carrier generally improves the application of the compounds, for instance, to plants, plants parts or seeds. Examples of suitable *solid carriers* include, but are not limited to, ammonium salts, in particular ammonium sulfates, ammonium phosphates and ammonium nitrates, natural rock flours, such as kaolins, clays, talc, chalk, quartz, attapulgite, montmorillonite and diatomaceous earth, silica gel and synthetic rock flours, such as finely divided silica, alumina and silicates. Examples of typically useful solid carriers for preparing granules include, but are not limited to crushed and fractionated natural rocks such as calcite, marble, pumice, sepiolite and dolomite, synthetic granules of inorganic and organic flours and granules of organic material such as paper, sawdust, coconut shells, maize cobs and tobacco stalks. Examples of suitable *liquid carriers* include, but are not limited to, water, organic solvents and combinations thereof. Examples of suitable *solvents* include polar and nonpolar organic chemical liquids, for example from the classes of aromatic and nonaromatic hydrocarbons (such as cyclohexane, paraffins, alkylbenzenes, xylene, toluene, tetrahydronaphthalene, alkylnaphthalenes, chlorinated aromatics or chlorinated aliphatic hydrocarbons such as chlorobenzenes, chloroethylenes or methylene chloride), alcohols and polyols (which may optionally also be substituted, etherified and/or esterified, such as ethanol, propanol, butanol, benzylalcohol, cyclohexanol or glycol), ketones (such as acetone, methyl ethyl ketone, methyl isobutyl ketone or cyclohexanone), esters (including fats and oils) and (poly)ethers, unsubstituted and substituted amines, amides (such as dimethylformamide or fatty acid amides) and esters thereof, lactams (such as N-alkylpyrrolidones, in particular N-methylpyrrolidone) and lactones, sulfones and sulfoxides (such as dimethyl sulfoxide), oils of vegetable or animal origin. The carrier may also be a liquefied gaseous extender, i.e. liquid which is gaseous at standard temperature and under standard pressure, for example aerosol propellants such as halohydrocarbons, butane, propane, nitrogen and carbon dioxide.

Preferred solid carriers are selected from clays, talc and silica.

Preferred liquid carriers are selected from water, fatty acid amides and esters thereof, aromatic and nonaromatic hydrocarbons, lactams and carbonic acid esters.

The amount of carrier typically ranges from 1 to 99.99%, preferably from 5 to 99.9%, more preferably from 10 to 99.5%, and most preferably from 20 to 99% by weight of the composition.

Liquid carriers are typically present in a range of from 20 to 90%, for example 30 to 80% by weight of the composition.

Solid carriers are typically present in a range of from 0 to 50%, preferably 5 to 45%, for example 10 to 30% by weight of the composition.

If the composition comprises two or more carriers, the outlined ranges refer to the total amount of carriers.

The surfactant can be an ionic (cationic or anionic), amphoteric or non-ionic surfactant, such as ionic or non-ionic emulsifier(s), foam former(s), dispersant(s), wetting agent(s), penetration enhancer(s) and any mixtures thereof. Examples of suitable surfactants include, but are not limited to, salts of polyacrylic acid, salts of lignosulfonic acid (such as sodium lignosulfonate), salts of phenolsulfonic acid or naphthalenesulfonic acid, polycondensates of ethylene oxide and/or propylene oxide with fatty alcohols, fatty acids or fatty amines (for example, polyoxyethylene fatty acid esters such as castor oil ethoxylate, polyoxyethylene fatty alcohol ethers, for example alkylaryl polyglycol ethers), substituted phenols (preferably alkylphenols or arylphenols) and ethoxylates thereof (such as tristyrylphenol ethoxylate), salts of sulfosuccinic esters, taurine derivatives (preferably alkyl taurates), phosphoric esters of polyethoxylated alcohols or phenols, fatty esters of polyols (such a fatty acid esters of glycerol, sorbitol or sucrose), sulfates (such as alkyl sulfates and alkyl ether sulfates), sulfonates (for example, alkylsulfonates, arylsulfonates and alkylbenzene sulfonates), phosphate esters, protein hydrolysates, lignosulfite waste liquors and methylcellulose. Any reference to salts in this paragraph refers preferably to the respective alkali, alkaline earth and ammonium salts.

Preferred surfactants are selected from polyoxyethylene fatty alcohol ethers, polyoxyethylene fatty acid esters, alkylbenzene sulfonates, such as calcium dodecylbenzenesulfonate, castor oil ethoxylate, sodium lignosulfonate and arylphenol ethoxylates, such as tristyrylphenol ethoxylate.

The amount of surfactants typically ranges from 5 to 40%, for example 10 to 20%, by weight of the composition.

Further examples of suitable auxiliaries include water repellents, siccatives, binders (adhesive, tackifier, fixing agent, such as carboxymethylcellulose, natural and synthetic polymers in the form of powders, granules or latices, such as gum arabic, polyvinyl alcohol and polyvinyl acetate, natural phospholipids such as cephalins and lecithins and synthetic phospholipids, polyvinylpyrrolidone and tylose), thickeners and secondary thickeners (such as cellulose ethers, acrylic acid derivatives, xanthan gum, modified clays, e.g. the products available under the name Bentone, and finely divided silica), stabilizers (e.g. cold stabilizers, preservatives (e.g. dichlorophene and benzyl alcohol hemiformal), antioxidants, light stabilizers, in particular UV stabilizers, or other agents which improve chemical and/or physical stability), dyes or pigments (such as inorganic pigments, e.g. iron oxide, titanium oxide and Prussian Blue; organic dyes, e.g. alizarin, azo and metal phthalocyanine dyes), antifoams (e.g. silicone antifoams and magnesium stearate), antifreezes, stickers, gibberellins and processing auxiliaries, mineral and vegetable oils, perfumes, waxes, nutrients (including trace nutrients, such as salts of iron, manganese, boron, copper, cobalt, molybdenum and zinc), protective colloids, thixotropic substances, penetrants, sequestering agents and complex formers.

The choice of the auxiliaries depends on the intended mode of application of the compound of the invention and/or on the physical properties of the compound(s). Furthermore, the auxiliaries may be chosen to impart particular properties (technical, physical and/or biological properties) to the compositions or use forms prepared therefrom. The choice of auxiliaries may allow customizing the compositions to specific needs.

The composition of the invention may be provided to the end user as ready-for-use formulation, i.e. the compositions may be directly applied to the plants or seeds by a suitable device, such as a spraying or dusting device. Alternatively, the compositions may be provided to the end user in the form of concentrates which have to be diluted, preferably with water, prior to use.

The composition of the invention can be prepared in conventional manners, for example by mixing the compound of the invention with one or more suitable auxiliaries, such as disclosed herein above.

The composition comprises a fungicidally effective amount of the compound(s) of the invention. The term "effective amount" denotes an amount, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound of the invention used. Usually, the composition according to the invention contains from 0.01 to 99% by weight, preferably from 0.05 to 98% by weight, more preferred from 0.1 to 95% by weight, even more preferably from 0.5 to 90% by weight, most preferably from 1 to 80% by weight of the compound of the invention. It is possible that a composition comprises two or more compounds of the invention. In such case the outlined ranges refer to the total amount of compounds of the present invention.

The composition of the invention may be in any customary composition type, such as solutions (e.g aqueous solutions), emulsions, water- and oil-based suspensions, powders (e.g. wettable powders, soluble powders), dusts, pastes, granules (e.g. soluble granules, granules for broadcasting), suspoemulsion concentrates, natural or synthetic products impregnated with the compound of the invention, fertilizers and also microencapsulations in polymeric substances. The compound of the invention may be present in a suspended, emulsified or dissolved form. Examples of particular suitable composition types are solutions, watersoluble concentrates (e.g. SL, LS), dispersible concentrates (DC), suspensions and suspension concentrates (e.g. SC, OD, OF, FS), emulsifiable concentrates (e.g. EC), emulsions (e.g. EW, EO, ES, ME, SE), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GW, GF). These and further compositions types are defined by the Food and Agriculture Organization of the United Nations (FAO). An overview is given in the "Catalogue of pesticide formulation types and international coding system", Technical Monograph No. 2, 6th Ed. May 2008, Croplife International.

Preferably, the composition of the invention is in form of one of the following types: EC, SC, FS, SE, OD and WG, more preferred EC, SC, OD and WG.

Further details about examples of composition types and their preparation are given below. If two or more compounds of the invention are present, the outlined amount of compound of the invention refers to the total amount of compounds of the present invention. This applies mutatis mutandis for any further component of the composition, if two or more representatives of such component, e.g. wetting agent, binder, are present.
i) Water-soluble concentrates (SL, LS)
   10-60 % by weight of at least one compound of the invention and 5-15 % by weight surfactant (e.g. polyoxyethylene fatty alcohol ether) are dissolved in such amount of water and/or water-soluble solvent (e.g. alcohols such as propylene glycol or carbonates such as propylene carbonate) to result in a total amount of 100 % by weight. Before application the concentrate is diluted with water.
ii) Dispersible concentrates (DC)
   5-25 % by weight of at least one compound of the invention and 1-10 % by weight surfactant and/or binder (e.g. polyvinylpyrrolidone) are dissolved in such amount of organic solvent (e.g. cyclohexanone) to result in a total amount of 100 % by weight. Dilution with water gives a dispersion.
iii) Emulsifiable concentrates (EC)
   15-70 % by weight of at least one compound of the invention and 5-10 % by weight surfactant (e.g. a mixture of calcium dodecylbenzenesulfonate and castor oil ethoxylate) are dissolved in such amount of water-insoluble organic solvent (e.g. aromatic hydrocarbon or fatty acid amide) and if needed additional water-soluble solvent to result in a total amount of 100 % by weight. Dilution with water gives an emulsion.
iv) Emulsions (EW, EO, ES)
   5-40 % by weight of at least one compound of the invention and 1-10 % by weight surfactant (e.g. a mixture of calcium dodecylbenzenesulfonate and castor oil ethoxylate) are dissolved in 20-40 % by weight water-insoluble organic solvent (e.g. aromatic hydrocarbon). This mixture is added to such amount of water by means of an emulsifying machine to result in a total amount of 100 % by weight. The resulting composition is a homogeneous emulsion. Before application the emulsion may be further diluted with water.
v) Suspensions and suspension concentrates
   v-1) Water-based (SC, FS)
      In a suitable grinding equipment, e.g. an agitated ball mill, 20-60 % by weight of at least one compound of the invention are comminuted with addition of 2-10 % by weight surfactant (e.g. sodium lignosulfonate and polyoxyethylene fatty alcohol ether), 0.1-2 % by weight thickener (e.g. xanthan gum) and water to give a fine active substance suspension. The water is added in such amount to result in a total amount of 100 % by weight. Dilution with water gives a stable suspension of the active substance. For FS type compositions up to 40 % by weight binder (e.g. polyvinylalcohol) is added.
   v-2) Oil-based (OD, OF)
      In a suitable grinding equipment, e.g. an agitated ball mill, 20-60 % by weight of at least one compound of the invention are comminuted with addition of 2-10 % by weight surfactant (e.g. sodium lignosulfonate and polyoxyethylene fatty alcohol ether), 0.1-2 % by weight thickener (e.g. modified clay, in particular Bentone, or silica) and an organic carrier to give a fine active substance oil suspension. The organic carrier is added in such amount to result in a total amount of 100 % by weight. Dilution with water gives a stable dispersion of the active substance.
vi) Water-dispersible granules and water-soluble granules (WG, SG)
   50-80 % by weight of at least one compound of the invention are ground finely with addition of surfactant (e.g. sodium lignosulfonate and polyoxyethylene fatty alcohol ether) and converted to water-dispersible or water-soluble granules by means of technical appliances (e. g. extrusion, spray tower, fluidized bed). The surfactant is used in such amount to result in a total amount of 100 % by weight. Dilution with water gives a stable dispersion or solution of the active substance.
vii) Water-dispersible powders and water-soluble powders (WP, SP, WS)
   50-80 % by weight of at least one compound of the invention are ground in a rotor-stator mill with addition of 1-8 % by weight surfactant (e.g. sodium lignosulfonate, polyoxyethylene fatty alcohol ether) and such amount of solid carrier, e.g. silica gel, to result in a total amount of 100 % by weight. Dilution with water gives a stable dispersion or solution of the active substance.
viii) Gel (GW, GF)
   In an agitated ball mill, 5-25 % by weight of at least one compound of the invention are comminuted with addition of 3-10 % by weight surfactant (e.g. sodium lignosulfonate), 1-5 % by weight binder (e.g. carboxymethylcellulose) and such amount of water to result in a total amount of 100 % by weight. This results in a fine suspension of the active substance. Dilution with water gives a stable suspension of the active substance.
ix) Microemulsion (ME)
   5-20 % by weight of at least one compound of the invention are added to 5-30 % by weight organic solvent blend (e.g. fatty acid dimethylamide and cyclohexanone), 10-25 % by weight surfactant blend (e.g. polyoxyethylene fatty alcohol ether and arylphenol ethoxylate), and such amount of water to result in a total amount of 100 % by weight. This mixture is stirred for 1 h to produce spontaneously a thermodynamically stable microemulsion.
x) Microcapsules (CS)
   An oil phase comprising 5-50 % by weight of at least one compound of the invention, 0-40 % by weight water-insoluble organic solvent (e.g. aromatic hydrocarbon), 2-15 % by weight acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 % by weight of at least one compound of the invention, 0-40 % by weight water-insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of polyurea microcapsules. The monomers amount to 1-10 % by weight of the total CS composition.
xi) Dustable powders (DP, DS)
   1-10 % by weight of at least one compound of the invention are ground finely and mixed intimately with such amount of solid carrier, e.g. finely divided kaolin, to result in a total amount of 100 % by weight.
xii) Granules (GR, FG)
   0.5-30 % by weight of at least one compound of the invention are ground finely and associated with such amount of solid carrier (e.g. silicate) to result in a total amount of 100 % by weight. Granulation is achieved by extrusion, spray-drying or the fluidized bed.
xiii) Ultra-low volume liquids (UL)
   1-50 % by weight of at least one compound of the invention are dissolved in such amount of organic solvent, e.g. aromatic hydrocarbon, to result in a total amount of 100 % by weight.

The compositions types i) to xiii) may optionally comprise further auxiliaries, such as 0.1-1 % by weight preservatives, 0.1-1 % by weight antifoams, 0.1-1 % by weight dyes and/or pigments, and 5-10% by weight antifreezes.

### Mixtures/Combinations

The compound and the composition of the invention can be mixed with other active ingredients like fungicides, bactericides, acaricides, nematicides, insecticides, biological control agents or herbicides. Mixtures with fertilizers, growth regulators, safeners, nitrification inhibitors, semiochemicals and/or other agriculturally beneficial agents are also possible. This may allow to broaden the activity spectrum or to prevent development of resistance. Examples of known fungicides, insecticides, acaricides, nematicides and bactericides are disclosed in the Pesticide Manual, 17th Edition.

Examples of fungicides which could be mixed with the compound and the composition of the invention are:
1) Inhibitors of the ergosterol biosynthesis, for example (1.001) cyproconazole, (1.002) difenoconazole, (1.003) epoxiconazole, (1.004) fenhexamid, (1.005) fenpropidin, (1.006) fenpropimorph, (1.007) fenpyrazamine, (1.008) fluquinconazole, (1.009) flutriafol, (1.010) imazalil, (1.011) imazalil sulfate, (1.012) ipconazole, (1.013) metconazole, (1.014) myclobutanil, (1.015) paclobutrazol, (1.016) prochloraz, (1.017) propiconazole, (1.018) prothioconazole, (1.019) pyrisoxazole, (1.020) spiroxamine, (1.021) tebuconazole, (1.022) tetraconazole, (1.023) triadimenol, (1.024) tridemorph, (1.025) triticonazole, (1.026) (1R,2S,5 S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)cyclopentanol, (1.028) (2R)-2-(1-chlorocyclopropyl)-4-[(1R)-2,2-dichlorocyclopropyl]-1-(1H-1,2,4-triazol-1-yl)butan-2-ol, (1.029) (2R)-2-(1-chlorocyclopropyl)-4- [(1S)-2,2-dichlorocyclopropyl]-1-(1H-1,2,4-triazol-1-yl)butan-2-ol, (1.030) (2R)-2-[4-(4-chlorophenoxy)-2-(trifluoromethyl)phenyl]-1-(1H-1,2,4-triazol-1-yl)propan-2-ol, (1.031) (2S)-2-(1-chlorocyclopropyl)-4-[(1R)-2,2-dichlorocyclopropyl]-1-(1H-1,2,4-triazol-1-yl)butan-2-ol, (1.032) (2S)-2-(1-chloro-cyclopropyl)-4-[(1S)-2,2-dichlorocyclopropyl]-1-(1H-1,2,4-triazol-1-yl)butan-2-ol, (1.033) (2S)-2-[4-(4-chlorophenoxy)-2-(trifluoromethyl)phenyl]-1-(1H-1,2,4-triazol-1-yl)propan-2-ol, (1.034) (R)-[3-(4-chloro-2-fluorophenyl)-5-(2,4-difluorophenyl)-1,2-oxazol-4-yl](pyridin-3-yl)methanol, (1.035) (S)-[3-(4-chloro-2-fluorophenyl)-5-(2,4-difluorophenyl)-1,2-oxazol-4-yl](pyridin-3-yl)methanol, (1.036) [3-(4-chloro-2-fluorophenyl)-5-(2,4-difluorophenyl)-1,2-oxazol-4-yl](pyridin-3-yl)methanol, (1.037) 1-({(2R,4S)-2-[2-chloro-4-(4-chlorophenoxy)phenyl]-4-methyl-1,3-dioxolan-2-yl}methyl)-1H-1,2,4-triazole, (1.038) 1-({(2S,4S)-2-[2-chloro-4-(4-chlorophenoxy)phenyl]-4-methyl-1,3-dioxolan-2-yl}methyl)-1H-1,2,4-triazole, (1.039) 1-{[3-(2-chlorophenyl)-2-(2,4-difluorophenyl)oxiran-2-yl]methyl}-1H-1,2,4-triazol-5-yl thiocyanate, (1.040) 1-{[rel(2R,3R)-3-(2-chlorophenyl)-2-(2,4-difluorophenyl)oxiran-2-yl]methyl}-1H-1,2,4-triazol-5-yl thiocyanate, (1.041) 1-{[rel(2R,3S)-3-(2-chlorophenyl)-2-(2,4-difluorophenyl)oxiran-2-yl]methyl}-1H-1,2,4-triazol-5-yl thiocyanate, (1.042) 2-[(2R,4R,5R)-1-(2,4-dichlorophenyl)-5-hydroxy-2,6,6-trimethylheptan-4-yl]-2,4-dihydro-3H-1,2,4-triazole-3-thione, (1.043) 2-[(2R,4R,5S)-1-(2,4-dichlorophenyl)-5-hydroxy-2,6,6-trimethylheptan-4-yl]-2,4-dihydro-3H-1,2,4-triazole-3-thione, (1.044) 2-[(2R,4S,5R)-1-(2,4-dichlorophenyl)-5-hydroxy-2,6,6-trimethylheptan-4-yl]-2,4-dihydro-3H-1,2,4-triazole-3-thione, (1.045) 2-[(2R,4S,5S)-1-(2,4-dichloro-phenyl)-5-hydroxy-2,6,6-trimethylheptan-4-yl]-2,4-dihydro-3H-l,2,4-triazole-3-thione, (1.046) 2-[(2S,4R,5R)-1-(2,4-dichlorophenyl)-5-hydroxy-2,6,6-trimethylheptan-4-yl]-2,4-dihydro-3H-1,2,4-triazole-3-thione, (1.047) 2-[(2S,4R,5S)-1-(2,4-dichlorophenyl)-5-hydroxy-2,6,6-trimethylheptan-4-yl]-2,4-dihydro-3H-1,2,4-triazole-3-thione, (1.048) 2-[(2S,4S,5R)-1-(2,4-dichlorophenyl)-5-hydroxy-2,6,6-trimethylheptan-4-yl]-2,4-dihydro-3H-1,2,4-triazole-3-thione, (1.049) 2-[(2S,4S,5S)-1-(2,4-dichlorophenyl)-5-hydroxy-2,6,6-trimethylheptan-4-yl]-2,4-dihydro-3H-1,2,4-triazole-3-thione, (1.050) 2-[1-(2,4-dichlorophenyl)-5-hydroxy-2,6,6-trimethylheptan-4-yl]-2,4-dihydro-3H-1,2,4-triazole-3-thione, (1.051) 2-[2-chloro-4-(2,4-dichlorophenoxy)phenyl]-1-(1H-1,2,4-triazol-1-yl)propan-2-ol, (1.052) 2-[2-chloro-4-(4-chlorophenoxy)phenyl]-1-(1H-1,2,4-triazol-1-yl)butan-2-ol, (1.053) 2-[4-(4-chlorophenoxy)-2-(trifluoromethyl)phenyl]-1-(1H-1,2,4-triazol-1-yl)butan-2-ol, (1.054) 2-[4-(4-chlorophenoxy)-2-(trifluoromethyl)phenyl]-1-(1H-1,2,4-triazol-1-yl)pentan-2-ol, (1.055) mefentrifluconazole, (1.056) 2-{[3-(2-chlorophenyl)-2-(2,4-difluorophenyl)oxiran-2-yl]methyl}-2,4-dihydro-3H-1,2,4-triazole-3-thione, (1.057) 2-{[rel(2R,3R)-3-(2-chlorophenyl)-2-(2,4-difluorophenyl)oxiran-2-yl]methyl} -2,4-dihydro-3H-1,2,4-triazole-3 -thione, (1.058) 2-{[rel(2R,3S)-3-(2-chlorophenyl)-2-(2,4-difluorophenyl)oxiran-2-yl]methyl}-2,4-dihydro-3H-1,2,4-triazole-3-thione, (1.059) 5-(4-chlorobenzyl)-2-(chloromethyl)-2-methyl-1-(1H-1,2,4-triazol-1-ylmethyl)cyclopentanol, (1.060) 5-(allylsulfanyl)-1-{[3-(2-chlorophenyl)-2-(2,4-difluorophenyl)oxiran-2-yl]methyl}-1H-1,2,4-triazole, (1.061) 5-(allylsulfanyl)-1-{[rel(2R,3R)-3-(2-chlorophenyl)-2-(2,4-difluorophenyl)oxiran-2-yl]methyl}-1H-1,2,4-triazole, (1.062) 5-(allylsulfanyl)-1-{[rel(2R,3S)-3-(2-chlorophenyl)-2-(2,4-difluorophenyl)oxiran-2-yl]methyl}-1H-1,2,4-triazole, (1.063) N'-(2,5-dimethyl-4-{[3-(1,1,2,2-tetrafluoroethoxy)phenyl]sulfanyl}phenyl)-N-ethyl-N-methylimidoformamide, (1.064) N'-(2,5-dimethyl-4-{ [3-(2,2,2-trifluoroethoxy)phenyl]sulfanyl}phenyl)-N-ethyl-N-methylimidoformamide, (1.065) N'-(2,5-dimethyl-4-{[3-(2,2,3,3-tetrafluoropropoxy)phenyl]sulfanyl}phenyl)-N-ethyl-N-methylimidoformamide, (1.066) N'-(2,5-dimethyl-4-{[3-(pentafluoroethoxy)phenyl]sulfanyl}phenyl)-N-ethyl-N-methylimidoformamide, (1.067) N'-(2,5-dimethyl-4-{3-[(1,1,2,2-tetrafluoroethyl)sulfanyl]-phenoxy}phenyl)-N-ethyl-N-methylimidoformamide, (1.068) N'-(2,5-dimethyl-4-{3-[(2,2,2-trifluoroethyl)sulfanyl]phenoxy}phenyl)-N-ethyl-N-methylimidoformamide, (1.069) N'-(2,5-dimethyl-4-{3-[(2,2,3,3-tetrafluoropropyl)sulfanyl]phenoxy}phenyl)-N-ethyl-N-methylimidoformamide, (1.070) N'-(2,5-dimethyl-4-{3-[(pentafluoroethyl)sulfanyl]phenoxy}phenyl)-N-ethyl-N-methylimidoformamide, (1.071) N'-(2,5-dimethyl-4-phenoxyphenyl)-N-ethyl-N-methylimidoformamide, (1.072) N'-(4-{[3-(difluoromethoxy)phenyl]sulfanyl}-2,5-dimethylphenyl)-N-ethyl-N-methylimidoformamide, (1.073) N'-(4-{3-[(difluoromethyl)sulfanyl]phenoxy}-2,5-dimethylphenyl)-N-ethyl-N-methylimidoformamide, (1.074) N'-[5-bromo-6-(2,3-dihydro-1H-inden-2-yloxy)-2-methylpyridin-3-yl]-N-ethyl-N-methylimidoformamide, (1.075) N'-{4-[(4,5-dichloro-1,3-thiazol-2-yl)oxy]-2,5-dimethylphenyl}-N-ethyl-N-methylimidoformamide, (1.076) N'-{5-bromo-6-[(1R)-1-(3,5-difluorophenyl)ethoxy]-2-methylpyridin-3-yl}-N-ethyl-N-methylimidoformamide, (1.077) N'-{5-bromo-o-[(1S)-1-(3,5-difluorophenyl)ethoxy]-2-methylpyridin-3-yl}-N-ethyl-N-methylimidoformamide, (1.078) N'-{5-bromo-6-[(cis-4-isopropyl-cyclohexyl)oxy]-2-methylpyridin-3-yl}-N-ethyl-N-methylimidoformamide, (1.079) N'-{5-bromo-6-[(trans-4-isopropylcyclohexyl)oxy]-2-methylpyridin-3-yl}-N-ethyl-N-methylimidoformamide, (1.080) N'-{5-bromo-6-[1-(3,5-difluorophenyl)ethoxy]-2-methylpyridin-3-yl}-N-ethyl-N-methylimidoformamide, (1.081) ipfentrifluconazole, (1.082) 2-[4-(4-chlorophenoxy)-2-(trifluoromethyl)phenyl]-1-(1H-1,2,4-triazol-1-yl)propan-2-ol, (1.083) 2-[6-(4-bromophenoxy)-2-(trifluoromethyl)-3-pyridyl]-1-(1,2,4-triazol-1-yl)propan-2-ol, (1.084) 2-[6-(4-chlorophenoxy)-2-(trifluoromethyl)-3-pyridyl]-1-(1,2,4-triazol-1-yl)propan-2-ol, (1.085) 3-[2-(1-chlorocyclopropyl)-3-(3-chloro-2-fluoro-phenyl)-2-hydroxypropyl]imidazole-4-carbonitrile, (1.086) 4-[[6-[rac-(2R)-2-(2,4-difluorophenyl)-1,1-difluoro-2-hydroxy-3-(5-thioxo-4H-1,2,4-triazol-1-yl)propyl]-3-pyridyl]oxy]benzonitrile, (1.087) N-isopropyl-N'-[5-methoxy-2-methyl-4-(2,2,2-trifluoro-1-hydroxy-1-phenylethyl)phenyl]-N-methylimidoformamide, (1.088) N'-{5-bromo-2-methyl-6-[(1-propoxypropan-2-yl)oxy]pyridin-3-yl}-N-ethyl-N-methylimidoformamide, (1.089) hexaconazole, (1.090) penconazole, (1.091) fenbuconazole.
2) Inhibitors of the respiratory chain at complex I or II, for example (2.001) benzovindiflupyr, (2.002) bixafen, (2.003) boscalid, (2.004) carboxin, (2.005) fluopyram, (2.006) flutolanil, (2.007) fluxapyroxad, (2.008) furametpyr, (2.009) Isofetamid, (2.010) isopyrazam (anti-epimeric enantiomer 1R,4S,9S), (2.011) isopyrazam (anti-epimeric enantiomer 1S,4R,9R), (2.012) isopyrazam (anti-epimeric racemate 1RS,4SR,9SR), (2.013) isopyrazam (mixture of syn-epimeric racemate 1RS,4SR,9RS and anti-epimeric racemate 1RS,4SR,9SR), (2.014) isopyrazam (syn-epimeric enantiomer 1R,4S,9R), (2.015) isopyrazam (syn-epimeric enantiomer 1S,4R,9S), (2.016) isopyrazam (syn-epimeric racemate 1RS,4SR,9RS), (2.017) penflufen, (2.018) penthiopyrad, (2.019) pydiflumetofen, (2.020) Pyraziflumid, (2.021) sedaxane, (2.022) 1,3-dimethyl-N-(1,1,3-trimethyl-2,3-dihydro-1H-inden-4-yl)-1H-pyrazole-4-carboxamide, (2.023) 1,3-dimethyl-N-[(3R)-1,1,3-trimethyl-2,3-dihydro-1H-inden-4-yl]-1H-pyrazole-4-carboxamide, (2.024) 1,3-dimethyl-N-[(3S)-1,1,3-trimethyl-2,3-dihydro-1H-inden-4-yl]-1H-pyrazole-4-carboxamide, (2.025) 1-methyl-3-(trifluoromethyl)-N-[2'-(trifluoromethyl)biphenyl-2-yl]-1H-pyrazole-4-carboxamide, (2.026) 2-fluoro-6-(trifluoromethyl)-N-(1,1,3-trimethyl-2,3-dihydro-1H-inden-4-yl)benzamide, (2.027) 3-(difluoromethyl)-1-methyl-N-(1,1,3-trimethyl-2,3-dihydro-1H-inden-4-yl)-1H-pyrazole-4-carboxamide, (2.028) inpyrfluxam, (2.029) 3-(difluoromethyl)-1-methyl-N-[(3S)-1,1,3-trimethyl-2,3-dihydro-1H-inden-4-yl]-1H-pyrazole-4-carboxamide, (2.030) fluindapyr, (2.031) 3-(difluoromethyl)-N-[(3R)-7-fluoro-1,1,3-trimethyl-2,3-dihydro-1H-inden-4-yl]-1-methyl-1H-pyrazole-4-carboxamide, (2.032) 3-(difluoromethyl)-N-[(3S)-7-fluoro-1,1,3-trimethyl-2,3-dihydro-1H-inden-4-yl]-1-methyl-1H-pyrazole-4-carboxamide, (2.033) 5,8-difluoro-N-[2-(2-fluoro-4-{[4-(trifluoromethyl)pyridin-2-yl]oxy}phenyl)ethyl]quinazolin-4-amine, (2.034) N-(2-cyclopentyl-5-fluorobenzyl)-N-cyclopropyl-3-(difluoromethyl)-5-fluoro-1-methyl-1H-pyrazole-4-carboxamide, (2.035) N-(2-tert-butyl-5-methylbenzyl)-N-cyclopropyl-3-(difluoromethyl)-5-fluoro-1-methyl-1H-pyrazole-4-carboxamide, (2.036) N-(2-tert-butylbenzyl)-N-cyclopropyl-3-(difluoromethyl)-5-fluoro-1-methyl-1H-pyrazole-4-carboxamide, (2.037) N-(5-chloro-2-ethylbenzyl)-N-cyclopropyl-3-(difluoromethyl)-5-fluoro-1-methyl-1H-pyrazole-4-carboxamide, (2.038) isoflucypram, (2.039) N-[(1R,4S)-9-(dichloromethylene)-1,2,3,4-tetrahydro-1,4-methanonaphthalen-5-yl]-3-(difluoromethyl)-1-methyl-1H-pyrazole-4-carboxamide, (2.040) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide, (2.041) N-[1-(2,4-dichlorophenyl)-1-methoxypropan-2-yl]-3-(difluoromethyl)-1-methyl-1H-pyrazole-4-carboxamide, (2.042) N-[2-chloro-6-(trifluoromethyl)benzyl]-N-cyclopropyl-3-(difluoromethyl)-5-fluoro-1-methyl-1H-pyrazole-4-carboxamide, (2.043) N-[3-chloro-2-fluoro-6-(trifluoromethyl)benzyl]-N-cyclopropyl-3-(difluoromethyl)-5-fluoro-1-methyl-1H-pyrazole-4-carboxamide, (2.044) N-[5-chloro-2-(trifluoromethyl)benzyl]-N-cyclopropyl-3-(difluoromethyl)-5-fluoro-1-methyl-1H-pyrazole-4-carboxamide, (2.045) N-cyclopropyl-3-(difluoromethyl)-5-fluoro-1-methyl-N-[5-methyl-2-(trifluoromethyl)benzyl]-1H-pyrazole-4-carboxamide, (2.046) N-cyclopropyl-3-(difluoromethyl)-5-fluoro-N-(2-fluoro-6-isopropylbenzyl)-1-methyl-1H-pyrazole-4-carboxamide, (2.047) N-cyclopropyl-3-(difluoromethyl)-5-fluoro-N-(2-isopropyl-5-methylbenzyl)-1-methyl-1H-pyrazole-4-carboxamide, (2.048) N-cyclopropyl-3-(difluoromethyl)-5-fluoro-N-(2-isopropylbenzyl)-1-methyl-1H-pyrazole-4-carbothioamide, (2.049) N-cyclopropyl-3-(difluoromethyl)-5-fluoro-N-(2-isopropylbenzyl)-1-methyl-1H-pyrazole-4-carboxamide, (2.050) N-cyclopropyl-3-(difluoromethyl)-5-fluoro-N-(5-fluoro-2-isopropylbenzyl)-1-methyl-1H-pyrazole-4-carboxamide, (2.051) N-cyclopropyl-3-(difluoromethyl)-N-(2-ethyl-4,5-dimethylbenzyl)-5-fluoro-1-methyl-1H-pyrazole-4-carboxamide, (2.052) N-cyclopropyl-3-(difluoromethyl)-N-(2-ethyl-5-fluorobenzyl)-5-fluoro-1-methyl-1H-pyrazole-4-carboxamide, (2.053) N-cyclopropyl-3-(difluoromethyl)-N-(2-ethyl-5-methylbenzyl)-5-fluoro-1-methyl-1H-pyrazole-4-carboxamide, (2.054) N-cyclopropyl-N-(2-cyclopropyl-5-fluorobenzyl)-3-(difluoromethyl)-5-fluoro-1-methyl-1H-pyrazole-4-carboxamide, (2.055) N-cyclopropyl-N-(2-cyclopropyl-5-methylbenzyl)-3-(difluoromethyl)-5-fluoro-1-methyl-1H-pyrazole-4-carboxamide, (2.056) N-cyclopropyl-N-(2-cyclopropylbenzyl)-3-(difluoromethyl)-5-fluoro-1-methyl-1H-pyrazole-4-carboxamide, (2.057) pyrapropoyne, (2.058) N-[rac-(1S,2S)-2-(2,4-dichlorophenyl)cyclobutyl]-2-(trifluoromethyl)-nicotinamide, (2.059) N-[(1S,2S)-2-(2,4-dichlorophenyl)cyclobutyl]-2-(trifluoromethyl)nicotinamide.
3) Inhibitors of the respiratory chain at complex III, for example (3.001) ametoctradin, (3.002) amisulbrom, (3.003) azoxystrobin, (3.004) coumethoxystrobin, (3.005) coumoxystrobin, (3.006) cyazofamid, (3.007) dimoxystrobin, (3.008) enoxastrobin, (3.009) famoxadone, (3.010) fenamidone, (3.011) flufenoxystrobin, (3.012) fluoxastrobin, (3.013) kresoxim-methyl, (3.014) metominostrobin, (3.015) orysastrobin, (3.016) picoxystrobin, (3.017) pyraclostrobin, (3.018) pyrametostrobin, (3.019) pyraoxystrobin, (3.020) trifloxystrobin, (3.021) (2E)-2-{2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, (3.022) (2E,3Z)-5-{[1-(4-chlorophenyl)-1H-pyrazol-3-yl]oxy}-2-(methoxyimino)-N,3-dimethylpent-3-enamide, (3.023) (2R)-2-{2-[(2,5-dimethylphenoxy)methyl]phenyl}-2-methoxy-N-methylacetamide, (3.024) (2S)-2-{2-[(2,5-dimethylphenoxy)methyl]phenyl}-2-methoxy-N-methylacetamide, (3.025) fenpicoxamid, (3.026) mandestrobin, (3.027) N-(3-ethyl-3,5,5-trimethylcyclohexyl)-3-formamido-2-hydroxybenzamide, (3.028) (2E,3Z)-5-{[1-(4-chloro-2-fluorophenyl)-1H-pyrazol-3-yl]oxy}-2-(methoxyimino)-N,3-dimethylpent-3-enamide, (3.029) methyl {5-[3-(2,4-dimethylphenyl)-1H-pyrazol-1-yl]-2-methylbenzyl}carbamate, (3.030) metyltetraprole, (3.031) florylpicoxamid.
4) Inhibitors of the mitosis and cell division, for example (4.001) carbendazim, (4.002) diethofencarb, (4.003) ethaboxam, (4.004) fluopicolide, (4.005) pencycuron, (4.006) thiabendazole, (4.007) thiophanate-methyl, (4.008) zoxamide, (4.009) pyridachlometyl, (4.010) 3-chloro-5-(4-chlorophenyl)-4-(2,6-difluorophenyl)-6-methylpyridazine, (4.011) 3-chloro-5-(6-chloropyridin-3-yl)-6-methyl-4-(2,4,6-trifluorophenyl)pyridazine, (4.012) 4-(2-bromo-4-fluorophenyl)-N-(2,6-difluorophenyl)-1,3-dimethyl-1H-pyrazol-5-amine, (4.013) 4-(2-bromo-4-fluorophenyl)-N-(2-bromo-6-fluorophenyl)-1,3-dimethyl-1H-pyrazol-5-amine, (4.014) 4-(2-bromo-4-fluorophenyl)-N-(2-bromophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.016) 4-(2-bromo-4-fluorophenyl)-N-(2-chlorophenyl)-1,3-dimethyl-1H-pyrazol-5-amine, (4.017) 4-(2-bromo-4-fluorophenyl)-N-(2-fluorophenyl)-1,3-dimethyl-1H-pyrazol-5-amine, (4.018) 4-(2-chloro-4-fluorophenyl)-N-(2,6-difluorophenyl)-1,3-dimethyl-1H-pyrazol-5-amine, (4.019) 4-(2-chloro-4-fluorophenyl)-N-(2-chloro-6-fluorophenyl)-1,3-dimethyl-1H-pyrazol-5-amine, (4.020) 4-(2-chloro-4-fluorophenyl)-N-(2-chlorophenyl)-1,3-dimethyl-1H-pyrazol-5-amine, (4.021) 4-(2-chloro-4-fluorophenyl)-N-(2-fluorophenyl)-1,3-dimethyl-1H-pyrazol-5-amine, (4.022) 4-(4-chlorophenyl)-5-(2,6-difluorophenyl)-3,6-dimethylpyridazine, (4.023) N-(2-bromo-6-fluorophenyl)-4-(2-chloro-4-fluorophenyl)-1,3-dimethyl-1H-pyrazol-5-amine, (4.024) N-(2-bromophenyl)-4-(2-chloro-4-fluorophenyl)-1,3-dimethyl-1H-pyrazol-5-amine, (4.025) N-(4-chloro-2,6-difluorophenyl)-4-(2-chloro-4-fluorophenyl)-1,3-dimethyl-1H-pyrazol-5-amine, (4.026) fluopimomide.
5) Compounds capable to have a multisite action, for example (5.001) bordeaux mixture, (5.002) captafol, (5.003) captan, (5.004) chlorothalonil, (5.005) copper hydroxide, (5.006) copper naphthenate, (5.007) copper oxide, (5.008) copper oxychloride, (5.009) copper(2+) sulfate, (5.010) dithianon, (5.011) dodine, (5.012) folpet, (5.013) mancozeb, (5.014) maneb, (5.015) metiram, (5.016) metiram zinc, (5.017) oxine-copper, (5.018) propineb, (5.019) sulfur and sulfur preparations including calcium poly sulfide, (5.020) thiram, (5.021) zineb, (5.022) ziram, (5.023) 6-ethyl-5,7-dioxo-6,7-dihydro-5H-pyrrolo[3',4':5,6][1,4]dithiino[2,3-c][1,2]thiazole-3-carbonitrile.
6) Compounds capable to induce a host defence, for example (6.001) acibenzolar-S-methyl, (6.002) isotianil, (6.003) probenazole, (6.004) tiadinil.
7) Inhibitors of the amino acid and/or protein biosynthesis, for example (7.001) cyprodinil, (7.002) kasugamycin, (7.003) kasugamycin hydrochloride hydrate, (7.004) oxytetracycline, (7.005) pyrimethanil, (7.006) 3-(5-fluoro-3,3,4,4-tetramethyl-3,4-dihydroisoquinolin-1-yl)quinoline.
8) Inhibitors of the ATP production, for example (8.001) silthiofam.
9) Inhibitors of the cell wall synthesis, for example (9.001) benthiavalicarb, (9.002) dimethomorph, (9.003) flumorph, (9.004) iprovalicarb, (9.005) mandipropamid, (9.006) pyrimorph, (9.007) valifenalate, (9.008) (2E)-3-(4-tert-butylphenyl)-3-(2-chloropyridin-4-yl)-1-(morpholin-4-yl)prop-2-en-1-one, (9.009) (2Z)-3-(4-tert-butylphenyl)-3-(2-chloropyridin-4-yl)-1-(morpholin-4-yl)prop-2-en-1-one.
10) Inhibitors of the lipid and membrane synthesis, for example (10.001) propamocarb, (10.002) propamocarb hydrochloride, (10.003) tolclofos-methyl.
11) Inhibitors of the melanin biosynthesis, for example (11.001) tricyclazole, (11.002) tolprocarb.
12) Inhibitors of the nucleic acid synthesis, for example (12.001) benalaxyl, (12.002) benalaxyl-M (kiralaxyl), (12.003) metalaxyl, (12.004) metalaxyl-M (mefenoxam).
13) Inhibitors of the signal transduction, for example (13.001) fludioxonil, (13.002) iprodione, (13.003) procymidone, (13.004) proquinazid, (13.005) quinoxyfen, (13.006) vinclozolin.
14) Compounds capable to act as an uncoupler, for example (14.001) fluazinam, (14.002) meptyldinocap.
15) Further fungicides selected from the group consisting of (15.001) abscisic acid, (15.002) benthiazole, (15.003) bethoxazin, (15.004) capsimycin, (15.005) carvone, (15.006) chinomethionat, (15.007) cufraneb, (15.008) cyflufenamid, (15.009) cymoxanil, (15.010) cyprosulfamide, (15.011) flutianil, (15.012) fosetyl-aluminium, (15.013) fosetyl-calcium, (15.014) fosetyl-sodium, (15.015) methyl isothiocyanate, (15.016) metrafenone, (15.017) mildiomycin, (15.018) natamycin, (15.019) nickel dimethyldithiocarbamate, (15.020) nitrothal-isopropyl, (15.021) oxamocarb, (15.022) oxathiapiprolin, (15.023) oxyfenthiin, (15.024) pentachlorophenol and salts, (15.025) phosphorous acid and its salts, (15.026) propamocarbfosetylate, (15.027) pyriofenone (chlazafenone), (15.028) tebufloquin, (15.029) tecloftalam, (15.030) tolnifanide, (15.031) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.032) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.033) 2-(6-benzylpyridin-2-yl)quinazoline, (15.034) dipymetitrone, (15.035) 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone, (15.036) 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone, (15.037) 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone, (15.038) 2-[6-(3-fluoro-4-methoxyphenyl)-5-methylpyridin-2-yl]quinazoline, (15.039) 2-{(5R)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.040) 2-{(5S)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.041) ipflufenoquin, (15.042) 2-{2-fluoro-6-[(8-fluoro-2-methylquinolin-3-yl)oxy]phenyl}propan-2-ol, (15.043) fluoxapiprolin, (15.044) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}phenyl methanesulfonate, (15.045) 2-phenylphenol and salts, (15.046) 3-(4,4,5-trifluoro-3,3-dimethyl-3,4-dihydroisoquinolin-1-yl)quinoline, (15.047) quinofumelin, (15.048) 4-amino-5-fluoropyrimidin-2-ol (tautomeric form: 4-amino-5-fluoropyrimidin-2(1H)-one), (15.049) 4-oxo-4-[(2-phenylethyl)amino]butanoic acid, (15.050) 5-amino-1,3,4-thiadiazole-2-thiol, (15.051) 5-chloro-N'-phenyl-N'-(prop-2-yn-1-yl)thiophene-2-sulfonohydrazide, (15.052) 5-fluoro-2-[(4-fluorobenzyl)oxy]-pyrimidin-4-amine, (15.053) 5-fluoro-2-[(4-methylbenzyl)oxy]pyrimidin-4-amine, (15.054) 9-fluoro-2,2-dimethyl-5-(quinolin-3-yl)-2,3-dihydro-1,4-benzoxazepine, (15.055) but-3-yn-1-yl{6-[({[(Z)-(1-methyl-1H-tetrazol-5-yl)(phenyl)methylene]amino}oxy)methyl]pyridin-2-yl}carbamate, (15.056) ethyl (2Z)-3-amino-2-cyano-3-phenylacrylate, (15.057) phenazine-1-carboxylic acid, (15.058) propyl 3,4,5-trihydroxybenzoate, (15.059) quinolin-8-ol, (15.060) quinolin-8-ol sulfate (2:1), (15.061) tert-butyl {6-[({[(1-methyl-1H-tetrazol-5-yl)(phenyl)methylene]amino}oxy)methyl]pyridin-2-yl}carbamate, (15.062) 5-fluoro-4-imino-3-methyl-1-[(4-methylphenyl)sulfonyl]-3,4-dihydropyrimidin-2(1H)-one, (15.063) aminopyrifen, (15.064) (N'-[2-chloro-4-(2-fluorophenoxy)-5-methylphenyl]-N-ethyl-N-methylimidoformamide), (15.065) (N'-(2-chloro-5-methyl-4-phenoxyphenyl)-N-ethyl-N-methylimidoformamide), (15.066) (2-{2-[(7,8-difluoro-2-methylquinolin-3-yl)oxy]-6-fluorophenyl}propan-2-ol), (15.067) (5-bromo-1-(5,6-dimethylpyridin-3-yl)-3,3-dimethyl-3,4-dihydroisoquinoline), (15.068) (3-(4,4-difluoro-5,5 -dimethyl-4,5 -dihydrothieno[2,3 -c]pyridin-7-yl)quinoline), (15.069) (1-(4,5-dimethyl-1H-benzimidazol-1-yl)-4,4-difluoro-3,3-dimethyl-3,4-dihydroisoquinoline), (15.070) 8-fluoro-3-(5-fluoro-3,3-dimethyl-3,4-dihydroisoquinolin-1-yl)quinolone, (15.071) 8-fluoro-3-(5-fluoro-3,3,4,4-tetramethyl-3,4-dihydroisoquinolin-1-yl)quinolone, (15.072) 3-(4,4-difluoro-3,3-dimethyl-3,4-dihydroisoquinolin-1-yl)-8-fluoroquinoline, (15.073) (N-methyl-N-phenyl-4-[5-(trifluoromethyl)-1,2,4-oxadiazol-3-yl]benzamide), (15.074) methyl {4-[5-(trifluoromethyl)-1,2,4-oxadiazol-3-yl]phenyl}carbamate, (15.075) (N-{4-[5-(trifluoromethyl)-1,2,4-oxadiazol-3-yl]benzyl}cyclopropanecarboxamide), (15.076) N-methyl-4-(5-(trifluoromethyl)-1,2,4-oxadiazol-3-yl]benzamide, (15.077) N-[(E)-methoxyimino-methyl]-4-[5-(trifluoromethyl)-1,2,4-oxadiazol-3-yl]benzamide, (15.078) N-[(Z)-methoxyiminomethyl]-4-[5-(trifluoromethyl)-1,2,4-oxadiazol-3-yl]benzamide, (15.079) N-[4-[5-(trifluoromethyl)-1,2,4-oxadiazol-3-yl]phenyl]cyclopropanecarboxamide, (15.080) N-(2-fluorophenyl)-4-[5-(trifluoromethyl)-1,2,4-oxadiazol-3-yl]benzamide, (15.081) 2,2-difluoro-N-methyl-2-[4-[5-(trifluoromethyl)-1,2,4-oxadiazol-3-yl]phenyl]acetamide, (15.082) N-allyl-N-[[4-[5-(trifluoromethyl)-1,2,4-oxadiazol-3-yl)phenyl]methyl]acetamide, (15.083) N-[(E)-N-methoxy-C-methyl-carbonimidoyl]-4-(5-(trifluoromethyl)-1,2,4-oxadiazol-3-yl]benzamide, (15.084) N-[(Z)-N-methoxy-C-methyl-carbonimidoyl]-4-[5-(trifluoromethyl)-1,2,4-oxadiazol-3-yl]benzamide, (15.085) N-allyl-N-[[4-[5-(trifluoromethyl)-1,2,4-oxadiazol-3-yl]phenyl]methyl]propanamide, (15.086) 4,4-dimethyl-1-[[4-[5-(trifluoromethyl)-1,2,4-oxadiazol-3-yl]phenyl]methyl]pyrrolidin-2-one, (15.087) N-methyl-4-[5-(trifluoromethyl)-1,2,4-oxadiazol-3-yl]benzenecarbothioamide, (15.088) 5-methyl-1-[[4-[5-(trifluoromethyl)-1,2,4-oxadiazol-3-yl]phenyl]methyl]pyrrolidin-2-one, (15.089) N-((2,3-difluoro-4-[5-(trifluoromethyl)-1,2,4-oxadiazol-3-yl]phenyl]methyl]-3,3,3-trifluoro-propanamide, (15.090) 1-methoxy-1-methyl-3-[[4-[5-(trifluoromethyl}-1,2,4-oxadiazol-3-yl]phenyl]methyl]urea, (15.091) 1,1-diethyl-3-[[4-[5-(trifluoromethyl}-1,2,4-oxadiazol-3-yl]phenyl]methyl]urea, (15.092) N-[[4-[5-(trifluoromethyl)-1,2,4-oxadiazol-3-yl]phenyl]methyl]propanamide, (15.093) N-methoxy-N-[[4-[5-(trifluoromethyl)-1,2,4-oxadiazol-3-yl]phenyl]-methyl]cyclopropanecarboxamide, (15.094) 1-methoxy-3-methyl-1-[[4-[5-(trifluoromethyl)-1,2,4-oxadiazol-3-yl]phenyl]methyl]urea, (15.095) N-methoxy-N-[[4-[5-(trifluoromethyl)-1,2,4-oxadiazol-3-yl]phenyl]methyl)cyclopropanecarboxamide, (15.096) N,2-dimethoxy-N-[[4-[5-(trifluoromethyl}-1,2,4-oxadiazol-3-yl]phenyl]methyl]propanamide, (15.097) N-ethyl-2-methyl-N-[[4-[5-(trifluoromethyl)-1,2,4-oxadiazol-3-yl)phenyl]methyl]propanamide, (15.098) 1-methoxy-3-methyl-1-[[4-[5-(trifluoromethyl)-1,2,4-oxadiazol-3-yl]phenyl]methyl]urea, (15.099) 1,3-dimethoxy-1-[[4-[5-(trifluoromethyl)-1,2,4-oxadiazol-3-yl]phenyl]methyl]urea, (15.100) 3-ethyl-1-methoxy-1-[[4-[5-(trifluoromethyl)-1,2,4-oxadiazol-3-yl]phenyl]methyl]urea, (15.101) 1-[[4-[5-(trifluoromethyl)-1,2,4-oxadiazol-3-yl]phenyl]-methyl]piperidin-2-one, (15.102) 4,4-dimethyl-2-[[4-[5-(trifluoromethyl)-1,2,4-oxadiazol-3-yl]phenyl]-methyl]isooxazolidin-3-one, (15.103) 5,5-dimethyl-2-[[4-[5-(trifluoromethyl)-1,2,4-oxadiazol-3-yl]phenyl]methyl]isoxazolidin-3-one, (15.104) 3,3-dimethyl-1-[[4-[5-(trifluoromethyl)-1,2,4-oxadiazol-3-yl]phenyl]methyl]piperidin-2-one, (15.105) 1-[[3-fluoro-4-(5-(trifluoromethyl)-1,2,4-oxadiazol-3-yl]-phenyl]methyl]azepan-2-one, (15.106) 4,4-dimethyl-2-[[4-(5-(trifluoromethyl)-1,2,4-oxadiazol-3-yl]-phenyl]methyl]isoxazolidin-3-one, (15.107) 5,5-dimethyl-2-[[4-[5-(trifluoromethyl)-1,2,4-oxadiazol-3-yl]phenyl]methyl]isoxazolidin-3-one, (15.108) ethyl 1-{4-[5-(trifluoromethyl)-1,2,4-oxadiazol-3-yl]benzyl}-1H-pyrazole-4-carboxylate, (15.109) N,N-dimethyl-1-{4-[5-(trifluoromethyl)-1,2,4-oxadiazol-3-yl]benzyl}-1H-1,2,4-triazol-3-amine, (15.110) N-{2,3-difluoro-4-[5-(trifluoromethyl)-1,2,4-oxadiazol-3-yl]benzyl}butanamide, (15.111) N-(1-methylcyclopropyl)-4-[5-(trifluoromethyl)-1,2,4-oxadiazol-3-yl]benzamide, (15.112) N-(2,4-difluorophenyl)-4-[5-(trifluoromethyl)-1,2,4-oxadiazo1-3-yl]benzamide, (15.113) 1-(5,6-dimethylpyridin-3-yl)-4,4-difluoro-3,3-dimethyl-3,4-dihydroisoquinoline, (15.114) 1-(6-(difluoromethyl)-5-methyl-pyridin-3-yl)-4,4-difluoro-3,3-dimethyl-3,4-dihydro-isoquinoline, (15.115) 1-(5-(fluoromethyl)-6-methyl-pyridin-3-yl)-4,4-difluoro-3,3-dimethyl-3,4-dihydroisoquinoline, (15.116) 1-(6-(difluoromethyl)-5-methoxy-pyridin-3-yl)-4,4-difluoro-3,3-dimethyl-3,4-dihydroisoquinoline, (15.117) 4-[5-(trifluoromethyl)-1,2,4-oxadiazol-3-yl]phenyl dimethylcarbamate, (15.118) N-{4-[5-(trifluoromethyl)-1,2,4-oxadiazol-3-yl]phenyl}propanamide, (15.119) 3-[2-(1-{[5-methyl-3-(trifluoromethyl)-1H-pyrazol-1-yl]acetyl }piperidin-4-yl)-1,3-thiazol-4-yl]-1,5-dihydro-2,4-benzodioxepin-6-yl methane sulfonate, (15.120) 9-fluoro-3-[2-(1-{[5-methyl-3-(trifluoromethyl)-1H-pyrazol-1-yl]acetyl}piperidin-4-yl)-1,3-thiazol-4-yl]-1,5-dihydro-2,4-benzodioxepin-6-yl methanesulfonate, (15.121) 3-[2-(1-{[3,5-bis(difluoromethyl)-1H-pyrazol-1-yl]acetyl}piperidin-4-yl)-1,3-thiazol-4-yl]-1,5-dihydro-2,4-benzodioxepin-6-yl methanesulfonate, (15.122) 3-[2-(1-{[3,5-bis(difluoromethyl)-1H-pyrazol-1-yl]acetyl}piperidin-4-yl)-1,3-thiazol-4-yl]-9-fluoro-1,5-dihydro-2,4-benzodioxepin-6-yl methanesulfonate, (15.123) 1-(6,7-dimethylpyrazolo[1,5-a]pyridin-3-yl)-4,4-difluoro-3,3-dimethyl-3,4-dihydroisoquinoline, (15.124) 8-fluoro-N-(4,4,4-trifluoro-2-methyl-1-phenylbutan-2-yl)quinoline-3-carboxamide, (15.125) 8-fluoro-N-[(2S)-4,4,4-trifluoro-2-methyl-1-phenylbutan-2-yl]quinoline-3-carboxamide, (15.126) N-(2,4-dimethyl-1-phenylpentan-2-yl)-8-fluoroquinoline-3-carboxamide and (15.127) N-[(2S)-2,4-dimethyl-1-phenylpentan-2-yl]-8-fluoroquinoline-3 -carboxamide.

All named mixing partners of the classes (1) to (15) as described here above can be present in the form of the free compound or, if their functional groups enable this, an agrochemically active salt thereof.

The compound and the composition of the invention may also be combined with one or more biological control agents.

As used herein, the term "biological control" is defined as control of harmful organisms such as a phytopathogenic fungi and/or insects and/or acarids and/or nematodes by the use or employment of a biological control agent.

As used herein, the term "biological control agent" is defined as an organism other than the harmful organisms and / or proteins or secondary metabolites produced by such an organism for the purpose of biological control. Mutants of the second organism shall be included within the definition of the biological control agent. The term "mutant" refers to a variant of the parental strain as well as methods for obtaining a mutant or variant in which the pesticidal activity is greater than that expressed by the parental strain. The "parent strain" is defined herein as the original strain before mutagenesis. To obtain such mutants the parental strain may be treated with a chemical such as N-methyl-N'-nitro-N-nitrosoguanidine, ethylmethanesulfone, or by irradiation using gamma, x-ray, or UV-irradiation, or by other means well known to those skilled in the art. Known mechanisms of biological control agents comprise enteric bacteria that control root rot by out-competing fungi for space on the surface of the root. Bacterial toxins, such as antibiotics, have been used to control pathogens. The toxin can be isolated and applied directly to the plant or the bacterial species may be administered so it produces the toxin *in situ.*

A "variant" is a strain having all the identifying characteristics of the NRRL or ATCC Accession Numbers as indicated in this text and can be identified as having a genome that hybridizes under conditions of high stringency to the genome of the NRRL or ATCC Accession Numbers.

"Hybridization" refers to a reaction in which one or more polynucleotides react to form a complex that is stabilized via hydrogen bonding between the bases of the nucleotide residues. The hydrogen bonding may occur by Watson-Crick base pairing, Hoogstein binding, or in any other sequence-specific manner. The complex may comprise two strands forming a duplex structure, three or more strands forming a multi-stranded complex, a single self-hybridizing strand, or any combination of these. Hybridization reactions can be performed under conditions of different "stringency". In general, a low stringency hybridization reaction is carried out at about 40 °C in 10 X SSC or a solution of equivalent ionic strength/temperature. A moderate stringency hybridization is typically performed at about 50 °C in 6 X SSC, and a high stringency hybridization reaction is generally performed at about 60 °C in 1 X SSC.

A variant of the indicated NRRL or ATCC Accession Number may also be defined as a strain having a genomic sequence that is greater than 85%, more preferably greater than 90% or more preferably greater than 95% sequence identity to the genome of the indicated NRRL or ATCC Accession Number. A polynucleotide or polynucleotide region (or a polypeptide or polypeptide region) has a certain percentage (for example, 80%, 85%, 90%, or 95%) of "sequence identity" to another sequence means that, when aligned, that percentage of bases (or amino acids) are the same in comparing the two sequences. This alignment and the percent homology or sequence identity can be determined using software programs known in the art, for example, those described in Current Protocols in Molecular Biology (F. M. Ausubel et al., eds., 1987).

NRRL is the abbreviation for the Agricultural Research Service Culture Collection, an international depositary authority for the purposes of deposing microorganism strains under the Budapest treaty on the international recognition of the deposit of microorganisms for the purposes of patent procedure, having the address National Center for Agricultural Utilization Research, Agricultural Research service, U.S. Department of Agriculture, 1815 North university Street, Peroira, Illinois 61604 USA.

ATCC is the abbreviation for the American Type Culture Collection, an international depositary authority for the purposes of deposing microorganism strains under the Budapest treaty on the international recognition of the deposit of microorganisms for the purposes of patent procedure, having the address ATCC Patent Depository, 10801 University Blvd., Manassas, VA 10110 USA.

Examples of biological control agents which may be combined with the compound and the composition of the invention are:
(A) Antibacterial agents selected from the group of:
   (A1) bacteria, such as (A1.1) *Bacillus subtilis,* in particular strain QST713/AQ713 (available as SERENADE OPTI or SERENADE ASO from Bayer CropScience LP, US, having NRRL Accession No. B21661, U.S. Patent No. 6,060,051); (A1.2) *Bacillus* sp., in particular strain D747 (available as DOUBLE NICKEL® from Kumiai Chemical Industry Co., Ltd.), having Accession No. FERM BP-8234, U.S. Patent No. 7,094,592; (A1.3) *Bacillus pumilus,* in particular strain BU F-33, having NRRL Accession No. 50185 (available as part of the CARTISSA® product from BASF, EPA Reg. No. 71840-19); (A1.4) *Bacillus subtilis var. amyloliquefaciens* strain FZB24 having Accession No. DSM 10271 (available from Novozymes as TAEGRO® or TAEGRO® ECO (EPA Registration No. 70127-5)); (A 1.5) a *Paenibacillus* sp. strain having Accession No. NRRL B-50972 or Accession No. NRRL B-67129, WO 2016/154297; (A1.6) *Bacillus subtilis* strain BU1814, (available as VELONDIS® PLUS, VELONDIS® FLEX and VELONDIS® EXTRA from BASF SE); (A1.7) *Bacillus mojavensis* strain R3B (Accession No. NCAIM (P) B001389) (WO 2013/034938) from Certis USA LLC, a subsidiary of Mitsui & Co.; (A1.8) *Bacillus subtilis* CX-9060 from Certis USA LLC, a subsidiary of Mitsui & Co.; (A1.9) *Paenibacillus polymyxa,* in particular strain AC-1 (e.g. TOPSEED® from Green Biotech Company Ltd.); (A1.10) *Pseudomonas proradix* (e.g. PRORADIX® from Sourcon Padena); (A1.11) *Pantoea agglomerans,* in particular strain E325 (Accession No. NRRL B-21856) (available as BLOOMTIME BIOLOGICAL™ FD BIOPESTICIDE from Northwest Agri Products); and
   (A2) fungi, such as (A2.1) *Aureobasidium pullulans,* in particular blastospores of strain DSM14940, blastospores of strain DSM 14941 ormixtures of blastospores of strains DSM14940 and DSM14941 (e.g., BOTECTOR® and BLOSSOM PROTECT®from bio-ferm, CH); (A2.2) *Pseudozyma aphidis* (as disclosed in WO2011/151819 by Yissum Research Development Company of the Hebrew University of Jerusalem); (A2.3) *Saccharomyces cerevisiae,* in particular strains CNCM No. 1-3936, CNCM No. 1-3937, CNCM No. I-3938 or CNCM No. I-3939 (WO 2010/086790) from Lesaffre et Compagnie, FR;
(B) biological fungicides selected from the group of:
   (B1) bacteria, for example (B1.1) *Bacillus subtilis,* in particular strain QST713/AQ713 (available as SERENADE OPTI or SERENADE ASO from Bayer CropScience LP, US, having NRRL Accession No. B21661 and described in U.S. Patent No. 6,060,051); (B1.2) *Bacillus pumilus,* in particular strain QST2808 (available as SONATA® from Bayer CropScience LP, US, having Accession No. NRRL B-30087 and described in U.S. Patent No. 6,245,551); (B1.3) *Bacillus pumilus,* in particular strain GB34 (available as Yield Shield® from Bayer AG, DE); (B1.4) *Bacillus pumilus,* in particular strain BU F-33, having NRRL Accession No. 50185 (available as part of the CARTISSA product from BASF, EPA Reg. No. 71840-19); (B1.5) *Bacillus amyloliquefaciens,* in particular strain D747 (available as Double Nickel™ from from Kumiai Chemical Industry Co., Ltd., having accession number FERM BP-8234, US Patent No. 7,094,592); (B1.6) *Bacillus subtilis* Y1336 (available as BIOBAC® WP from Bion-Tech, Taiwan, registered as a biological fungicide in Taiwan under Registration Nos. 4764, 5454, 5096 and 5277); (B1.7) *Bacillus subtilis* strain MBI 600 (available as SUBTILEX from BASF SE), having Accession Number NRRL B-50595, U.S. Patent No. 5,061,495; (B1.8) *Bacillus subtilis* strain GB03 (available as Kodiak® from Bayer AG, DE); (B1.9) *Bacillus subtilis var. amyloliquefaciens* strain FZB24 having Accession No. DSM 10271 (available from Novozymes as TAEGRO® or TAEGRO® ECO (EPA Registration No. 70127-5)); (B1.10) *Bacillus mycoides,* isolate J , having Accession No. B-30890 (available as BMJ TGAI® or WG and LifeGard™ from Certis USA LLC, a subsidiary of Mitsui & Co.); (B1.11) *Bacillus licheniformis,* in particular strain SB3086 , having Accession No. ATCC 55406, WO 2003/000051 (available as ECOGUARD® Biofungicide and GREEN RELEAF™ from Novozymes); (B1.12) a *Paenibacillus* sp. strain having Accession No. NRRL B-50972 or Accession No. NRRL B-67129, WO 2016/154297; (B1.13) *Bacillus subtilis* strain BU1814, (available as VELONDIS® PLUS, VELONDIS® FLEX and VELONDIS® EXTRA from BASF SE); (B1.14) *Bacillus subtilis* CX-9060 from Certis USA LLC, a subsidiary of Mitsui & Co.; (B1.15) *Bacillus amyloliquefaciens* strain F727 (also known as strain MBI110) (NRRL Accession No. B-50768; WO 2014/028521) (STARGUS® from Marrone Bio Innovations); (B1.16) *Bacillus amyloliquefaciens* strain FZB42, Accession No. DSM 23117 (available as RHIZOVITAL® from ABiTEP, DE); (B1.17) *Bacillus licheniformis* FMCH001 and *Bacillus subtilis* FMCH002 (QUARTZO® (WG) and PRESENCE® (WP) from FMC Corporation); (B1.18) *Bacillus mojavensis* strain R3B (Accession No. NCAIM (P) B001389) (WO 2013/034938) from Certis USA LLC, a subsidiary of Mitsui & Co.; (B1.19) *Paenibacillus polymyxa* ssp. *plantarum* (WO 2016/020371) from BASF SE; (B1.20) *Paenibacillus epiphyticus* (WO 2016/020371) from BASF SE; (B.1.21) *Pseudomonas chlororaphis* strain AFS009, having Accession No. NRRL B-50897, WO 2017/019448 (e.g., HOWLER™ and ZIO® from AgBiome Innovations, US); (B1.22) *Pseudomonas chlororaphis,* in particular strain MA342 (e.g. CEDOMON®, CERALL®, and CEDRESS® by Bioagri and Koppert); (B1.23) *Streptomyces lydicus* strain WYEC108 (also known as *Streptomyces lydicus* strain WYCD108US) (ACTINO-IRON® and ACTINOVATE® from Novozymes); (B1.24) *Agrobacterium radiobacter* strain K84 (e.g. GALLTROL-A® from AgBioChem, CA); (B1.25) *Agrobacterium radiobacter* strain K1026 (e.g. NOGALL™ from BASF SE); (B1.26) *Bacillus subtilis* KTSB strain (FOLIACTIVE® from Donaghys); (B1.27) *Bacillus subtilis* IAB/BS03 (AVIV™ from STK Bio-Ag Technologies); (B1.28) *Bacillus subtilis* strain Y1336 (available as BIOBAC® WP from Bion-Tech, Taiwan, registered as a biological fungicide in Taiwan under Registration Nos. 4764, 5454, 5096 and 5277); (B1.29) *Bacillus amyloliquefaciens* isolate B246 (e.g. AVOGREEN™ from University of Pretoria); (B1.30) *Bacillus methylotrophicus* strain BAC-9912 (from Chinese Academy of Sciences' Institute of Applied Ecology); (B1.31) *Pseudomonas proradix* (e.g. PRORADIX® from Sourcon Padena); (B1.32) *Streptomyces griseoviridis* strain K61 (also known as *Streptomyces galbus* strain K61) (Accession No. DSM 7206) (MYCOSTOP® from Verdera; PREFENCE® from BioWorks; cf. Crop Protection 2006, 25, 468-475); (B1.33) *Pseudomonas fluorescens* strain A506 (e.g. BLIGHTBAN® A506 by NuFarm); and
   (B2) fungi, for example: (B2.1) *Coniothyrium minitans,* in particular strain CON/M/91-8 (Accession No. DSM-9660; e.g. Contans ® from Bayer CropScience Biologics GmbH); (B2.2) *Metschnikowia fructicola,* in particular strain NRRL Y-30752; (B2.3) *Microsphaeropsis ochracea;* (B2.5) *Trichoderma atroviride,* in particular strain SC1 (having Accession No. CBS 122089, WO 2009/116106 and U.S. Patent No. 8,431,120 (from Bi-PA)), strain 77B (T77 from Andermatt Biocontrol) or strain LU132 (e.g. Sentinel from Agrimm Technologies Limited); (B2.6) *Trichoderma harzianum* strain T-22 (e.g. Trianum-P from Andermatt Biocontrol or Koppert) or strain Cepa Simb-T5 (from Simbiose Agro); (B2.14) *Gliocladium roseum* (also known as *Clonostachys rosea f. rosea*), in particular strain 321U from Adjuvants Plus, strain ACM941 as disclosed in Xue (Efficacy of Clonostachys rosea strain ACM941 and fungicide seed treatments for controlling the root tot complex of field pea, Can Jour Plant Sci 83(3): 519-524), or strain IK726 (Jensen DF, et al. Development of a biocontrol agent for plant disease control with special emphasis on the near commercial fungal antagonist Clonostachys rosea strain 'IK726'; Australas Plant Pathol. 2007;36:95-101); (B2.35) *Talaromyces flavus,* strain V117b; (B2.36) *Trichoderma viride,* in particular strain B35 (Pietr et al., 1993, Zesz. Nauk. A R w Szczecinie 161: 125-137); (B2.37) *Trichoderma asperellum,* in particular strain SKT-1, having Accession No. FERM P-16510 (e.g. ECO-HOPE® from Kumiai Chemical Industry), strain T34 (e.g. T34 Biocontrol by Biocontrol Technologies S.L., ES) or strain ICC 012 from Isagro; (B2.38) *Trichoderma atroviride,* strain CNCM 1-1237 (e.g. Esquive® WP from Agrauxine, FR); (B2.39) *Trichoderma atroviride,* strain no. V08/002387; (B2.40) *Trichoderma atroviride,* strain NMI no. V08/002388; (B2.41) *Trichoderma atroviride,* strain NMI no. V08/002389; (B2.42) *Trichoderma atroviride,* strain NMI no. V08/002390; (B2.43) *Trichoderma atroviride,* strain LC52 (e.g. Tenet by Agrimm Technologies Limited); (B2.44) *Trichoderma atroviride,* strain ATCC 20476 (IMI 206040); (B2.45) *Trichoderma atroviride,* strain T11 (IMI352941/ CECT20498); (B2.46) *Trichoderma harmatum;* (B2.47) *Trichoderma harzianum;* (B2.48) *Trichoderma harzianum rifai T39* (e.g. Trichodex® from Makhteshim, US); (B2.49) *Trichoderma asperellum,* in particular, strain kd (e.g. T-Gro from Andermatt Biocontrol); (B2.50) *Trichoderma harzianum,* strain ITEM 908 (e.g. Trianum-P from Koppert); (B2.51) *Trichoderma harzianum,* strain TH35 (e.g. Root-Pro by Mycontrol); (B2.52) *Trichoderma virens* (also known as *Gliocladium virens*), in particular strain GL-21 (e.g. SoilGard by Certis, US); (B2.53) *Trichoderma viride,* strain TV1(e.g. Trianum-P by Koppert); (B2.54) *Ampelomyces quisqualis,* in particular strain AQ 10 (e.g. AQ 10® by IntrachemBio Italia); (B2.56) *Aureobasidium pullulans,* in particular blastospores of strain DSM14940; (B2.57) *Aureobasidium pullulans,* in particular blastospores of strain DSM 14941; (B2.58) *Aureobasidium pullulans,* in particular mixtures of blastospores of strains DSM14940 and DSM 14941 (e.g. Botector® by bio-ferm, CH); (B2.64) *Cladosporium cladosporioides,* strain H39, having Accession No. CBS122244, US 2010/0291039 (by Stichting Dienst Landbouwkundig Onderzoek); (B2.69) *Gliocladium catenulatum* (Synonym: *Clonostachys rosea f. catenulate*) strain J1446 (e.g. Prestop ® by Lallemand); (B2.70) *Lecanicillium lecanii* (formerly known as *Verticillium lecanii*) *conidia* of strain KV01 (e.g. Vertalec® by Koppert/Arysta); (B2.71) *Penicillium vermiculatum;* (B2.72) *Pichia anomala,* strain WRL-076 (NRRL Y-30842), U.S. Patent No. 7,579,183; (B2.75) *Trichoderma atroviride,* strain SKT-1 (FERM P-16510), JP Patent Publication (Kokai) 11-253151 A; (B2.76) *Trichoderma atroviride,* strain SKT-2 (FERM P-16511), JP Patent Publication (Kokai) 11-253151 A; (B2.77) *Trichoderma atroviride,* strain SKT-3 (FERM P-17021), JP Patent Publication (Kokai) 11-253151 A; (B2.78) *Trichoderma gamsii* (formerly *T. viride),* strain ICC080 (IMI CC 392151 CABI, e.g. BioDerma by AGROBIOSOL DE MEXICO, S.A. DE C.V.); (B2.79) *Trichoderma harzianum,* strain DB 103 (available as T-GRO® 7456 by Dagutat Biolab); (B2.80) *Trichoderma polysporum,* strain IMI 206039 (e.g. Binab TF WP by BINAB Bio-Innovation AB, Sweden); (B2.81) *Trichoderma stromaticum,* having Accession No. Ts3550 (e.g. Tricovab by CEPLAC, Brazil); (B2.83) *Ulocladium oudemansii strain U3, having Accession No. NM 99*/*06216 (e.g., BOTRY-ZEN*® *by Botry-Zen Ltd, New Zealand and BOTRYSTOP*® *from BioWorks, Inc.);* (B2.84) *Verticillium albo-atrum* (formerly *V dahliae*), strain WCS850 having Accession No. WCS850, deposited at the Central Bureau for Fungi Cultures (e.g., DUTCH TRIG® by Tree Care Innovations); (B2.86) *Verticillium chlamydosporium*; (B2.87) mixtures of *Trichoderma asperellum* strain ICC 012 (also known as *Trichoderma harzianum* ICC012), having Accession No. CABI CC IMI 392716 and *Trichoderma gamsii* (formerly *T. viride)* strain ICC 080, having Accession No. IMI 392151 (e.g., BIO-TAM™from Isagro USA, Inc. and BIODERMA® by Agrobiosol de Mexico, S.A. de C.V.); (B2.88) *Trichoderma asperelloides* JM41R (Accession No. NRRL B-50759) (TRICHO PLUS® from BASF SE); (B2.89) *Aspergillus flavus* strain NRRL 21882 (products known as AFLA-GUARD®from Syngenta/ChemChina); (B2.90) *Chaetomium cupreum* (Accession No. CABI 353812) (e.g. BIOKUPRUM™ by AgriLife); (B2.91) *Saccharomyces cerevisiae,* in particular strain LASO2 (from Agro-Levures et Dérivés), strain LAS117 cell walls (CEREVISANE® from Lesaffre; ROMEO® from BASF SE), strains CNCM No. I-3936, CNCM No. 1-3937, CNCM No. 1-3938, CNCM No. I-3939 (WO 2010/086790) from Lesaffre et Compagnie, FR; (B2.92) *Trichoderma virens* strain G-41, formerly known as *Gliocladium virens* (Accession No. ATCC 20906) (e.g., ROOTSHIELD® PLUS WP and TURFSHIELD® PLUS WP from BioWorks, US); (B2.93) *Trichoderma hamatum,* having Accession No. ATCC 28012; (B2.94) *Ampelomyces quisqualis* strain AQ10, having Accession No. CNCM I-807 (e.g., AQ 10® by IntrachemBio Italia); (B2.95) *Phlebiopsis gigantea* strain VRA 1992 (ROTSTOP® C from Danstar Ferment); (B2.96) *Penicillium steckii* (DSM 27859; WO 2015/067800) from BASF SE; (B2.97) *Chaetomium globosum* (available as RIVADIOM® by Rivale); (B2.98) *Cryptococcus flavescens,* strain 3C (NRRL Y-50378); (B2.99) *Dactylaria candida;* (B2.100) *Dilophosphora alopecuri* (available as TWIST FUNGUS®); (B2.101) *Fusarium oxysporum,* strain Fo47 (available as FUSACLEAN® by Natural Plant Protection); (B2.102) *Pseudozyma flocculosa,* strain PF-A22 UL (available as SPORODEX® L by Plant Products Co., CA); (B2.103) *Trichoderma gamsii* (formerly *T. viride*), strain ICC 080 (IMI CC 392151 CABI) (available as BIODERMA® by AGROBIOSOL DE MEXICO, S.A. DE C.V.); (B2.104) *Trichoderma fertile* (e.g. product TrichoPlus from BASF); (B2.105) *Muscodor roseus,* in particular strain A3-5 (Accession No. NRRL 30548); (B2.106) *Simplicillium lanosoniveum*;
      biological control agents having an effect for improving plant growth and/or plant health which may be combined in the compound combinations according to the invention including
   (C1) bacteria selected from the group consisting of *Bacillus pumilus,* in particular strain QST2808 (having Accession No. NRRL No. B-30087); *Bacillus subtilis,* in particular strain QST713/AQ713 (having NRRL Accession No. B-21661 and described in U.S. Patent No. 6,060,051; available as SERENADE® OPTI or SERENADE® ASO from Bayer CropScience LP, US); *Bacillus subtilis,* in particular strain AQ30002 (having Accession Nos. NRRL B-50421 and described in U.S. Patent Application No. 13/330,576); *Bacillus subtilis,* in particular strain AQ30004 (and NRRL B-50455 and described in U.S. Patent Application No. 13/330,576); *Sinorhizobium meliloti* strain NRG-185-1 (NITRAGIN® GOLD from Bayer CropScience); *Bacillus subtilis* strain BU1814, (available as TEQUALIS® from BASF SE); *Bacillus subtilis* rm303 (RHIZOMAX® from Biofilm Crop Protection); *Bacillus amyloliquefaciens* pm414 (LOLI-PEPTA® from Biofilm Crop Protection); *Bacillus mycoides* BT155 (NRRL No. B-50921), *Bacillus mycoides* EE118 (NRRL No. B-50918), *Bacillus mycoides* EE141 (NRRL No. B-50916), *Bacillus mycoides* BT46-3 (NRRL No. B-50922), *Bacillus cereus* family member EE128 (NRRL No. B-50917), *Bacillus thuringiensis* BT013A (NRRL No. B-50924) also known as *Bacillus thuringiensis* 4Q7, *Bacillus cereus* family member EE349 (NRRL No. B-50928), *Bacillus amyloliquefaciens* SB3281 (ATCC # PTA-7542; WO 2017/205258), *Bacillus amyloliquefaciens* TJ1000 (available as QUIKROOTS® from Novozymes); *Bacillus firmus,* in particular strain CNMC 1-1582 (e.g. VOTIVO® from BASF SE); *Bacillus pumilus,* in particular strain GB34 (e.g. YIELD SHIELD® from Bayer Crop Science, DE); *Bacillus amyloliquefaciens,* in particular strain IN937a; *Bacillus amyloliquefaciens,* in particular strain FZB42 (e.g. RHIZOVITAL® from ABiTEP, DE); *Bacillus amyloliquefaciens* BS27 (Accession No. NRRL B-5015); a mixture of *Bacillus licheniformis* FMCH001 and *Bacillus subtilis* FMCH002 (available as QUARTZO® (WG), PRESENCE® (WP) from FMC Corporation); *Bacillus cereus,* in particular strain BP01 (ATCC 55675; e.g. MEPICHLOR® from Arysta Lifescience, US); *Bacillus subtilis,* in particular strain MBI 600 (e.g. SUBTILEX® from BASF SE); *Bradyrhizobium japonicum* (e.g. OPTIMIZE® from Novozymes); *Mesorhizobium cicer* (e.g., NODULATOR from BASF SE); *Rhizobium leguminosarium biovar viciae* (e.g., NODULATOR from BASF SE); *Delftia acidovorans,* in particular strain RAY209 (e.g. BIOBOOST® from Brett Young Seeds); *Lactobacillus sp.* (e.g. LACTOPLANT® from LactoPAFI); *Paenibacillus polymyxa,* in particular strain AC-1 (e.g. TOPSEED® from Green Biotech Company Ltd.); *Pseudomonas proradix* (e.g. PRORADIX® from Sourcon Padena); *Azospirillum brasilense* (e.g., VIGOR® from KALO, Inc.); *Azospirillum lipoferum* (e.g., VERTEX-IF™ from TerraMax, Inc.); a mixture of *Azotobacter vinelandii* and *Clostridium pasteurianum* (available as INVIGORATE® from Agrinos); *Pseudomonas aeruginosa,* in particular strain PN1; *Rhizobium leguminosarum,* in particular *bv. viceae* strain Z25 (Accession No. CECT 4585); *Azorhizobium caulinodans,* in particular strain ZB-SK-5; *Azotobacter chroococcum,* in particular strain H23; *Azotobacter vinelandii,* in particular strain ATCC 12837; *Bacillus siamensis,* in particular strain KCTC 13613T; *Bacillus tequilensis,* in particular strain NII-0943; *Serratia marcescens,* in particular strain SRM (Accession No. MTCC 8708); *Thiobacillus sp.* (e.g. CROPAID® from Cropaid Ltd UK); and
   (C2) fungi selected from the group consisting of *Purpureocillium lilacinum* (previously known as *Paecilomyces lilacinus*) strain 251 (AGAL 89/030550; e.g. BioAct from Bayer CropScience Biologics GmbH) *Penicillium bilaii,* strain ATCC 22348 (e.g. JumpStart® from Acceleron BioAg), *Talaromyces flavus*,strain V117b; *Trichoderma atroviride* strain CNCM 1-1237 (e.g. Esquive® WP from Agrauxine, FR), *Trichoderma viride,* e.g. strain B35 (Pietr et al., 1993, Zesz. Nauk. A R w Szczecinie 161: 125-137); *Trichoderma atroviride* strain LC52 (also known as *Trichoderma atroviride* strain LU132; e.g. Sentinel from Agrimm Technologies Limited); *Trichoderma atroviride* strain SC1 described in International Application No. PCT/IT2008/000196); *Trichoderma asperellum* strain kd (e.g. T-Gro from Andermatt Biocontrol); *Trichoderma asperellum* strain Eco-T (Plant Health Products, ZA); *Trichoderma harzianum* strain T-22 (e.g. Trianum-P from Andermatt Biocontrol or Koppert); *Myrothecium verrucaria* strain AARC-0255 (e.g. DiTera™ from Valent Biosciences); *Penicillium bilaii* strain ATCC ATCC20851; *Pythium oligandrum* strain M1 (ATCC 38472; e.g. Polyversum from Bioprepraty, CZ); *Trichoderma virens* strain GL-21 (e.g. SoilGard® from Certis, USA); *Verticillium albo-atrum* (formerly *V. dahliae*) strain WCS850 (CBS 276.92; e.g. Dutch Trig from Tree Care Innovations); *Trichoderma atroviride,* in particular strain no. V08/002387, strain no. NMI No. V08/002388, strain no. NMI No. V08/002389, strain no. NMI No. V08/002390; *Trichoderma harzianum* strain ITEM 908; *Trichoderma harzianum,* strain TSTh20; *Trichoderma harzianum* strain 1295-22; *Pythium oligandrum* strain DV74; *Rhizopogon amylopogon* (e.g. comprised in Myco-Sol from Helena Chemical Company); *Rhizopogon fulvigleba* (e.g. comprised in Myco-Sol from Helena Chemical Company); and *Trichoderma virens* strain GI-3;
      insecticidally active biological control agents selected from
   (D1) bacteria selected from the group consisting of *Bacillus thuringiensis subsp. aizawai,* in particular strain ABTS-1857 (SD-1372; e.g. XENTARI® from Valent BioSciences); *Bacillus mycoides,* isolate J. (e.g. BmJ from Certis USA LLC, a subsidiary of Mitsui & Co.); *Bacillus sphaericus,* in particular Serotype H5a5b strain 2362 (strain ABTS-1743) (e.g. VECTOLEX® from Valent BioSciences, US); *Bacillus thuringiensis subsp.* kurstaki strain BMP 123 from Becker Microbial Products, IL; *Bacillus thuringiensis subsp. aizawai,* in particular serotype H-7 (e.g. FLORBAC® WG from Valent BioSciences, US); *Bacillus thuringiensis subsp. kurstaki* strain HD-1 (e.g. DIPEL® ES from Valent BioSciences, US); *Bacillus thuringiensis subsp. kurstaki* strain BMP 123 by Becker Microbial Products, IL; *Bacillus thuringiensis israelensis* strain BMP 144 (e.g. AQUABAC® by Becker Microbial Products IL); *Burkholderia spp.,* in particular *Burkholderia rinojensis* strain A396 (also known as *Burkholderia rinojensis* strain MBI 305) (Accession No. NRRL B-50319; WO 2011/106491 and WO 2013/032693; e.g. MBI-206 TGAI and ZELTO® from Marrone Bio Innovations); *Chromobacterium subtsugae,* in particular strain PRAA4-1T (MBI-203; e.g. GRANDEVO® from Marrone Bio Innovations); *Paenibacillus popilliae* (formerly *Bacillus popilliae;* e.g. MILKY SPORE POWDER™ and MILKY SPORE GRANULAR™ from St. Gabriel Laboratories); *Bacillus thuringiensis* subsp. *israelensis* (serotype H-14) strain AM65-52 (Accession No. ATCC 1276) (e.g. VECTOBAC® by Valent BioSciences, US); *Bacillus thuringiensis* var. *kurstaki* strain EVB-113-19 (e.g., BIOPROTEC® from AEF Global); *Bacillus thuringiensis subsp. tenebrionis* strain NB 176 (SD-5428; e.g. NOVODOR® FC from BioFa DE); *Bacillus thuringiensis var. japonensis* strain Buibui; *Bacillus thuringiensis subsp. kurstaki* strain ABTS 351; *Bacillus thuringiensis subsp. kurstaki* strain PB 54; *Bacillus thuringiensis subsp. kurstaki* strain SA 11; *Bacillus thuringiensis subsp. kurstaki* strain SA 12; *Bacillus thuringiensis subsp. kurstaki* strain EG 2348; *Bacillus thuringiensis* var. *Colmeri* (e.g. TIANBAOBTC by Changzhou Jianghai Chemical Factory); *Bacillus thuringiensis subsp. aizawai* strain GC-91; *Serratia entomophila* (e.g. INVADE® by Wrightson Seeds); *Serratia marcescens,* in particular strain SRM (Accession No. MTCC 8708); and *Wolbachia pipientis* ZAP strain (e.g., ZAP MALES® from MosquitoMate); and
   (D2) fungi selected from the group consisting of *Isaria fumosorosea* (previously known as *Paecilomyces fumosoroseus*) strain apopka 97; *Beauveria bassiana* strain ATCC 74040 (e.g. NATURALIS® from Intrachem Bio Italia); *Beauveria bassiana* strain GHA (Accession No. ATCC74250; e.g. BOTANIGUARD® ES and MYCONTROL-O® from Laverlam International Corporation); *Zoophtora radicans; Metarhizium robertsii* 15013-1 (deposited under NRRL accession number 67073), *Metarhizium robertsii* 23013-3 (deposited under NRRL accession number 67075), and *Metarhizium anisopliae* 3213-1 (deposited under NRRL accession number 67074) (WO 2017/066094; Pioneer Hi-Bred International); *Beauveria bassiana* strain ATP02 (Accession No. DSM 24665). Among these, *Isaria fumosorosea* (previously known as *Paecilomyces fumosoroseus*) strain apopka 97 is particularly preferred;
(E) viruses selected from the group consisting of *Adoxophyes orana* (summer fruit tortrix) granulosis virus (GV), *Cydia pomonella* (codling moth) granulosis virus (GV), *Helicoverpa armigera* (cotton bollworm) nuclear polyhedrosis virus (NPV), *Spodoptera exigua* (beet armyworm) mNPV, *Spodoptera frugiperda* (fall armyworm) mNPV, and *Spodoptera littoralis* (African cotton leafworm) NPV;
(F) bacteria and fungi which can be added as 'inoculant' to plants or plant parts or plant organs and which, by virtue of their particular properties, promote plant growth and plant health. Examples are: *Agrobacterium spp., Azorhizobium caulinodans, Azospirillum spp., Azotobacter spp., Bradyrhizobium spp., Burkholderia spp.,* in particular *Burkholderia cepacia* (formerly known as *Pseudomonas cepacia*), *Gigaspora spp.,* or *Gigaspora monosporum, Glomus spp., Laccaria spp., Lactobacillus buchneri, Paraglomus spp., Pisolithus tinctorus, Pseudomonas spp., Rhizobium spp.,* in particular *Rhizobium trifolii, Rhizopogon spp., Scleroderma spp., Suillus spp.,* and *Streptomyces spp.;* and
(G) plant extracts and products formed by microorganisms including proteins and secondary metabolites which can be used as biological control agents, such as *Allium sativum, Artemisia absinthium,* azadirachtin, Biokeeper WP, *Cassia nigricans, Celastrus angulatus, Chenopodium anthelminticum,* chitin, Armour-Zen, *Dryopteris filix-mas, Equisetum arvense,* Fortune Aza, Fungastop, Heads Up *(Chenopodium quinoa* saponin extract), *Pyrethrum*/*Pyrethrins, Quassia amara, Quercus, Quillaja,* Regalia, "Requiem ™ Insecticide", rotenone, *ryania*/ryanodine, *Symphytum officinale, Tanacetum vulgare,* thymol, Triact 70, TriCon, *Tropaeulum majus, Urtica dioica,* Veratrin, *Viscum album, Brassicaceae* extract, in particular oilseed rape powder or mustard powder, as well as bioinsecticidal / acaricidal active substances obtained from olive oil, in particular unsaturated fatty/carboxylic acids having carbon chain lengths C₁₆-C₂₀ as active ingredients, such as, for example, contained in the product with the trade name FLiPPER®.

The compound and the composition of the invention may be combined with one or more active ingredients selected from insecticides, acaricides and nematicides.

"Insecticides" as well as the term "insecticidal" refers to the ability of a substance to increase mortality or inhibit growth rate of insects. As used herein, the term "insects" comprises all organisms in the class "Insecta" .

"Nematicide" and "nematicidal" refers to the ability of a substance to increase mortality or inhibit the growth rate of nematodes. In general, the term "nematode" comprises eggs, larvae, juvenile and mature forms of said organism.

"Acaricide" and "acaricidal" refers to the ability of a substance to increase mortality or inhibit growth rate of ectoparasites belonging to the class Arachnida, sub-class Acari.

Examples of insecticides, acaricides and nematicides, respectively, which could be mixed with the compound and the composition of the invention are:
(1) Acetylcholinesterase (AChE) inhibitors, such as, for example, carbamates, for example alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC and xylylcarb; or organophosphates, for example acephate, azamethiphos, azinphos-ethyl, azinphos-methyl, cadusafos, chlorethoxyfos, chlorfenvinphos, chlormephos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothiophosphoryl) salicylate, isoxathion, malathion, mecarbam, methamidophos, methidathion, mevinphos, monocrotophos, naled, omethoate, oxydemeton-methyl, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, triclorfon and vamidothion.
(2) GABA-gated chloride channel blockers, such as, for example, cyclodiene-organochlorines, for example chlordane and endosulfan or phenylpyrazoles (fiproles), for example ethiprole and fipronil.
(3) Sodium channel modulators, such as, for example, pyrethroids, e.g. acrinathrin, allethrin, d-cis-trans allethrin, d-trans allethrin, bifenthrin, bioallethrin, bioallethrin s-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans-isomer], deltamethrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, momfluorothrin, permethrin, phenothrin [(1R)-trans-isomer], prallethrin, pyrethrins (pyrethrum), resmethrin, silafluofen, tefluthrin, tetramethrin, tetramethrin [(1R)- isomer)], tralomethrin and transfluthrin or DDT or methoxychlor.
(4) Nicotinic acetylcholine receptor (nAChR) competitive modulators, such as, for example, neonicotinoids, e.g. acetamiprid, clothianidin, dinotefuran, imidacloprid, nitenpyram, thiacloprid and thiamethoxam or nicotine or sulfoxaflor or flupyradifurone.
(5) Nicotinic acetylcholine receptor (nAChR) allosteric modulators, such as, for example, spinosyns, e.g. spinetoram and spinosad.
(6) Glutamate-gated chloride channel (GluCl) allosteric modulators, such as, for example, avermectins/milbemycins, for example abamectin, emamectin benzoate, lepimectin and milbemectin.
(7) Juvenile hormone mimics, such as, for example, juvenile hormone analogues, e.g. hydroprene, kinoprene and methoprene or fenoxycarb or pyriproxyfen.
(8) Miscellaneous non-specific (multi-site) inhibitors, such as, for example, alkyl halides, e.g. methyl bromide and other alkyl halides; or chloropicrine or sulphuryl fluoride or borax or tartar emetic or methyl isocyanate generators, e.g. diazomet and metam.
(9) Modulators of Chordotonal Organs, such as, for example pymetrozine or flonicamid.
(10) Mite growth inhibitors, such as, for example clofentezine, hexythiazox and diflovidazin or etoxazole.
(11) Microbial disruptors of the insect gut membrane, such as, for example *Bacillus thuringiensis* subspecies *israelensis, Bacillus sphaericus, Bacillus thuringiensis* subspecies *aizawai, Bacillus thuringiensis* subspecies *kurstaki, Bacillus thuringiensis* subspecies *tenebrionis,* and *B.t.* plant proteins: Cry1Ab, Cry1Ac, Cry1Fa, Cry1A.105, Cry2Ab, Vip3A, mCry3A, Cry3Ab, Cry3Bb, Cry34Ab1/35Ab1.
(12) Inhibitors of mitochondrial ATP synthase, such as, ATP disruptors such as, for example, diafenthiuron or organotin compounds, for example azocyclotin, cyhexatin and fenbutatin oxide or propargite or tetradifon.
(13) Uncouplers of oxidative phosphorylation via disruption of the proton gradient, such as, for example, chlorfenapyr, DNOC and sulfluramid.
(14) Nicotinic acetylcholine receptor channel blockers, such as, for example, bensultap, cartap hydrochloride, thiocylam, and thiosultap-sodium.
(15) Inhibitors of chitin biosynthesis, type 0, such as, for example, bistrifluron, chlorfluazuron, diflubenzuron, flucycloxuron, flufenoxuron, hexaflumuron, lufenuron, novaluron, noviflumuron, teflubenzuron and triflumuron.
(16) Inhibitors of chitin biosynthesis, type 1, for example buprofezin.
(17) Moulting disruptor (in particular for Diptera, i.e. dipterans), such as, for example, cyromazine.
(18) Ecdysone receptor agonists, such as, for example, chromafenozide, halofenozide, methoxyfenozide and tebufenozide.
(19) Octopamine receptor agonists, such as, for example, amitraz.
(20) Mitochondrial complex III electron transport inhibitors, such as, for example, hydramethylnone or acequinocyl or fluacrypyrim.
(21) Mitochondrial complex I electron transport inhibitors, such as, for example from the group of the METI acaricides, e.g. fenazaquin, fenpyroximate, pyrimidifen, pyridaben, tebufenpyrad and tolfenpyrad or rotenone (Derris).
(22) Voltage-dependent sodium channel blockers, such as, for example indoxacarb or metaflumizone.
(23) Inhibitors of acetyl CoA carboxylase, such as, for example, tetronic and tetramic acid derivatives, e.g. spirodiclofen, spiromesifen and spirotetramat.
(24) Mitochondrial complex IV electron transport inhibitors, such as, for example, phosphines, e.g. aluminium phosphide, calcium phosphide, phosphine and zinc phosphide or cyanides, e.g. calcium cyanide, potassium cyanide and sodium cyanide.
(25) Mitochondrial complex II electron transport inhibitors, such as, for example, *beta*-ketonitrile derivatives, e.g. cyenopyrafen and cyflumetofen and carboxanilides, such as, for example, pyflubumide.
(28) Ryanodine receptor modulators, such as, for example, diamides, e.g. chlorantraniliprole, cyantraniliprole and flubendiamide,
   further active compounds such as, for example, Afidopyropen, Afoxolaner, Azadirachtin, Benclothiaz, Benzoximate, Bifenazate, Broflanilide, Bromopropylate, Chinomethionat, Chloroprallethrin, Cryolite, Cyclaniliprole, Cycloxaprid, Cyhalodiamide, Dicloromezotiaz, Dicofol, epsilon-Metofluthrin, epsilon-Momfluthrin, Flometoquin, Fluazaindolizine, Fluensulfone, Flufenerim, Flufenoxystrobin, Flufiprole, Fluhexafon, Fluopyram, Fluralaner, Fluxametamide, Fufenozide, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprole, Tetrachlorantraniliprole, Tigolaner, Tioxazafen, Thiofluoximate, Triflumezopyrim and iodomethane; furthermore preparations based on *Bacillus firmus* (1-1582, BioNeem, Votivo), and also the following compounds: 1-{2-fhioro-4-methyl-5-[(2,2,2-trifluoroethyl)sulphinyl]phenyl}-3-(trifluoromethyl)-1H-1,2,4-triazole-5-amine (known from WO2006/043635) (CAS 885026-50-6), {1'-[(2E)-3-(4-chlorophenyl)prop-2-en-1-yl]-5-fluorospiro[indol-3,4'-piperidin]-1(2H)-yl}(2-chloropyridin-4-yl)methanone (known from WO2003/106457) (CAS 637360-23-7), 2-chloro-N-[2-{1-[(2E)-3-(4-chlorophenyl)prop-2-en-1-yl]piperidin-4-yl}-4-(trifluoromethyl)phenyl]isonicotinamide (known from WO2006/003494) (CAS 872999-66-1), 3-(4-chloro-2,6-dimethylphenyl)-4-hydroxy-8-methoxy-1,8-diazaspiro[4.5]dec-3-en-2-one (known from WO 2010052161) (CAS 1225292-17-0), 3-(4-chloro-2,6-dimethylphenyl)-8-methoxy-2-oxo-1,8-diazaspiro[4.5]dec-3-en-4-yl ethyl carbonate (known from EP2647626) (CAS 1440516-42-6), 4-(but-2-yn-1-yloxy)-6-(3,5-dimethylpiperidin-1-yl)-5-fluoropyrimidine (known from WO2004/099160) (CAS 792914-58-0), PF1364 (known from JP2010/018586) (CAS 1204776-60-2), N-[(2E)-1-[(6-chloropyridin-3-yl)methyl]pyridin-2(1H)-ylidene]-2,2,2-trifluoroacetamide (known from WO2012/029672) (CAS 1363400-41-2), (3*E*)-3-[1-[(6-chloro-3-pyridyl)methyl]-2-pyridylidene]-1,1,1-trifluoro-propan-2-one (known from WO2013/144213) (CAS 1461743-15-6), , *N*-[3-(benzylcarbamoyl)-4-chlorophenyl]-1-methyl-3-(pentafluoroethyl)-4-(trifluoromethyl)-1*H*-pyrazole-5-carboxamide (known from WO2010/051926) (CAS 1226889-14-0), 5-bromo-4-chloro-*N*-[4-chloro-2-methyl-6-(methylcarbamoyl)phenyl]-2-(3-chloro-2-pyridyl)pyrazole-3-carboxamide (known from CN103232431) (CAS 1449220-44-3), 4-[5-(3,5-dichlorophenyl)-4,5-dihydro-5-(trifluoromethyl)-3-isoxazolyl]-2-methyl-*N*-(*cis*-1-oxido-3-thietanyl)-benzamide, 4-[5-(3,5-dichlorophenyl)-4,5-dihydro-5-(trifluoromethyl)-3-isoxazolyl]-2-methyl-*N*-(*trans*-1-oxido-3-thietanyl)-benzamide and 4-[(5*S*)-5-(3,5-dichlorophenyl)-4,5-dihydro-5-(trifluoromethyl)-3-isoxazolyl]-2-methyl-*N*-(*cis*-1-oxido-3-thietanyl)benzamide (known from WO 2013/050317 A1) (CAS 1332628-83-7), *N*-[3-chloro-1-(3-pyridinyl)-1*H*-pyrazol-4-yl]-*N*-ethyl-3-[(3,3,3-trifluoropropyl)sulfinyl]-propanamide, (+)-*N-*[3-chloro-1-(3-pyridinyl)-1*H*-pyrazol-4-yl]-*N*-ethyl-3-[(3,3,3-trifluoropropyl)sulfinyl]-propanamide and (-)-*N*-[3-chloro-1-(3-pyridinyl)-1*H*-pyrazol-4-yl]-*N*-ethyl-3-[(3,3,3-trifluoropropyl)sulfinyl]-propanamide (known from WO 2013/162715 A2, WO 2013/162716 A2, US 2014/0213448 A1) (CAS 1477923-37-7), 5-[[(2*E*)-3-chloro-2-propen-1-yl]amino]-1-[2,6-dichloro-4-(trifluoromethyl)phenyl]-4-[(trifluoromethyl)sulfmyl]-1*H*-pyrazole-3-carbonitrile (known from CN 101337937 A) (CAS 1105672-77-2), 3-bromo-*N*-[4-chloro-2-methyl-6-[(methylamino)thioxomethyl]phenyl]-1-(3-chloro-2-pyridinyl)-1*H*-pyrazole-5-carboxamide, (Liudaibenjiaxuanan, known from CN 103109816 A) (CAS 1232543-85-9); *N*-[4-chloro-2-[[(1,1-dimethylethyl)amino]carbonyl]-6-methylphenyl]-1-(3-chloro-2-pyridinyl)-3-(fluoromethoxy)-1*H*-Pyrazole-5-carboxamide (known from WO 2012/034403 A1) (CAS 1268277-22-0), *N*-[2-(5-amino-1,3,4-thiadiazol-2-yl)-4-chloro-6-methylphenyl]-3-bromo-1-(3-chloro-2-pyridinyl)-1*H-*pyrazole-5-carboxamide (known from WO 2011/085575 A1) (CAS 1233882-22-8), 4-[3-[2,6-dichloro-4-[(3,3-dichloro-2-propen-1-yl)oxy]phenoxy]propoxy]-2-methoxy-6-(trifluoromethyl)-pyrimidine (known from CN 101337940 A) (CAS 1108184-52-6); (2*E*)- and 2(*Z*)-2-[2-(4-cyanophenyl)-1-[3-(trifluoromethyl)phenyl]ethylidene]-*N*-[4-(difluoromethoxy)phenyl]-hydrazinecarboxamide (known from CN 101715774 A) (CAS 1232543-85-9); 3-(2,2-dichloroethenyl)-2,2-dimethyl-4-(1*H-*benzimidazol-2-yl)phenyl-cyclopropanecarboxylic acid ester (known from CN 103524422 A) (CAS 1542271-46-4); (4aS)-7-chloro-2,5-dihydro-2-[[(methoxycarbonyl)[4-[(trifluoromethyl)thio]phenyl] amino]carbonyl]-indeno[1,2-*e*][1,3,4]oxadiazine-4a(3*H*)-carboxylic acid methyl ester (known from CN 102391261 A) (CAS 1370358-69-2); 6-deoxy-3-*O*-ethyl-2,4-di-*O*-methyl-, 1-[*N*-[4-[1-[4-(1,1,2,2,2-pentafluoroethoxy)phenyl]-1*H*-1,2,4-triazol-3-yl]phenyl]carbamate]-α-L-mannopyranose (known from US 2014/0275503 A1) (CAS 1181213-14-8); 8-(2-cyclopropylmethoxy-4-trifluoromethyl-phenoxy)-3-(6-trifluoromethyl-pyridazin-3-yl)-3-aza-bicyclo[3.2.1]octane (CAS 1253850-56-4), (8*-anti*)-8-(2-cyclopropylmethoxy-4-trifluoromethyl-phenoxy)-3-(6-trifluoromethyl-pyridazin-3-yl)-3-aza-bicyclo[3.2.1]octane (CAS 933798-27-7), (8-*syn*)-8-(2-cyclopropylmethoxy-4-trifluoromethyl-phenoxy) -3-(6-trifluoromethyl-pyridazin-3-yl)-3-aza-bicyclo[3.2.1]octane (known from WO 2007040280 A1, WO 2007040282 A1) (CAS 934001-66-8), N-[3-chloro-1-(3-pyridinyl)-1H-pyrazol-4-yl]-N-ethyl-3-[(3,3,3-trifluoropropyl)thio]-propanamide (known from WO 2015/058021 A1, WO 2015/058028 A1) (CAS 1477919-27-9) and N-[4-(aminothioxomethyl)-2-methyl-6-[(methylamino)carbonyl]phenyl]-3-bromo-1-(3-chloro-2-pyridinyl)-1*H*-pyrazole-5-carboxamide (known from CN 103265527 A) (CAS 1452877-50-7), 5-(1,3-dioxan-2-yl)-4-[[4-(trifluoromethyl)phenyl]methoxy]-pyrimidine (known from WO 2013/115391 A1) (CAS 1449021-97-9), 3-(4-chloro-2,6-dimethylphenyl)-4-hydroxy-8-methoxy-1-methyl-1,8-diazaspiro[4.5]dec-3-en-2-one (known from WO 2010/066780 A1, WO 2011/151146 A1) (CAS 1229023-34-0), 3-(4-chloro-2,6-dimethylphenyl)-8-methoxy-1-methyl-1,8-diazaspiro[4.5]decane-2,4-dione (known from WO 2014/187846 A1) (CAS 1638765-58-8), 3-(4-chloro-2,6-dimethylphenyl)-8-methoxy-1-methyl-2-oxo-1,8-diazaspiro[4.5]dec-3-en-4-yl-carbonic acid ethyl ester (known from WO 2010/066780 A1, WO 2011151146 A1) (CAS 1229023-00-0), N-[1-[(6-chloro-3-pyridinyl)methyl]-2(1*H*)-pyridinylidene]-2,2,2-trifluoro-acetamide (known from DE 3639877 A1, WO 2012029672 A1) (CAS 1363400-41-2), [N(*E*)]-N-[1-[(6-chloro-3-pyridinyl)methyl]-2(1H)-pyridinylidene]-2,2,2-trifluoroacetamide, (known from WO 2016005276 A1) (CAS 1689566-03-7), [N(*Z*)]-N-[1-[(6-chloro-3-pyridinyl)methyl]-2(1H)-pyridinylidene]-2,2,2-trifluoro-acetamide, (CAS 1702305-40-5), 3-*endo*-3-[2-propoxy-4-(trifluoromethyl)phenoxy]-9-[[5-(trifluoromethyl)-2-pyridinyl]oxy]-9-azabicyclo[3.3.1]nonane (known from WO 2011/105506 A1, WO 2016/133011 A1) (CAS 1332838-17-1).

Examples of herbicides which could be mixed with the compound and the composition of the invention are:
Acetochlor, acifluorfen, acifluorfen-sodium, aclonifen, alachlor, allidochlor, alloxydim, alloxydim-sodium, ametryn, amicarbazone, amidochlor, amidosulfuron, 4-amino-3-chloro-5-fluoro-6-(7-fluoro-1H-indol-6-yl)pyridine-2-carboxylic acid, aminocyclopyrachlor, aminocyclopyrachlor-potassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bixlozone, bromacil, bromobutide, bromofenoxim, bromoxynil, bromoxynil-butyrate, -potassium, -heptanoate, and -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, 1-{2-chloro-3-[(3-cyclopropyl-5-hydroxy-1-methyl-1H-pyrazol-4-yl)carbonyl]-6-(trifluormethyl)phenyl}piperidin-2-on, 4-{2-chloro-3-[(3,5-dimethyl-1H-pyrazol-1-yl)methyl]-4-(methylsulfonyl)benzoyl}-1,3-dimethyl-1H-pyrazol-5-yl-1,3-dimethyl-1H-pyrazol-4-carboxylat, chlorfenac, chlorfenac-sodium, chlorfenprop, chlorflurenol, chlorflurenol-methyl, chloridazon, chlorimuron, chlorimuron-ethyl, 2-[2-chloro-4-(methylsulfonyl)-3-(morpholin-4-ylmethyl)benzoyl]-3-hydroxycyclohex-2-en-1-on, 4-{2-chloro-4-(methylsulfonyl)-3-[(2,2,2-trifluorethoxy)methyl]benzoyl}-1-ethyl-1H-pyrazol-5-yl-1,3-dimethyl-1H-pyrazol-4-carboxylat, chlorophthalim, chlorotoluron, chlorthal-dimethyl, 3-[5-chloro-4-(trifluormethyl)pyridine-2-yl]-4-hydroxy-1-methylimidazolidine-2-on, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyranil, cyclopyrimorate, cyclosulfamuron, cycloxydim, cyhalofop, cyhalofop-butyl, cyprazine, 2,4-D, 2,4-D-butotyl, -butyl,-dimethylammonium, -diolamin, -ethyl, -2-ethylhexyl, -isobutyl, -isooctyl, -isopropylammonium,-potassium, -triisopropanolammonium, and -trolamine, 2,4-DB, 2,4-DB-butyl, -dimethylammonium,-isooctyl, -potassium, and -sodium, daimuron (dymron), dalapon, dazomet, n-decanol, desmedipham, detosyl-pyrazolate (DTP), dicamba, dichlobenil, dichlorprop, dichlorprop-P, diclofop, diclofop-methyl, diclofop-P-methyl, diclosulam, difenzoquat, diflufenican, diflufenzopyr, diflufenzopyr-sodium, dimefuron, dimepiperate, dimethachlor, dimethametryn, dimethenamid, dimethenamid-P, 3-(2,6-dimethylphenyl)-6-[(2-hydroxy-6-oxocyclohex-1-en-1-yl)carbonyl]-1-methylchinazolm-2,4(1H,3H)-dion, 1,3-dimethyl-4-[2-(methylsulfonyl)-4-(trifluormethyl)benzoyl]-1H-pyrazol-5-yl-1,3-dimethyl-1H-pyrazol-4-carboxylat, dimetrasulfuron, dinitramine, dinoterb, diphenamid, diquat, diquat-dibromid, dithiopyr, diuron, DMPA, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, ethyl-[(3-{2-chloro-4-fluoro-5-[3-methyl-2,6-dioxo-4-(trifluormethyl)-3,6-dihydropyrimidin-1(2H)-yl]phenoxy}pyridin-2-yl)oxy]acetat, F-9960, F-5231, i.e. N-{2-chloro-4-fluoro-5-[4-(3-fluoropropyl)-5-oxo-4,5-dihydro-1H-tetrazol-1-yl]phenyl}ethanesulfonamide, F-7967, i. e. 3-[7-chloro-5-fluoro-2-(trifluoromethyl)-1H-benzimidazol-4-yl]-1-methyl-6-(trifluoromethyl)pyrimidine-2,4(1H,3H)-dione, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, -dimethylammonium and -methyl, fluoroglycofen, fluoroglycofen-ethyl, flupropanate, flupyrsulfuron, flupyrsulfuron-methyl-sodium, fluridone, fluro-chloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, -potassium, -sodium, and -trimesium, H-9201, i.e. O-(2,4-dimethyl-6-nitrophenyl) O-ethyl isopropylphosphoramidothioate, halauxifen, halauxifen-methyl ,halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(dimethoxyphosphoryl) ethyl-(2,4-dichlorophenoxy)acetate, 4-hydroxy-1-methoxy-5 -methyl-3 -[4-(trifluormethyl)pyridine-2-yl]imidazolidine-2-on, 4-hydroxy-1-methyl-3-[4-(trifluormethyl)pyridine-2-yl]imidazolidine-2-on, (5-hydroxy-1-methyl-1H-pyrazol-4-yl)(3,3,4-trimethyl-1,1-dioxido-2,3-dihydro-1-benzothiophen-5-yl)methanon, 6-[(2-hydroxy-6-oxocyclohex-1-en-1-yl)carbonyl]-1,5-dimethyl-3-(2-methylphenyl)chinazolin-2,4(1H,3H)-dion, imazamethabenz, imazamethabenz-methyl, imazamox, imazamox-ammonium, imazapic, imazapic-ammonium, imazapyr, imazapyr-isopropylammonium, imazaquin, imazaquin-ammonium, imazethapyr, imazethapyr-immonium, imazosulfuron, indanofan, indaziflam, iodosulfuron, iodosulfuron-methyl-sodium, ioxynil, ioxynil-octanoate, -potassium and -sodium, ipfencarbazone, isoproturon, isouron, isoxaben, isoxaflutole, karbutilate, KUH-043, i.e. 3-({[5-(difluoromethyl)-1-methyl-3-(trifluoromethyl)-1H-pyrazol-4-yl]methyl}sulfonyl)-5,5-dimethyl-4,5-dihydro-1,2-oxazole, ketospiradox, lactofen, lenacil, linuron, MCPA, MCPA-butotyl,-dimethylammonium, -2-ethylhexyl, -isopropylammonium, -potassium, and -sodium, MCPB, MCPB-methyl, -ethy,1 and -sodium, mecoprop, mecoprop-sodium, and -butotyl, mecoprop-P, mecoprop-P-butotyl, -dimethylammonium, -2-ethylhexyl, and -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, 2-({2-[(2-methoxyethoxy)methyl] -6-(trifluormethyl)pyridin-3 -yl} carbonyl)cyclohexan-1,3 -dion, methyl isothiocyanate, 1-methyl-4-[(3,3,4-trimethyl-1,1-dioxido-2,3-dihydro-1-benzothiophen-5-yl)carbonyl]-1H-pyrazol-5-ylpropan-1-sulfonat, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-(3-chloro-4-isopropylphenyl)-2-methylpentan amide, NGGC-011, napropamide, NC-310, i.e. [5-(benzyloxy)-1-methyl-1H-pyrazol-4-yl](2,4-dichlorophenyl)methanone, neburon, nicosulfuron, nonanoic acid (pelargonic acid), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, QYM-201, QYR-301, rimsulfuron, saflufenacil, sethoxydim, siduron, simazine, simetryn, SL-261, sulcotrion, sulfentrazone, sulfometuron, sulfometuron-methyl, sulfosulfuron, SYN-523, SYP-249, i.e. 1-ethoxy-3-methyl-1-oxobut-3-en-2-yl 5-[2-chloro-4-(trifluoromethyl)phenoxy]-2-nitrobenzoate, SYP-300, i.e. 1-[7-fluoro-3-oxo-4-(prop-2-yn-1-yl)-3,4-dihydro-2H-1,4-benzoxazin-6-yl]-3-propyl-2-thioxoimidazolidine-4,5-dione, 2,3,6-TBA, TCA (trichloroacetic acid), TCA-sodium, tebuthiuron, tefuryltrione, tembotrione, tepraloxydim, terbacil, terbucarb, terbumeton, terbuthylazin, terbutryn, tetflupyrolimet, thenylchlor, thiazopyr, thiencarbazone, thiencarbazone-methyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, ZJ-0862, i.e. 3,4-dichloro-N-{2-[(4,6-dimethoxypyrimidin-2-yl)oxy]benzyl } aniline.

Examples for plant growth regulators are:
Acibenzolar, acibenzolar-S-methyl, 5-aminolevulinic acid, ancymidol, 6-benzylaminopurine, Brassinolid, catechine, chlormequat chloride, cloprop, cyclanilide, 3-(cycloprop-1-enyl) propionic acid, daminozide, dazomet, n-decanol, dikegulac, dikegulac-sodium, endothal, endothal-dipotassium, -disodium, and - mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, jasmonic acid, maleic hydrazide, mepiquat chloride, 1-methylcyclopropene, methyl jasmonate, 2-(1-naphthyl)acetamide, 1-naphthylacetic acid, 2- naphthyloxyacetic acid, nitrophenolate-mixture, paclobutrazol, N-(2-phenylethyl)-beta-alanine, N-phenylphthalamic acid, prohexadione, prohexadione-calcium, prohydrojasmone, salicylic acid, strigolactone, tecnazene, thidiazuron, triacontanol, trinexapac, trinexapac-ethyl, tsitodef, uniconazole, uniconazole-P.

Examples of safeners which could be mixed with the compound and the composition of the invention are, for example, benoxacor, cloquintocet (-mexyl), cyometrinil, cyprosulfamide, dichlormid, fenchlorazole (-ethyl), fenclorim, flurazole, fluxofenim, furilazole, isoxadifen (-ethyl), mefenpyr (-diethyl), naphthalic anhydride, oxabetrinil, 2-methoxy-N-({4-[(methylcarbamoyl)amino]phenyl}-sulphonyl)benzamide (CAS 129531-12-0), 4-(dichloroacetyl)-1-oxa-4-azaspiro[4.5]decane (CAS 71526-07-3), 2,2,5-trimethyl-3-(dichloroacetyl)-1,3-oxazolidine (CAS 52836-31-4).

Examples of nitrification inhibitors wich can be mixed with the compound and the composition of the invention are selected from the group consisting of 2-(3,4-dimethyl-1 H-pyrazol-1 -yl)succinic acid, 2-(4,5-dimethyl-1H-pyrazol-1-yl)succinic acid, 3,4-dimethyl pyrazolium glycolate, 3,4-dimethyl pyrazolium citrate, 3,4-dimethyl pyrazolium lactate, 3,4-dimethyl pyrazolium mandelate, 1 ,2,4-triazole, 4-Chloro-3-methylpyrazole, N-((3(5)-methyl-1H-pyrazole-1-yl)methyl)acetamide, N-((3(5)-methyl-1 H-pyrazole-1-yl)methyl)formamide, N-((3(5),4-dimethylpyrazole-1-yl)methyl)formamide, N-((4-chloro-3(5)-methyl-pyrazole-1-yl)methyl)formamide; reaction adducts of dicyandiamide, urea and formaldehyde, triazonyl- formaldehyde-dicyandiamide adducts, 2-cyano-1-((4-oxo-1,3,5-triazman-1-yl)methyl)guanidine, 1-((2-cyanoguanidino)methyl)urea, 2-cyano-1-((2-cyanoguanidino)methyl)-guanidine, 2-chloro-6-(trichloromethyl)-pyridine (nitrapyrin or N-serve), dicyandiamide, 3,4-dimethyl pyrazole phosphate, 4,5-dimethyl pyrazole phosphate, 3,4-dimethylpyrazole, 4,5-dimethyl pyrazole, ammoniumthiosulfate, neem, products based on ingredients of neem, linoleic acid, alpha-linolenic acid, methyl p-coumarate, methyl ferulate, methyl 3-(4-hydroxyphenyl) propionate, karanjin, brachialacton, p-benzoquinone sorgoleone, 4-amino-1,2,4-triazole hydrochloride, 1-amido-2-thiourea, 2-amino-4-chloro-6-methylpyrimidine, 2-mercapto-benzothiazole, 5-ethoxy-3-trichloromethyl-1,2,4-thiodiazole (terrazole, etridiazole), 2-sulfanilamidothiazole, 3-methylpyrazol, 1,2,4-triazol thiourea, cyan amide, melamine, zeolite powder, catechol, benzoquinone, sodium tetraborate, allylthiourea, chlorate salts, and zinc sulfate.

The compound and the composition of the invention may be combined with one or more agriculturally beneficial agents.

Examples of agriculturally beneficial agents include biostimulants, plant growth regulators, plant signal molecules, growth enhancers, microbial stimulating molecules, biomolecules, soil amendments, nutrients, plant nutrient enhancers, etc., such as lipo-chitooligosaccharides (LCO), chitooligosaccharides (CO), chitinous compounds, flavonoids, jasmonic acid or derivatives thereof (e.g., jasmonates), cytokinins, auxins, gibberellins, absiscic acid, ethylene, brassinosteroids, salicylates, macro- and micro-nutrients, linoleic acid or derivatives thereof, linolenic acid or derivatives thereof, karrikins, and beneficial microorganisms (e.g., *Rhizobium* spp., *Bradyrhizobium* spp., *Sinorhizobium* spp., *Azorhizobium* spp., *Glomus* spp., *Gigaspora* spp., *Hymenoscyphous* spp., *Oidiodendron* spp., *Laccaria* spp., *Pisolithus* spp., *Rhizopogon* spp., *Scleroderma* spp., *Rhizoctonia* spp., *Acinetobacter* spp., *Arthrobacter* spp., *Arthrobotrys* spp., *Aspergillus* spp., *Azospirillum* spp., *Bacillus* spp., *Burkholderia* spp., *Candida* spp., *Chryseomonas* spp., *Enterobacter* spp., *Eupenicillium* spp., *Exiguobacterium* spp., *Klebsiella* spp., *Kluyvera* spp., *Microbacterium* spp., *Mucor* spp., *Paecilomyces* spp., *Paenibacillus* spp., *Penicillium* spp., *Pseudomonas* spp., *Serratia* spp., *Stenotrophomonas* spp., *Streptomyces* spp., *Streptosporangium* spp., *Swaminathania* spp., *Thiobacillus* spp., *Torulospora* spp., *Vibrio* spp., *Xanthobacter* spp., *Xanthomonas* spp., etc.), and combinations thereof.

### Methods and uses

The compound and the composition of the invention have potent microbicidal activity and/or plant defense modulating potential. They can be used for controlling unwanted microorganisms, such as unwanted fungi and bacteria, on plants. They can be particularly useful in crop protection (they control microorganisms that cause plants diseases) or for protecting materials (e.g. industrial materials, timber, storage goods) as described in more details herein below. More specifically, the compound and the composition of the invention can be used to protect seeds, germinating seeds, emerged seedlings, plants, plant parts, fruits, harvest goods and/or the soil in which the plants grow from unwanted microorganisms.

Control or controlling as used herein encompasses protective, curative and eradicative treatment of unwanted microorganisms. Unwanted microorganisms may be pathogenic bacteria, pathogenic virus, pathogenic oomycetes or pathogenic fungi, more specifically phytopathogenic bacteria, phytopathogenic virus, phytopathogenic oomycetes or phytopathogenic fungi. As detailed herein below, these phytopathogenic microorganims are the causal agents of a broad spectrum of plants diseases.

More specifically, the compound and the composition of the invention can be used as fungicides. For the purpose of the specification, the term "fungicide" refers to a compound or composition that can be used in crop protection for the control of unwanted fungi, such as Plasmodiophoromycetes, Chytridiomycetes, Zygomycetes, Ascomycetes, Basidiomycetes and Deuteromycetes and/or for the control of Oomycetes.

The compound and the composition of the invention may also be used as antibacterial agent. In particular, they may be used in crop protection, for example for the control of unwanted bacteria, such as Pseudomonadaceae, Rhizobiaceae, Xanthomonadaceae, Enterobacteriaceae, Corynebacteriaceae and Streptomycetaceae.

The compound and the composition of the invention may also be used as antiviral agent in crop protection. For example the compound and the composition of the invention may have effects on diseases from plant viruses, such as the tobacco mosaic virus (TMV), tobacco rattle virus, tobacco stunt virus (TStuV), tobacco leaf curl virus (VLCV), tobacco nervilia mosaic virus (TVBMV), tobacco necrotic dwarf virus (TNDV), tobacco streak virus (TSV), potato virus X (PVX), potato viruses Y, S, M, and A, potato acuba mosaic virus (PAMV), potato mop-top virus (PMTV), potato leaf-roll virus (PLRV), alfalfa mosaic virus (AMV), cucumber mosaic virus (CMV), cucumber green mottlemosaic virus (CGMMV), cucumber yellows virus (CuYV), watermelon mosaic virus (WMV), tomato spotted wilt virus (TSWV), tomato ringspot virus (TomRSV), sugarcane mosaic virus (SCMV), rice drawf virus, rice stripe virus, rice black-streaked drawf virus, strawberry mottle virus (SMoV), strawberry vein banding virus (SVBV), strawberry mild yellow edge virus (SMYEV), strawberry crinkle virus (SCrV), broad beanwilt virus (BBWV), and melon necrotic spot virus (MNSV).

The present invention also relates to a method for controlling unwanted microorganisms, such as unwanted fungi, oomycetes and bacteria, on plants comprising the step of applying at least one compound of the invention or at least one composition of the invention to the microorganisms and/or their habitat (to the plants, plant parts, seeds, fruits or to the soil in which the plants grow).

Typically, when the compound and the composition of the invention are used in curative or protective methods for controlling phytopathogenic fungi and/or phytopathogenic oomycetes, an effective and plant-compatible amount thereof is applied to the plants, plant parts, fruits, seeds or to the soil or substrates in which the plants grow. Suitable substrates that may be used for cultivating plants include inorganic based substrates, such as mineral wool, in particular stone wool, perlite, sand or gravel; organic substrates, such as peat, pine bark or sawdust; and petroleum based substrates such as polymeric foams or plastic beads. Effective and plant-compatible amount means an amount that is sufficient to control or destroy the fungi present or liable to appear on the cropland and that does not entail any appreciable symptom of phytotoxicity for said crops. Such an amount can vary within a wide range depending on the fungus to be controlled, the type of crop, the crop growth stage, the climatic conditions and the respective compound or composition of the invention used. This amount can be determined by systematic field trials that are within the capabilities of a person skilled in the art.

### Plants and plant parts

The compound and the composition of the invention may be applied to any plants or plant parts.

Plants mean all plants and plant populations, such as desired and undesired wild plants or crop plants (including naturally occurring crop plants). Crop plants may be plants which can be obtained by conventional breeding and optimization methods or by biotechnological and genetic engineering methods or combinations of these methods, including the genetically modified plants (GMO or transgenic plants) and the plant cultivars which are protectable and non-protectable by plant breeders' rights.

Plant cultivars are understood to mean plants which have new properties ("traits") and have been obtained by conventional breeding, by mutagenesis or by recombinant DNA techniques. They can be cultivars, varieties, bio- or genotypes.

Plant parts are understood to mean all parts and organs of plants above and below the ground, such as shoots, leaves, needles, stalks, stems, flowers, fruit bodies, fruits, seeds, roots, tubers and rhizomes. The plant parts also include harvested material and vegetative and generative propagation material, for example cuttings, tubers, rhizomes, slips and seeds.

Plants which may be treated in accordance with the methods of the invention include the following: cotton, flax, grapevine, fruit, vegetables, such as *Rosaceae sp.* (for example pome fruits such as apples and pears, but also stone fruits such as apricots, cherries, almonds and peaches, and soft fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for example banana trees and plantations), *Rubiaceae sp.* (for example coffee), *Theaceae sp., Sterculiceae sp., Rutaceae sp.* (for example lemons, oranges and grapefruit); *Solanaceae sp.* (for example tomatoes), *Liliaceae sp., Asteraceae sp.* (for example lettuce), *Umbelliferae sp., Cruciferae sp., Chenopodiaceae sp., Cucurbitaceae sp.* (for example cucumber), *Alliaceae sp.* (for example leek, onion), *Papilionaceae sp.* (for example peas); major crop plants, such as *Gramineae sp.* (for example maize, turf, cereals such as wheat, rye, rice, barley, oats, millet and triticale), *Asteraceae sp.* (for example sunflower), *Brassicaceae sp.* (for example white cabbage, red cabbage, broccoli, cauliflower, Brussels sprouts, pak choi, kohlrabi, radishes, and oilseed rape, mustard, horseradish and cress), *Fabacae sp.* (for example bean, peanuts), *Papilionaceae sp.* (for example soya bean), *Solanaceae sp.* (for example potatoes), *Chenopodiaceae sp.* (for example sugar beet, fodder beet, swiss chard, beetroot); useful plants and ornamental plants for gardens and wooded areas; and genetically modified varieties of each of these plants.

Plants and plant cultivars which may be treated by the above disclosed methods include plants and plant cultivars which are resistant against one or more biotic stresses, i.e. said plants show a better defense against animal and microbial pests, such as against nematodes, insects, mites, phytopathogenic fungi, bacteria, viruses and/or viroids.

Plants and plant cultivars which may be treated by the above disclosed methods include those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.

Plants and plant cultivars which may be treated by the above disclosed methods include those plants characterized by enhanced yield characteristics. Increased yield in said plants may be the result of, for example, improved plant physiology, growth and development, such as water use efficiency, water retention efficiency, improved nitrogen use, enhanced carbon assimilation, improved photosynthesis, increased germination efficiency and accelerated maturation. Yield may furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content and composition for example cotton or starch, protein content, oil content and composition, nutritional value, reduction in anti-nutritional compounds, improved processability and better storage stability.

Plants and plant cultivars which may be treated by the above disclosed methods include plants and plant cultivars which are hybrid plants that already express the characteristic of heterosis or hybrid vigor which results in generally higher yield, vigor, health and resistance towards biotic and abiotic stresses.

### Transgenic plants, seed treatment and integration events

The compound according to the invention can be advantageously used to treat transgenic plants, plant cultivars or plant parts that received genetic material which imparts advantageous and/or useful properties (traits) to these plants, plant cultivars or plant parts. Therefore, it is contemplated that the present invention may be combined with one or more recombinant traits or transgenic event(s) or a combination thereof. For the purposes of this application, a transgenic event is created by the insertion of a specific recombinant DNA molecule into a specific position (locus) within the chromosome of the plant genome. The insertion creates a novel DNA sequence referred to as an "event" and is characterized by the inserted recombinant DNA molecule and some amount of genomic DNA immediately adjacent to/flanking both ends of the inserted DNA. Such trait(s) or transgenic event(s) include, but are not limited to, pest resistance, water use efficiency, yield performance, drought tolerance, seed quality, improved nutritional quality, hybrid seed production, and herbicide tolerance, in which the trait is measured with respect to a plant lacking such trait or transgenic event. Concrete examples of such advantageous and/or useful properties (traits) are better plant growth, vigor, stress tolerance, standability, lodging resistance, nutrient uptake, plant nutrition, and/or yield, in particular improved growth, increased tolerance to high or low temperatures, increased tolerance to drought or to levels of water or soil salinity, enhanced flowering performance, easier harvesting, accelerated ripening, higher yields, higher quality and/or a higher nutritional value of the harvested products, better storage life and/or processability of the harvested products, and increased resistance against animal and microbial pests, such as against insects, arachnids, nematodes, mites, slugs and snails.

Among DNA sequences encoding proteins which confer properties of tolerance to such animal and microbial pests, in particular insects, mention will particularly be made of the genetic material from *Bacillus thuringiensis* encoding the Bt proteins widely described in the literature and well known to those skilled in the art. Mention will also be made of proteins extracted from bacteria such as *Photorhabdus* (WO97/17432 and WO98/08932). In particular, mention will be made of the Bt Cry or VIP proteins which include the CrylA, CryIAb, CryIAc, CryIIA, CryIIIA, CryIIIB2, Cry9c Cry2Ab, Cry3Bb and CryIF proteins or toxic fragments thereof and also hybrids or combinations thereof, especially the CrylF protein or hybrids derived from a CrylF protein (e.g. hybrid CrylA-CrylF proteins or toxic fragments thereof), the CrylA-type proteins or toxic fragments thereof, preferably the CrylAc protein or hybrids derived from the CrylAc protein (e.g. hybrid CrylAb-CrylAc proteins) or the CrylAb or Bt2 protein or toxic fragments thereof, the Cry2Ae, Cry2Af or Cry2Ag proteins or toxic fragments thereof, the CrylA.105 protein or a toxic fragment thereof, the VIP3Aa19 protein, the VIP3Aa20 protein, the VIP3A proteins produced in the COT202 or COT203 cotton events, the VIP3Aa protein ora toxic fragment thereof as described in Estruch et al. (1996), Proc Natl Acad Sci US A. 28;93(11):5389-94, the Cry proteins as described in WO2001/47952, the insecticidal proteins from *Xenorhabdus* (as described in WO98/50427), *Serratia* (particularly from S. *entomophila*) or *Photorhabdus* species strains, such as Tc-proteins from Photorhabdus as described in WO98/08932. Also any variants or mutants of any one of these proteins differing in some amino acids (1-10, preferably 1-5) from any of the above named sequences, particularly the sequence of their toxic fragment, or which are fused to a transit peptide, such as a plastid transit peptide, or another protein or peptide, is included herein.

Another and particularly emphasized example of such properties is conferred tolerance to one or more herbicides, for example imidazolinones, sulphonylureas, glyphosate or phosphinothricin. Among DNA sequences encoding proteins which confer properties of tolerance to certain herbicides on the transformed plant cells and plants, mention will be particularly be made to the bar or PAT gene or the Streptomyces coelicolor gene described in WO2009/152359 which confers tolerance to glufosinate herbicides, a gene encoding a suitable EPSPS (5-Enolpyruvylshikimat-3-phosphat-synthase) which confers tolerance to herbicides having EPSPS as a target, especially herbicides such as glyphosate and its salts, a gene encoding glyphosate-n-acetyltransferase, or a gene encoding glyphosate oxidoreductase. Further suitable herbicide tolerance traits include at least one ALS (acetolactate synthase) inhibitor (e.g. WO2007/024782), a mutated Arabidopsis ALS/AHAS gene (e.g. U.S. Patent 6,855,533), genes encoding 2,4-D-monooxygenases conferring tolerance to 2,4-D (2,4- dichlorophenoxyacetic acid) and genes encoding Dicamba monooxygenases conferring tolerance to dicamba (3,6-dichloro-2- methoxybenzoic acid).

Yet another example of such properties is resistance to one or more phytopathogenic fungi, for example Asian Soybean Rust. Among DNA sequences encoding proteins which confer properties of resistance to such diseases, mention will particularly be made of the genetic material from *glycine tomentella,* for example from any one of publically available accession lines PI441001 , PI483224, PI583970, PI446958, PI499939, PI505220, PI499933, PI441008, PI505256 or PI446961 as described in WO2019/103918.

Further and particularly emphasized examples of such properties are increased resistance against bacteria and/or viruses owing, for example, to systemic acquired resistance (SAR), systemin, phytoalexins, elicitors and also resistance genes and correspondingly expressed proteins and toxins.

Particularly useful transgenic events in transgenic plants or plant cultivars which can be treated with preference in accordance with the invention include Event 531/ PV-GHBK04 (cotton, insect control, described in WO2002/040677), Event 1143-14A (cotton, insect control, not deposited, described in WO2006/128569); Event 1143-51B (cotton, insect control, not deposited, described in WO2006/128570); Event 1445 (cotton, herbicide tolerance, not deposited, described in US-A 2002-120964 or WO2002/034946); Event 17053 (rice, herbicide tolerance, deposited as PTA-9843, described in WO2010/117737); Event 17314 (rice, herbicide tolerance, deposited as PTA-9844, described in WO2010/117735); Event 281-24-236 (cotton, insect control - herbicide tolerance, deposited as PTA-6233, described in WO2005/103266 or US-A 2005-216969); Event 3006-210-23 (cotton, insect control -herbicide tolerance, deposited as PTA-6233, described in US-A 2007-143876 orWO2005/103266); Event 3272 (corn, quality trait, deposited as PTA-9972, described in WO2006/098952 or US-A 2006-230473); Event 33391 (wheat, herbicide tolerance, deposited as PTA-2347, described in WO2002/027004), Event 40416 (corn, insect control - herbicide tolerance, deposited as ATCC PTA-11508, described in WO 11/075593); Event 43A47 (corn, insect control - herbicide tolerance, deposited as ATCC PTA-11509, described in WO2011/075595); Event 5307 (corn, insect control, deposited as ATCC PTA-9561, described in WO2010/077816); Event ASR-368 (bent grass, herbicide tolerance, deposited as ATCC PTA-4816, described in US-A 2006-162007 or WO2004/053062); Event B16 (corn, herbicide tolerance, not deposited, described in US-A 2003-126634); Event BPS-CV127- 9 (soybean, herbicide tolerance, deposited as NCIMB No. 41603, described in WO2010/080829); Event BLR1 (oilseed rape, restoration of male sterility, deposited as NCIMB 41193, described in WO2005/074671), Event CE43-67B (cotton, insect control, deposited as DSM ACC2724, described in US-A 2009-217423 or WO2006/128573); Event CE44-69D (cotton, insect control, not deposited, described in US-A 2010- 0024077); Event CE44-69D (cotton, insect control, not deposited, described in WO2006/128571); Event CE46-02A (cotton, insect control, not deposited, described in WO2006/128572); Event COT102 (cotton, insect control, not deposited, described in US-A 2006-130175 or WO2004/039986); Event COT202 (cotton, insect control, not deposited, described in US-A 2007-067868 or WO2005/054479); Event COT203 (cotton, insect control, not deposited, described in WO2005/054480);); Event DAS21606-3 / 1606 (soybean, herbicide tolerance, deposited as PTA-11028, described in WO2012/033794), Event DAS40278 (corn, herbicide tolerance, deposited as ATCC PTA-10244, described in WO2011/022469); Event DAS-44406-6 / pDAB8264.44.06.1 (soybean, herbicide tolerance, deposited as PTA-11336, described in WO2012/075426), Event DAS-14536-7 /pDAB8291.45.36.2 (soybean, herbicide tolerance, deposited as PTA-11335, described in WO2012/075429), Event DAS-59122-7 (corn, insect control - herbicide tolerance, deposited as ATCC PTA 11384, described in US-A 2006-070139); Event DAS-59132 (corn, insect control - herbicide tolerance, not deposited, described in WO2009/100188); Event DAS68416 (soybean, herbicide tolerance, deposited as ATCC PTA-10442, described in WO2011/066384 or WO2011/066360); Event DP-098140-6 (corn, herbicide tolerance, deposited as ATCC PTA-8296, described in US-A 2009- 137395 orWO 08/112019); Event DP-305423-1 (soybean, quality trait, not deposited, described in US-A 2008-312082 or WO2008/054747); Event DP-32138-1 (corn, hybridization system, deposited as ATCC PTA-9158, described in US-A 2009-0210970 or WO2009/103049); Event DP-356043-5 (soybean, herbicide tolerance, deposited as ATCC PTA-8287, described in US-A 2010-0184079 or WO2008/002872); EventEE-I (brinjal, insect control, not deposited, described in WO 07/091277); Event Fil 17 (corn, herbicide tolerance, deposited as ATCC 209031, described in US-A 2006-059581 orWO 98/044140); Event FG72 (soybean, herbicide tolerance, deposited as PTA-11041, described in WO2011/063413), Event GA21 (corn, herbicide tolerance, deposited as ATCC 209033, described in US-A 2005-086719 or WO 98/044140); Event GG25 (corn, herbicide tolerance, deposited as ATCC 209032, described in US-A 2005-188434 orWO98/044140); Event GHB119 (cotton, insect control -herbicide tolerance, deposited as ATCC PTA-8398, described in WO2008/151780); Event GHB614 (cotton, herbicide tolerance, deposited as ATCC PTA-6878, described in US-A 2010-050282 or WO2007/017186); Event GJ11 (corn, herbicide tolerance, deposited as ATCC 209030, described in US-A 2005-188434 or WO98/044140); Event GM RZ13 (sugar beet, virus resistance, deposited as NCIMB-41601, described in WO2010/076212); Event H7-1 (sugar beet, herbicide tolerance, deposited as NCIMB 41158 or NCIMB 41159, described in US-A 2004-172669 or WO 2004/074492); Event JOPLIN1 (wheat, disease tolerance, not deposited, described in US-A 2008-064032); Event LL27 (soybean, herbicide tolerance, deposited as NCIMB41658, described in WO2006/108674 or US-A 2008-320616); Event LL55 (soybean, herbicide tolerance, deposited as NCIMB 41660, described in WO 2006/108675 or US-A 2008-196127); Event LLcotton25 (cotton, herbicide tolerance, deposited as ATCC PTA-3343, described in WO2003/013224 or US- A 2003-097687); Event LLRICE06 (rice, herbicide tolerance, deposited as ATCC 203353, described in US 6,468,747 or WO2000/026345); Event LLRice62 (rice, herbicide tolerance, deposited as ATCC 203352, described in WO2000/026345), Event LLRICE601 (rice, herbicide tolerance, deposited as ATCC PTA-2600, described in US-A 2008-2289060 or WO2000/026356); Event LY038 (corn, quality trait, deposited as ATCC PTA-5623, described in US-A 2007-028322 or WO2005/061720); Event MIR162 (corn, insect control, deposited as PTA-8166, described in US-A 2009-300784 or WO2007/142840); Event MIR604 (corn, insect control, not deposited, described in US-A 2008-167456 or WO2005/103301); Event MON15985 (cotton, insect control, deposited as ATCC PTA-2516, described in US-A 2004-250317 or WO2002/100163); Event MON810 (corn, insect control, not deposited, described in USA 2002-102582); Event MON863 (corn, insect control, deposited as ATCC PTA-2605, described in WO2004/011601 or US-A 2006-095986); Event MON87427 (corn, pollination control, deposited as ATCC PTA-7899, described in WO2011/062904); Event MON87460 (corn, stress tolerance, deposited as ATCC PTA-8910, described in WO2009/111263 or US-A 2011-0138504); Event MON87701 (soybean, insect control, deposited as ATCC PTA- 8194, described in US-A 2009-130071 or WO2009/064652); Event MON87705 (soybean, quality trait - herbicide tolerance, deposited as ATCC PTA-9241, described in US-A 2010-0080887 or WO2010/037016); Event MON87708 (soybean, herbicide tolerance, deposited as ATCC PTA-9670, described in WO2011/034704); Event MON87712 (soybean, yield, deposited as PTA-10296, described in WO2012/051199), Event MON87754 (soybean, quality trait, deposited as ATCC PTA-9385, described in WO2010/024976); Event MON87769 (soybean, quality trait, deposited as ATCC PTA-8911, described in US-A 2011-0067141 or WO2009/102873); Event MON88017 (corn, insect control - herbicide tolerance, deposited as ATCC PTA-5582, described in US-A 2008-028482 or WO2005/059103); Event MON88913 (cotton, herbicide tolerance, deposited as ATCC PTA-4854, described in WO2004/072235 or US-A 2006-059590); Event MON88302 (oilseed rape, herbicide tolerance, deposited as PTA-10955, described in WO2011/153186), Event MON88701 (cotton, herbicide tolerance, deposited as PTA-11754, described in WO2012/134808), Event MON89034 (corn, insect control, deposited as ATCC PTA-7455, described in WO 07/140256 or US-A 2008-260932); Event MON89788 (soybean, herbicide tolerance, deposited as ATCC PTA-6708, described in US-A 2006-282915 or WO2006/130436); Event MSl 1 (oilseed rape, pollination control - herbicide tolerance, deposited as ATCC PTA-850 or PTA-2485, described in WO2001/031042); Event MS8 (oilseed rape, pollination control - herbicide tolerance, deposited as ATCC PTA-730, described in WO2001/041558 or US-A 2003-188347); Event NK603 (corn, herbicide tolerance, deposited as ATCC PTA-2478, described in US-A 2007-292854); Event PE-7 (rice, insect control, not deposited, described in WO2008/114282); Event RF3 (oilseed rape, pollination control - herbicide tolerance, deposited as ATCC PTA-730, described in WO2001/041558 or US-A 2003-188347); Event RT73 (oilseed rape, herbicide tolerance, not deposited, described in WO2002/036831 or US-A 2008-070260); Event SYHT0H2 / SYN-000H2-5 (soybean, herbicide tolerance, deposited as PTA-11226, described in WO2012/082548), Event T227-1 (sugar beet, herbicide tolerance, not deposited, described in WO2002/44407 or US-A 2009-265817); Event T25 (corn, herbicide tolerance, not deposited, described in US-A 2001-029014 or WO2001/051654); Event T304-40 (cotton, insect control - herbicide tolerance, deposited as ATCC PTA-8171, described in US-A 2010-077501 or WO2008/122406); Event T342-142 (cotton, insect control, not deposited, described in WO2006/128568); Event TC1507 (corn, insect control - herbicide tolerance, not deposited, described in US-A 2005-039226 or WO2004/099447); Event VIP1034 (corn, insect control - herbicide tolerance, deposited as ATCC PTA-3925, described in WO2003/052073), Event 32316 (corn, insect control-herbicide tolerance, deposited as PTA-11507, described in WO2011/084632), Event 4114 (corn, insect control-herbicide tolerance, deposited as PTA-11506, described in WO2011/084621), event EE-GM3 / FG72 (soybean, herbicide tolerance, ATCC Accession N° PTA-11041) optionally stacked with event EE-GM1/LL27 or event EE-GM2/LL55 (WO2011/063413A2), event DAS-68416-4 (soybean, herbicide tolerance, ATCC Accession N° PTA-10442, WO2011/066360A1), event DAS-68416-4 (soybean, herbicide tolerance, ATCC Accession N° PTA-10442, WO2011/066384A1), event DP-040416-8 (corn, insect control, ATCC Accession N° PTA-11508, WO2011/075593A1), event DP-043A47-3 (corn, insect control, ATCC Accession N° PTA-11509, WO2011/075595A1), event DP- 004114-3 (corn, insect control, ATCC Accession N° PTA-11506, WO2011/084621A1), event DP-032316-8 (corn, insect control, ATCC Accession N° PTA-11507, WO2011/084632A1), event MON-88302-9 (oilseed rape, herbicide tolerance, ATCC Accession N° PTA-10955, WO2011/153186A1), event DAS-21606-3 (soybean, herbicide tolerance, ATCC Accession No. PTA-11028, WO2012/033794A2), event MON-87712-4 (soybean, quality trait, ATCC Accession N°. PTA-10296, WO2012/051199A2), event DAS-44406-6 (soybean, stacked herbicide tolerance, ATCC Accession N°. PTA-11336, WO2012/075426A1), event DAS-14536-7 (soybean, stacked herbicide tolerance, ATCC Accession N°. PTA-11335, WO2012/075429A1), event SYN-000H2-5 (soybean, herbicide tolerance, ATCC Accession N°. PTA-11226, WO2012/082548A2), event DP-061061-7 (oilseed rape, herbicide tolerance, no deposit N° available, WO2012071039A1), event DP-073496-4 (oilseed rape, herbicide tolerance, no deposit N° available, US2012131692), event 8264.44.06.1 (soybean, stacked herbicide tolerance, Accession N° PTA-11336, WO2012075426A2), event 8291.45.36.2 (soybean, stacked herbicide tolerance, Accession N°. PTA-11335, WO2012075429A2), event SYHT0H2 (soybean, ATCC Accession N°. PTA-11226, WO2012/082548A2), event MON88701 (cotton, ATCC Accession N° PTA-11754, WO2012/134808A1), event KK179-2 (alfalfa, ATCC Accession N° PTA-11833, WO2013/003558A1), event pDAB8264.42.32.1 (soybean, stacked herbicide tolerance, ATCC Accession N° PTA-11993, WO2013/010094A1), event MZDT09Y (corn, ATCC Accession N° PTA-13025, WO2013/012775A1).

Further, a list of such transgenic event(s) is provided by the United States Department of Agriculture's (USDA) Animal and Plant Health Inspection Service (APHIS) and can be found on their website on the world wide web at aphis.usda.gov. For this application, the status of such list as it is/was on the filing date of this application, is relevant.

The genes/events which impart the desired traits in question may also be present in combinations with one another in the transgenic plants. Examples of transgenic plants which may be mentioned are the important crop plants, such as cereals (wheat, rice, triticale, barley, rye, oats), maize, soya beans, potatoes, sugar beet, sugar cane, tomatoes, peas and other types of vegetable, cotton, tobacco, oilseed rape and also fruit plants (with the fruits apples, pears, citrus fruits and grapes), with particular emphasis being given to maize, soya beans, wheat, rice, potatoes, cotton, sugar cane, tobacco and oilseed rape. Traits which are particularly emphasized are the increased resistance of the plants to insects, arachnids, nematodes and slugs and snails, as well as the increased resistance of the plants to one or more herbicides.

Commercially available examples of such plants, plant parts or plant seeds that may be treated with preference in accordance with the invention include commercial products, such as plant seeds, sold or distributed under the GENUITY®, DROUGHTGARD®, SMARTSTAX®, RIB COMPLETE®, ROUNDUP READY®, VT DOUBLE PRO®, VT TRIPLE PRO®, BOLLGARD II®, ROUNDUP READY 2 YIELD®, YIELDGARD®, ROUNDUP READY® 2 XTEN^{DTM}, INTACTA RR2 PRO®, VISTIVE GOLD®, and/or XTENDFLEX™ trade names.

### Pathogens

Non-limiting examples of pathogens of fungal diseases which may be treated in accordance with the invention include:
diseases caused by powdery mildew pathogens, for example *Blumeria* species, for example *Blumeria graminis; Podosphaera* species, for example *Podosphaera leucotricha; Sphaerotheca* species, for example *Sphaerotheca fuliginea; Uncinula* species, for example *Uncinula necator;*
diseases caused by rust disease pathogens, for example *Gymnosporangium* species, for example *Gymnosporangium sabinae; Hemileia* species, for example *Hemileia vastatrix; Phakopsora* species, for example *Phakopsora pachyrhizi* or *Phakopsora meibomiae; Puccinia* species, for example *Puccinia recondita, Puccinia graminis* oder *Puccinia striiformis; Uromyces* species, for example *Uromyces appendiculatus;*
diseases caused by pathogens from the group of the Oomycetes, for example *Albugo* species, for example *Albugo candida; Bremia* species, for example *Bremia lactucae; Peronospora* species, for example *Peronospora pisi or P. brassicae; Phytophthora* species, for example *Phytophthora infestans; Plasmopara* species, for example *Plasmopara viticola; Pseudoperonospora* species, for example *Pseudoperonospora humuli* or *Pseudoperonospora cubensis; Pythium* species, for example *Pythium ultimum;*
leaf blotch diseases and leaf wilt diseases caused, for example, by *Alternaria* species, for example *Alternaria solani; Cercospora* species, for example *Cercospora beticola; Cladiosporium* species, for example *Cladiosporium cucumerinum; Cochliobolus* species, for example Cochliobolus sativus (conidial form: *Drechslera,* syn: *Helminthosporium*) or *Cochliobolus miyabeanus; Colletotrichum* species, for example *Colletotrichum lindemuthanium; Corynespora* species, for example *Corynespora cassiicola; Cycloconium* species, for example *Cycloconium oleaginum; Diaporthe* species, for example *Diaporthe citri; Elsinoe* species, for example *Elsinoe fawcettii; Gloeosporium* species, for example *Gloeosporium laeticolor; Glomerella* species, for example *Glomerella cingulata; Guignardia* species, for example *Guignardia bidwelli; Leptosphaeria* species, for example *Leptosphaeria maculans; Magnaporthe* species, for example *Magnaporthe grisea; Microdochium* species, for example *Microdochium nivale; Mycosphaerella* species, for example *Mycosphaerella graminicola, Mycosphaerella arachidicola* or *Mycosphaerella fijiensis; Phaeosphaeria* species, for example *Phaeosphaeria nodorum; Pyrenophora* species, for example *Pyrenophora teres* or *Pyrenophora tritici repentis; Ramularia* species, for example *Ramularia collo-cygni* or *Ramularia areola; Rhynchosporium* species, for example *Rhynchosporium secalis; Septoria* species, for example *Septoria apii* or *Septoria lycopersici; Stagonospora* species, for example *Stagonospora nodorum; Typhula* species, for example *Typhula incarnata; Venturia* species, for example *Venturia inaequalis;*
root and stem diseases caused, for example, by *Corticium* species, for example *Corticium graminearum; Fusarium* species, for example *Fusarium oxysporum; Gaeumannomyces* species, for example *Gaeumannomyces graminis; Plasmodiophora* species, for example *Plasmodiophora brassicae; Rhizoctonia* species, for example *Rhizoctonia solani; Sarocladium* species, for example *Sarocladium oryzae; Sclerotium* species, for example *Sclerotium oryzae; Tapesia* species, for example *Tapesia acuformis; Thielaviopsis* species, for example *Thielaviopsis basicola;*
ear and panicle diseases (including corn cobs) caused, for example, by *Alternaria* species, for example *Alternaria spp.; Aspergillus* species, for example *Aspergillus flavus; Cladosporium* species, for example *Cladosporium cladosporioides; Claviceps* species, for example *Claviceps purpurea; Fusarium* species, for example *Fusarium culmorum; Gibberella* species, for example *Gibberella zeae; Monographella* species, for example *Monographella nivalis; Stagnospora* species, for example *Stagnospora nodorum;*
diseases caused by smut fungi, for example *Sphacelotheca* species, for example *Sphacelotheca reiliana; Tilletia* species, for example *Tilletia caries* or *Tilletia controversa; Urocystis* species, for example *Urocystis occulta; Ustilago* species, for example *Ustilago nuda;*
fruit rot caused, for example, by *Aspergillus* species, for example *Aspergillus flavus; Botrytis* species, for example *Botrytis cinerea; Monilinia* species, for example *Monilinia laxa; Penicillium* species, for example *Penicillium expansum* or *Penicillium purpurogenum; Rhizopus* species, for example *Rhizopus stolonifer; Sclerotinia* species, for example *Sclerotinia sclerotiorum; Verticilium* species, for example *Verticilium alboatrum;*
seed- and soil-borne rot and wilt diseases, and also diseases of seedlings, caused, for example, by *Alternaria* species, for example *Alternaria brassicicola; Aphanomyces* species, for example *Aphanomyces euteiches; Ascochyta* species, for example *Ascochyta lentis; Aspergillus* species, for example *Aspergillus flavus; Cladosporium* species, for example *Cladosporium herbarum; Cochliobolus* species, for example *Cochliobolus sativus* (conidial form: *Drechslera,* Bipolaris Syn: *Helminthosporium*); *Colletotrichum* species, for example *Colletotrichum coccodes; Fusarium* species, for example *Fusarium culmorum; Gibberella* species, for example *Gibberella zeae; Macrophomina* species, for example *Macrophomina phaseolina; Microdochium* species, for example *Microdochium nivale; Monographella* species, for example *Monographella nivalis; Penicillium* species, for example *Penicillium expansum; Phoma* species, for example *Phoma lingam; Phomopsis* species, for example *Phomopsis sojae; Phytophthora* species, for example *Phytophthora cactorum; Pyrenophora* species, for example *Pyrenophora graminea; Pyricularia* species, for example *Pyricularia oryzae; Pythium* species, for example *Pythium ultimum; Rhizoctonia* species, for example *Rhizoctonia solani; Rhizopus* species, for example *Rhizopus oryzae; Sclerotium* species, for example *Sclerotium rolfsii; Septoria* species, for example *Septoria nodorum; Typhula* species, for example *Typhula incarnata; Verticillium* species, for example *Verticillium dahliae;*
cancers, galls and witches' broom caused, for example, by *Nectria* species, for example *Nectria galligena;*
wilt diseases caused, for example, by *Verticillium* species, for example *Verticillium longisporum; Fusarium* species, for example *Fusarium oxysporum;*
deformations of leaves, flowers and fruits caused, for example, by *Exobasidium* species, for example *Exobasidium vexans; Taphrina* species, for example *Taphrina deformans;*
degenerative diseases in woody plants, caused, for example, by *Esca* species, for example *Phaeomoniella chlamydospora, Phaeoacremonium aleophilum* or *Fomitiporia mediterranea; Ganoderma* species, for example *Ganoderma boninense;*
diseases of plant tubers caused, for example, by *Rhizoctonia* species, for example *Rhizoctonia solani; Helminthosporium* species, for example *Helminthosporium solani;*
diseases caused by bacterial pathogens, for example *Xanthomonas* species, for example *Xanthomonas campestris pv. oryzae; Pseudomonas* species, for example *Pseudomonas syringae pv. lachrymans; Erwinia* species, for example *Erwinia amylovora; Liberibacter* species, for example *Liberibacter asiaticus; Xyella* species, for example *Xylella fastidiosa; Ralstonia* species, for example *Ralstonia solanacearum; Dickeya* species, for example *Dickeya solani; Clavibacter* species, for example *Clavibacter michiganensis; Streptomyces* species, for example *Streptomyces scabies.*
diseases of soya beans:
   Fungal diseases on leaves, stems, pods and seeds caused, for example, by *Alternaria* leaf spot *(Alternaria spec. atrans tenuissima*), *Anthracnose* (*Colletotrichum gloeosporoides dematium var. truncatum*), brown spot (*Septoria glycines*), *cercospora* leaf spot and blight (*Cercospora kikuchii*), *choanephora* leaf blight (*Choanephora infundibulifera trispora (Syn.)*), *dactuliophora* leaf spot (*Dactuliophora glycines*), downy mildew *(Peronospora manshurica*), *drechslera* blight (*Drechslera glycini*), frogeye leaf spot (*Cercospora sojina*), *leptosphaerulina* leaf spot (*Leptosphaerulina trifolii*), *phyllostica* leaf spot (*Phyllosticta sojaecola*), pod and stem blight (*Phomopsis sojae*), powdery mildew (*Microsphaera diffusa*), *pyrenochaeta* leaf spot (*Pyrenochaeta glycines*), rhizoctonia aerial, foliage, and web blight (*Rhizoctonia solani*), rust (*Phakopsora pachyrhizi, Phakopsora meibomiae*), scab (*Sphaceloma glycines*), *stemphylium* leaf blight (*Stemphylium botryosum*), sudden death syndrome (*Fusarium virguliforme*), target spot (*Corynespora cassiicola*)*.*

Fungal diseases on roots and the stem base caused, for example, by black root rot (*Calonectria crotalariae*), charcoal rot (*Macrophomina phaseolina*), fusarium blight or wilt, root rot, and pod and collar rot (*Fusarium oxysporum, Fusarium orthoceras, Fusarium semitectum, Fusarium equiseti*), mycoleptodiscus root rot (*Mycoleptodiscus terrestris*), neocosmospora (*Neocosmospora vasinfecta*), pod and stem blight (*Diaporthe phaseolorum*), stem canker (*Diaporthe phaseolorum var. caulivora*), phytophthora rot (*Phytophthora megasperma*), brown stem rot (*Phialophora gregata*), pythium rot (*Pythium aphanidermatum, Pythium irregulare, Pythium debaryanum, Pythium myriotylum, Pythium ultimum*), rhizoctonia root rot, stem decay, and damping-off (*Rhizoctonia solani*), sclerotinia stem decay (*Sclerotinia sclerotiorum*), sclerotinia southern blight (*Sclerotinia rolfsii*), thielaviopsis root rot (*Thielaviopsis basicola*)*.*

### Mycotoxins

In addition, the compound and the composition of the invention may reduce the mycotoxin content in the harvested material and the foods and feeds prepared therefrom. Mycotoxins include particularly, but not exclusively, the following: deoxynivalenol (DON), nivalenol, 15-Ac-DON, 3-Ac-DON, T2- and HT2-toxin, fumonisins, zearalenon, moniliformin, fusarin, diaceotoxyscirpenol (DAS), beauvericin, enniatin, fusaroproliferin, fusarenol, ochratoxins, patulin, ergot alkaloids and aflatoxins which can be produced, for example, by the following fungi: *Fusarium* spec., such as *F. acuminatum, F. asiaticum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides,* and also by *Aspergillus* spec., such *as A. flavus, A. parasiticus, A. nomius, A. ochraceus, A. clavatus, A. terreus, A. versicolor, Penicillium* spec., such as *P. verrucosum, P. viridicatum, P. citrinum, P. expansum, P. claviforme, P. roqueforti, Claviceps* spec., such as *C. purpurea, C. fusiformis, C. paspali, C. africana, Stachybotrys* spec. and others.

### Material Protection

The compound and the composition of the invention may also be used in the protection of materials, especially for the protection of industrial materials against attack and destruction by phytopathogenic fungi.

In addition, the compound and the composition of the invention may be used as antifouling compositions, alone or in combinations with other active ingredients.

Industrial materials in the present context are understood to mean inanimate materials which have been prepared for use in industry. For example, industrial materials which are to be protected from microbial alteration or destruction may be adhesives, glues, paper, wallpaper and board/cardboard, textiles, carpets, leather, wood, fibers and tissues, paints and plastic articles, cooling lubricants and other materials which can be infected with or destroyed by microorganisms. Parts of production plants and buildings, for example cooling-water circuits, cooling and heating systems and ventilation and air-conditioning units, which may be impaired by the proliferation of microorganisms may also be mentioned within the scope of the materials to be protected. Industrial materials within the scope of the present invention preferably include adhesives, sizes, paper and card, leather, wood, paints, cooling lubricants and heat transfer fluids, more preferably wood.

The compound and the composition of the invention may prevent adverse effects, such as rotting, decay, discoloration, decoloration or formation of mould.

In the case of treatment of wood the compound and the composition of the invention may also be used against fungal diseases liable to grow on or inside timber.

Timber means all types of species of wood, and all types of working of this wood intended for construction, for example solid wood, high-density wood, laminated wood, and plywood. In addition, the compound and the composition of the invention may be used to protect objects which come into contact with saltwater or brackish water, especially hulls, screens, nets, buildings, moorings and signalling systems, from fouling.

The compound and the composition of the invention may also be employed for protecting storage goods. Storage goods are understood to mean natural substances of vegetable or animal origin or processed products thereof which are of natural origin, and for which long-term protection is desired. Storage goods of vegetable origin, for example plants or plant parts, such as stems, leaves, tubers, seeds, fruits, grains, may be protected freshly harvested or after processing by (pre)drying, moistening, comminuting, grinding, pressing or roasting. Storage goods also include timber, both unprocessed, such as construction timber, electricity poles and barriers, or in the form of finished products, such as furniture. Storage goods of animal origin are, for example, hides, leather, furs and hairs. The compound and the composition of the invention may prevent adverse effects, such as rotting, decay, discoloration, decoloration or formation of mould.

Microorganisms capable of degrading or altering industrial materials include, for example, bacteria, fungi, yeasts, algae and slime organisms. The compound and the composition of the invention preferably act against fungi, especially moulds, wood-discoloring and wood-destroying fungi (*Ascomycetes, Basidiomycetes, Deuteromycetes* and *Zygomycetes*), and against slime organisms and algae. Examples include microorganisms of the following genera: *Alternaria,* such as *Alternaria tenuis; Aspergillus,* such as *Aspergillus niger; Chaetomium,* such as *Chaetomium globosum; Coniophora,* such as *Coniophora puetana; Lentinus,* such as *Lentinus tigrinus; Penicillium,* such as *Penicillium glaucum; Polyporus,* such as *Polyporus versicolor; Aureobasidium,* such as *Aureobasidium pullulans; Sclerophoma,* such as *Sclerophoma pityophila; Trichoderma,* such as *Trichoderma viride; Ophiostoma spp., Ceratocystis spp., Humicola spp., Petriella spp., Trichurus spp., Coriolus spp., Gloeophyllum spp., Pleurotus spp., Poria spp., Serpula spp.* and *Tyromyces spp., Cladosporium spp., Paecilomyces spp. Mucor spp., Escherichia,* such as *Escherichia coli; Pseudomonas,* such as *Pseudomonas aeruginosa; Staphylococcus,* such as *Staphylococcus aureus, Candida spp.* and *Saccharomyces spp.,* such as *Saccharomyces cerevisae.*

### Seed Treatment

The compound and the composition of the invention may also be used to protect seeds from unwanted microorganisms, such as phytopathogenic microorganisms, for instance phytopathogenic fungi or phytopathogenic oomycetes. The term seed(s) as used herein include dormant seeds, primed seeds, pregerminated seeds and seeds with emerged roots and leaves.

Thus, the present invention also relates to a method for protecting seeds from unwanted microorganisms which comprises the step of treating the seeds with the compound or the composition of the invention.

The treatment of seeds with the compound or the composition of the invention protects the seeds from phytopathogenic microorganisms, but also protects the germinating seeds, the emerging seedlings and the plants after emergence from the treated seeds. Therefore, the present invention also relates to a method for protecting seeds, germinating seeds and emerging seedlings.

The seeds treatment may be performed prior to sowing, at the time of sowing or shortly thereafter.

When the seeds treatment is performed prior to sowing (e.g. so-called on-seed applications), the seeds treatment may be performed as follows: the seeds may be placed into a mixer with a desired amount of the compound or the composition of the invention, the seeds and the compound or the composition of the invention are mixed until an homogeneous distribution on seeds is achieved. If appropriate, the seeds may then be dried.

The invention also relates to seeds coated with the compound or the composition of the invention.

Preferably, the seeds are treated in a state in which it is sufficiently stable for no damage to occur in the course of treatment. In general, seeds can be treated at any time between harvest and shortly after sowing. It is customary to use seeds which have been separated from the plant and freed from cobs, shells, stalks, coats, hairs or the flesh of the fruits. For example, it is possible to use seeds which have been harvested, cleaned and dried down to a moisture content of less than 15% by weight. Alternatively, it is also possible to use seeds which, after drying, for example, have been treated with water and then dried again, or seeds just after priming, or seeds stored in primed conditions or pre-germinated seeds, or seeds sown on nursery trays, tapes or paper.

The amount of the compound or the composition of the invention applied to the seeds is typically such that the germination of the seed is not impaired, or that the resulting plant is not damaged. This must be ensured particularly in case the the compound of the invention would exhibit phytotoxic effects at certain application rates. The intrinsic phenotypes of transgenic plants should also be taken into consideration when determining the amount of the compound of the invention to be applied to the seed in order to achieve optimum seed and germinating plant protection with a minimum amount of compound being employed.

The compound of the invention can be applied as such, directly to the seeds, i.e. without the use of any other components and without having been diluted. Also the composition of the invention can be applied to the seeds.

The compound and the composition of the invention are suitable for protecting seeds of any plant variety. Preferred seeds are that of cereals (such as wheat, barley, rye, millet, triticale, and oats), oilseed rape, maize, cotton, soybean, rice, potatoes, sunflower, beans, coffee, peas, beet (e.g. sugar beet and fodder beet), peanut, vegetables (such as tomato, cucumber, onions and lettuce), lawns and ornamental plants. More preferred are seeds of wheat, soybean, oilseed rape, maize and rice.

The compound and the composition of the invention may be used for treating transgenic seeds, in particular seeds of plants capable of expressing a polypeptide or protein which acts against pests, herbicidal damage or abiotic stress, thereby increasing the protective effect. Seeds of plants capable of expressing a polypeptide or protein which acts against pests, herbicidal damage or abiotic stress may contain at least one heterologous gene which allows the expression of said polypeptide or protein. These heterologous genes in transgenic seeds may originate, for example, from microorganisms of the species Bacillus, Rhizobium, Pseudomonas, Serratia, Trichoderma, Clavibacter, Glomus or Gliocladium. These heterologous genes preferably originate from Bacillus sp., in which case the gene product is effective against the European corn borer and/or the Western corn rootworm. Particularly preferably, the heterologous genes originate from Bacillus thuringiensis.

### Application

The compound of the invention can be applied as such, or for example in the form of as ready-to-use solutions, emulsions, water- or oil-based suspensions, powders, wettable powders, pastes, soluble powders, dusts, soluble granules, granules for broadcasting, suspoemulsion concentrates, natural products impregnated with the compound of the invention, synthetic substances impregnated with the compound of the invention, fertilizers or microencapsulations in polymeric substances.

Application is accomplished in a customary manner, for example by watering, spraying, atomizing, broadcasting, dusting, foaming or spreading-on. It is also possible to deploy the compound of the invention by the ultra-low volume method, via a drip irrigation system or drench application, to apply it in-furrow or to inject it into the soil stem or trunk. It is further possible to apply the compound of the invention by means of a wound seal, paint or other wound dressing.

The effective and plant-compatible amount of the compound of the invention which is applied to the plants, plant parts, fruits, seeds or soil will depend on various factors, such as the compound/composition employed, the subject of the treatment (plant, plant part, fruit, seed or soil), the type of treatment (dusting, spraying, seed dressing), the purpose of the treatment (curative and protective), the type of microorganisms, the development stage of the microorganisms, the sensitivity of the microorganisms, the crop growth stage and the environmental conditions.

When the compound of the invention is used as a fungicide, the application rates can vary within a relatively wide range, depending on the kind of application. For the treatment of plant parts, such as leaves, the application rate may range from 0.1 to 10 000 g/ha, preferably from 10 to 1000 g/ha, more preferably from 50 to 300 g/ha (in the case of application by watering or dripping, it is even possible to reduce the application rate, especially when inert substrates such as rockwool or perlite are used). For the treatment of seeds, the application rate may range from 0.1 to 200 g per 100 kg of seeds, preferably from 1 to 150 g per 100 kg of seeds, more preferably from 2.5 to 25 g per 100 kg of seeds, even more preferably from 2.5 to 12.5 g per 100 kg of seeds. For the treatment of soil, the application rate may range from 0.1 to 10 000 g/ha, preferably from 1 to 5000 g/ha.

These application rates are merely examples and are not intended to limit the scope of the present invention.

The compound and the composition of the invention can be used in combination with models e.g. embedded in computer programs for site specific crop management, satellite farming, precision farming or precision agriculture. Such models support the site specific management of agricultural sites with data from various sources such as soils, weather, crops (e.g. type, growth stage, plant health), weeds (e.g. type, growth stage), diseases, pests, nutrients, water, moisture, biomass, satellite data, yield etc. with the purpose to optimize profitability, sustainability and protection of the environment. In particular, such models can help to optimize agronomical decisions, control the precision of pesticide applications and record the work performed.

As an example, the compound of the invention can be applied to a crop plant according to appropriate dose regime if a model models the development of a fungal disease and calculates that a threshold has been reached for which it is recommendable to apply the compound of the invention to the crop plant.

Commercially available systems which include agronomic models are e.g. FieldScripts™ from The Climate Corporation, Xarvio™ from BASF, AGLogic™ from John Deere, etc.

The compound of the invention can also be used in combination with smart spraying equipment such as e.g. spot spraying or precision spraying equipment attached to or housed within a farm vehicle such as a tractor, robot, helicopter, airplane, unmanned aerial vehicle (UAV) such as a drone, etc. Such an equipment usually includes input sensors (such as e.g. a camera) and a processing unit configured to analyze the input data and configured to provide a decision based on the analysis of the input data to apply the compound of the invention to the crop plants (respectively the weeds) in a specific and precise manner. The use of such smart spraying equipment usually also requires positions systems (e.g. GPS receivers) to localize recorded data and to guide or to control farm vehicles; geographic information systems (GIS) to represent the information on intelligible maps, and appropriate farm vehicles to perform the required farm action such as the spraying.

In an example, fungal diseases can be detected from imagery acquired by a camera. In an example fungal diseases can be identified and/or classified based on that imagery. Such identification and/ classification can make use of image processing algorithms. Such image processing algorithms can utilize machine learning algorithms, such as trained neutral networks, decision trees and utilize artificial intelligence algorithms. In this manner, the compounds described herein can be applied only where needed.

### Antimycotic Effects

The compound and the composition of the invention may also have very good antimycotic effects. They have a very broad antimycotic activity spectrum, especially against dermatophytes and yeasts, moulds and diphasic fungi (for example against Candida species, such as Candida albicans, Candida glabrata), and Epidermophyton floccosum, Aspergillus species, such as Aspergillus niger and Aspergillus fumigatus, Trichophyton species, such as Trichophyton mentagrophytes, Microsporon species such as Microsporon canis and audouinii. The enumeration of these fungi by no means constitutes a restriction of the mycotic spectrum covered, and is merely of illustrative character.

The compound and the composition of the invention may also be used to control important fungal pathogens in fish and crustacea farming, e.g. saprolegnia diclina in trouts, saprolegnia parasitica in crayfish.

The compound and the composition of the invention may therefore be used both in medical and in non-medical applications.

### Plant Growth Regulation

The compound and the composition of the invention may, at particular concentrations or application rates, also be used as herbicides, safeners, growth regulators or agents to improve plant properties, or as microbicides, for example as bactericides, viricides (including compositions against viroids) or as compositions against MLO (Mycoplasma-like organisms) and RLO (Rickettsia-like organisms).

The compound and the composition of the invention may intervene in physiological processes of plants and may therefore also be used as plant growth regulators. Plant growth regulators may exert various effects on plants. The effect of the substances depends essentially on the time of application in relation to the developmental stage of the plant, and also on the amounts of active ingredient applied to the plants or their environment and on the type of application. In each case, growth regulators should have a particular desired effect on the crop plants.

Growth regulating effects, comprise earlier germination, better emergence, more developed root system and/or improved root growth, increased ability of tillering, more productive tillers, earlier flowering, increased plant height and/or biomass, shorting of stems, improvements in shoot growth, number of kernels/ear, number of ears/m², number of stolons and/or number of flowers, enhanced harvest index, bigger leaves, less dead basal leaves, improved phyllotaxy, earlier maturation / earlier fruit finish, homogenous riping, increased duration of grain filling, better fruit finish, bigger fruit/vegetable size, sprouting resistance and reduced lodging.

Increased or improved yield is referring to total biomass per hectare, yield per hectare, kernel/fruit weight, seed size and/or hectolitre weight as well as to improved product quality, comprising:
improved processability relating to size distribution (kernel, fruit), homogenous riping, grain moisture, better milling, better vinification, better brewing, increased juice yield, harvestability, digestibility, sedimentation value, falling number, pod stability, storage stability, improved fiber length/strength/uniformity, increase of milk and/or meet quality of silage fed animals, adaptation to cooking and frying;
improved marketability relating to improved fruit/grain quality, size distribution (kernel, fruit), increased storage / shelf-life, firmness / softness, taste (aroma, texture), grade (size, shape, number of berries), number of berries/fruits per bunch, crispness, freshness, coverage with wax, frequency of physiological disorders, colour;
increased desired ingredients such as e.g. protein content, fatty acids, oil content, oil quality, aminoacid composition, sugar content, acid content (pH), sugar/acid ratio (Brix), polyphenols, starch content, nutritional quality, gluten content/index, energy content, taste;
decreased undesired ingredients such as e.g. less mycotoxines, less aflatoxins, geosmin level, phenolic aromas, lacchase, polyphenol oxidases and peroxidases, nitrate content.

Plant growth-regulating compounds can be used, for example, to slow down the vegetative growth of the plants. Such growth depression is of economic interest, for example, in the case of grasses, since it is thus possible to reduce the frequency of grass cutting in ornamental gardens, parks and sport facilities, on roadsides, at airports or in fruit crops. Also of significance is the inhibition of the growth of herbaceous and woody plants on roadsides and in the vicinity of pipelines or overhead cables, or quite generally in areas where vigorous plant growth is unwanted.

Also important is the use of growth regulators for inhibition of the longitudinal growth of cereal. This reduces or completely eliminates the risk of lodging of the plants prior to harvest. In addition, growth regulators in the case of cereals can strengthen the culm, which also counteracts lodging. The employment of growth regulators for shortening and strengthening culms allows the deployment of higher fertilizer volumes to increase the yield, without any risk of lodging of the cereal crop.

In many crop plants, vegetative growth depression allows denser planting, and it is thus possible to achieve higher yields based on the soil surface. Another advantage of the smaller plants obtained in this way is that the crop is easier to cultivate and harvest.

Reduction of the vegetative plant growth may also lead to increased or improved yields because the nutrients and assimilates are of more benefit to flower and fruit formation than to the vegetative parts of the plants.

Alternatively, growth regulators can also be used to promote vegetative growth. This is of great benefit when harvesting the vegetative plant parts. However, promoting vegetative growth may also promote generative growth in that more assimilates are formed, resulting in more or larger fruits.

Furthermore, beneficial effects on growth or yield can be achieved through improved nutrient use efficiency, especially nitrogen (N)-use efficiency, phosphorous (P)-use efficiency, water use efficiency, improved transpiration, respiration and/or CO₂ assimilation rate, better nodulation, improved Cametabolism.

Likewise, growth regulators can be used to alter the composition of the plants, which in turn may result in an improvement in quality of the harvested products. Under the influence of growth regulators, parthenocarpic fruits may be formed. In addition, it is possible to influence the sex of the flowers. It is also possible to produce sterile pollen, which is of great importance in the breeding and production of hybrid seed.

Use of growth regulators can control the branching of the plants. On the one hand, by breaking apical dominance, it is possible to promote the development of side shoots, which may be highly desirable particularly in the cultivation of ornamental plants, also in combination with an inhibition of growth. On the other hand, however, it is also possible to inhibit the growth of the side shoots. This effect is of particular interest, for example, in the cultivation of tobacco or in the cultivation of tomatoes.

Under the influence of growth regulators, the amount of leaves on the plants can be controlled such that defoliation of the plants is achieved at a desired time. Such defoliation plays a major role in the mechanical harvesting of cotton, but is also of interest for facilitating harvesting in other crops, for example in viticulture. Defoliation of the plants can also be undertaken to lower the transpiration of the plants before they are transplanted.

Furthermore, growth regulators can modulate plant senescence, which may result in prolonged green leaf area duration, a longer grain filling phase, improved yield quality.

Growth regulators can likewise be used to regulate fruit dehiscence. On the one hand, it is possible to prevent premature fruit dehiscence. On the other hand, it is also possible to promote fruit dehiscence or even flower abortion to achieve a desired mass ("thinning"). In addition it is possible to use growth regulators at the time of harvest to reduce the forces required to detach the fruits, in order to allow mechanical harvesting or to facilitate manual harvesting.

Growth regulators can also be used to achieve faster or else delayed ripening of the harvested material before or after harvest. This is particularly advantageous as it allows optimal adjustment to the requirements of the market. Moreover, growth regulators in some cases can improve the fruit colour. In addition, growth regulators can also be used to synchronize maturation within a certain period of time. This establishes the prerequisites for complete mechanical or manual harvesting in a single operation, for example in the case of tobacco, tomatoes or coffee.

By using growth regulators, it is additionally possible to influence the resting of seed or buds of the plants, such that plants such as pineapple or ornamental plants in nurseries, for example, germinate, sprout or flower at a time when they are normally not inclined to do so. In areas where there is a risk of frost, it may be desirable to delay budding or germination of seeds with the aid of growth regulators, in order to avoid damage resulting from late frosts.

Finally, growth regulators can induce resistance of the plants to frost, drought or high salinity of the soil. This allows the cultivation of plants in regions which are normally unsuitable for this purpose.

### Plant Defense Modulators

The compound and the composition of the invention may also exhibit a potent strengthening effect in plants. Accordingly, they may be used for mobilizing the defences of the plant against attack by undesirable microorganisms.

Plant-strengthening (resistance-inducing) substances in the present context are substances capable of stimulating the defence system of plants in such a way that the treated plants, when subsequently inoculated with undesirable microorganisms, develop a high degree of resistance to these microorganisms.

Further, in context with the present invention plant physiology effects comprise the following:
Abiotic stress tolerance, comprising tolerance to high or low temperatures, drought tolerance and recovery after drought stress, water use efficiency (correlating to reduced water consumption), flood tolerance, ozone stress and UV tolerance, tolerance towards chemicals like heavy metals, salts, pesticides.

Biotic stress tolerance, comprising increased fungal resistance and increased resistance against nematodes, viruses and bacteria. In context with the present invention, biotic stress tolerance preferably comprises increased fungal resistance and increased resistance against nematodes and bacteria

Increased plant vigor, comprising plant health / plant quality and seed vigor, reduced stand failure, improved appearance, increased recovery after periods of stress, improved pigmentation (e.g. chlorophyll content, stay-green effects) and improved photosynthetic efficiency.

### Preparation Examples

The preparation and the use of the inventive active ingredients of the formula (I) is illustrated by the examples which follow. However, the invention is not limited to these examples.

### Synthesis of N'-[5-bromo-2-methyl-4-[2-(2,2,2-trifluoroethylsulfanyl)pyrimidin-4-yl]oxyphenyl]-N-ethyl-N-methyl-formamidine (Example I-005, general synthesis A)

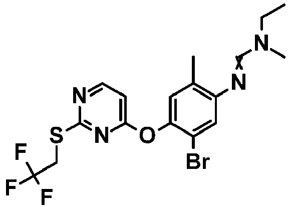

Step 1: To a solution of 4-amino-2-bromo-5-methyl-phenol (1 g, 4.94 mmol) in acetone (6 mL) was added 2,4-dichloropyrimidine (737 mg, 4.94 mmol) and NaOH (2.72 mL, 2 M in water). The reaction was stirred at RT for 16 h, acetone was removed in vacuo and additional water (30 mL) was added. The resulting mixture was extracted with EtOAc (3 x 30mL). The combined organic layers were washed with brine (1x40 mL), dried over anhydrous Na₂SO₄. After filtration, the filtrate was concentrated under reduced pressure to afford 5-bromo-4-(2-chloropyrimidin-4-yl)oxy-2-methyl-aniline (1.27 g, 75%) which was used without further purification.
Step 2: NaH (61 mg, 1.52 mmol; 60% in mineral oil) was suspended under argon in DMF (1 mL) and 2,2,2-trifluoroethanethiol (166 mg, 1.43 mmol) in DMF (1 mL) was added at RT. After 20 min, 5-bromo-4-(2-chloropyrimidin-4-yl)oxy-2-methyl-aniline (300 mg, 0.95 mmol) in DMF (5 mL) was added dropwise and the mixture was heated to 80°C for 3 h. The reaction was cooled to room temperature, diluted with water (15 mL), and extracted with ethyl acetate (3 x 20 mL). The combined organic phases were washed with water (20 ml) and brine (20 mL), dried over Na₂SO₄ and concentrated in vacuo. Purification of the crude residue via column chromatography (CombiFlash® Rf+, SiO₂, c-hexane/ethyl acetate gradient 0-100%) yielded 5-bromo-2-methyl-4-[2-(2,2,2-trifluoroethylsulfanyl)pyrimidin-4-yl]oxy-aniline (170 mg, 44%).
Step 3: 5-bromo-2-methyl-4-[2-(2,2,2-trifluoroethylsulfanyl)pyrimidin-4-yl]oxy-aniline (170 mg, 0.43 mmol) and N-(dimethoxymethyl)-N-methyl-ethanamine (86 mg, 0.64 mmol) were dissolved in toluene (10 mL) and stirred for 16 h at 80 °C. The solution was cooled to RT and evaporated to dryness. The crude residue was further purified via column chromatography (CombiFlash® Rf+, SiO₂, c-hexane/ethyl acetate gradient 0-100%) to give N'-[5-bromo-2-methyl-4-[2-(2,2,2-trifluoroethylsulfanyl)pyrimidin-4-yl]oxy-phenyl]-N-ethyl-N-methyl-formamidine (175 mg, 86%).

### Synthesis of N'-[4-(2-tert-butylpyrimidin-4-yl)oxy-5-cyano-2-methyl-phenyl]-N-ethyl-N-methyl-formamidine (Example 1-044, general synthesis B)

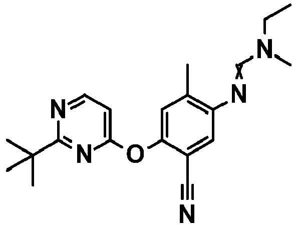

Step 1: 2-tert-butyl-1H-pyrimidin-6-one (380 mg, 2.49 mmol) and CS₂CO₃ (870 mg, 2.66 mmol) were suspended in DMF (8 mL) and stirred at RT for 20 min. The solution was cooled to 0°C and 2-fluoro-4-methyl-5-nitro-benzonitrile (200 mg, 1.66 mmol) in DMF (4 mL) was added dropwise. After 16 h at RT, the mixture was diluted with water (20 mL), and extracted with ethyl acetate (3 x 25 mL). The combined organic phases were washed with water (20 ml) and brine (20 mL), dried over Na₂SO₄ and concentrated in vacuo. Purification of the crude residue via column chromatography (CombiFlash® Rf+, SiO₂, c-hexane/ethyl acetate gradient 0-100%) yielded 2-(2-tert-butylpyrimidin-4-yl)oxy-4-methyl-5-nitro-benzonitrile (292 mg, 53%).
Step 2: A suspension of 2-(2-tert-butylpyrimidin-4-yl)oxy-4-methyl-5-nitro-benzonitrile (290 mg, 0.93 mmol) and SnCl₂-2H₂O (1.06 g, 4.67 mmol) in EtOH (10 mL) was stirred at 90°C for 1 h. The mixture was cooled to RT, solid ice was added and the pH-value was adjusted with solid Na₂CO3 to pH = 10. The mixture was filtered over Celite® and washed with ethyl acetate. The aqueous phases were further extracted with ethyl acetate (3 x 25 mL), the combined organic phases dried over Na₂SO₄ and concentrated in vacuo. Purification of the crude residue via column chromatography (CombiFlash® Rf+, SiO₂, c-hexane/ethyl acetate gradient 0-100%) yielded 5-amino-2-(2-tert-butylpyrimidin-4-yl)oxy-4-methyl-benzonitrile (56 mg, 20%).
Step 3: 5-amino-2-(2-tert-butylpyrimidin-4-yl)oxy-4-methyl-benzonitrile (56 mg, 0.19 mmol) and N-(dimethoxymethyl)-N-methyl-ethanamine (40 mg, 0.29 mmol) were dissolved in toluene (5 mL) and stirred for 16 h at 80 °C. The solution was cooled to RT and evaporated to dryness. The crude residue was further purified via column chromatography (CombiFlash® Rf+, SiO₂, c-hexane/ethyl acetate gradient 0-100%) to give N'-[4-(2-tert-butylpyrimidin-4-yl)oxy-5-cyano-2-methyl-phenyl]-N-ethyl-N-methyl-formamidine (17 mg, 23%).

### Synthesis of N'-[4-[2-(cyclopentoxy)pyrimidin-4-yl]oxy-2,5-dimethyl-phenyl]-N-ethyl-N-methyl-formamidine (Example 1-041, general synthesis C)

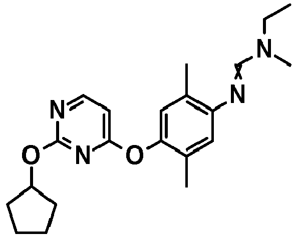

Step 1: 2,5-dimethyl-4-(2-methylsulfanylpyrimidin-4-yl)oxy-aniline (2.00 g, 7.65 mmol, Intermediate prepared according to route A1) was dissolved in CH₂Cl₂ (40 mL), di-*tert*-butyl dicarbonate (2.00 g, 9.18 mmol) was slowly added and the reaction was stirred at RT for 16 h. Another batch of di-*tert*-butyl dicarbonate (1.00 g, 4.60 mmol) was slowly added and the reaction was stirred for 3 h at 40 °C. Water (50 mL) was added and the aqueous phase was further extracted with CH₂Cl₂ (2 x 30 mL). The combined organic phases were dried over Na₂SO₄, concentrated in vacuo and purified via column chromatography (CombiFlash® Rf+, SiO₂, c-hexane/ethyl acetate gradient 0-100%) to give *tert*-butyl N-[2,5-dimethyl-4-(2-methylsulfanylpyrimidin-4-yl)oxy-phenyl]carbamate (2.70 g, 96%).
Step 2: Solid *m*CPBA (410 mg, 1.82 mmol, 77% purity) was added to a solution of *tert*-butyl N-[2,5-dimethyl-4-(2-methylsulfanylpyrimidin-4-yl)oxy-phenyl]carbamate (300 mg, 0.83 mmol) in CH₂Cl₂ (10 mL) at 0 °C. After 16 h at RT, the reaction was quenched with a Na₂S₂O₃/NaHCO₃-solution (1:1, 20 mL, sat., aq.) and the aqueous phase was further extracted with CH₂Cl₂ (2 x 20 mL). The combined organic phases were dried over Na₂SO₄ and concentrated in vacuo to afford tert-butyl N-[2,5-dimethyl-4-(2-methylsulfonylpyrimidin-4-yl)oxyphenyl]carbamate (286 mg, 63%) which was used directly in the next step.
Step 3: c-Pentanol (75 mg, 0.87 mmol) in DMF (5 mL) was treated with NaH (35 mg, 0.87 mmol, 60% purity) at RT and the reaction was stirred for 20 min. Then, *tert*-butyl N-[2,5-dimethyl-4-(2-methylsulfonylpyrimidin-4-yl)oxy-phenyl]carbamate (286 mg, 0.72 mmol) in DMF (5 mL) was added dropwise. The reaction mixture was stirred overnight at room temperature, then diluted with water (20 mL), extracted with ethyl acetate (2 x 20 mL) and the combined organic phases dried over Na₂SO₄. After filtration and evaporation, the crude residue was purified via column chromatography (CombiFlash® Rf+, SiO₂, *c*-hexane/ethyl acetate gradient 0-100%) to give *tert-*butyl N-[4-[2-(cyclopentoxy)pyrimidin-4-yl]oxy-2,5-dimethyl-phenyl]carbamate (200 mg, 67%).
Step 4: A solution of *tert*-butyl N-[4-[2-(cyclopentoxy)pyrimidin-4-yl]oxy-2,5-dimethylphenyl]carbamate (200 mg, 0.50 mmol) in 1,4-dioxane (2 mL) was treated at 0°C with HCl in 1,4-dioxane (2 mL; 4 M) and stirred overnight at RT. The solvent was evaporated to give 4-[2-(cyclopentoxy)pyrimidin-4-yl]oxy-2,5-dimethyl-aniline hydrochloride (170 mg, 99%) which was used without further purification in the next step.
Step 5: 4-[2-(cyclopentoxy)pyrimidin-4-yl]oxy-2,5-dimethyl-aniline hydrochloride (170 mg, 0.50 mmol), DIPEA (72 mg, 0.55 mmol) and N-(dimethoxymethyl)-N-methyl-ethanamine (101 mg, 0.76 mmol) were dissolved in toluene (5 mL) and stirred for 16 h at 80 °C. The solution was cooled to RT and evaporated to dryness. The crude residue was further purified via column chromatography (CombiFlash® Rf+, SiO₂, c-hexane/ethyl acetate gradient 0-100%) to give N'-[4-[2-(cyclopentoxy)pyrimidin-4-yl]oxy-2,5-dimethyl-phenyl]-N-ethyl-N-methyl-formamidine (132 mg, 63%).

### Examples

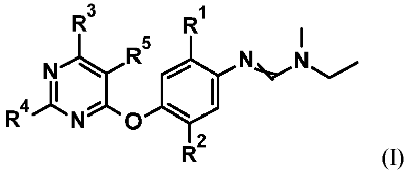

**Table 2: Experimental data for selected compounds according to formula (I):**

| **Ex N°** | **R1** | **R2** | **R₃** | **R4** | **R5** | **LogP** |
|---|---|---|---|---|---|---|
| I.001 | CH₃ | Br | H | tert-butylthio | H | 1.72^{[a]};4.95^{[b]} |
| I.002 | CH₃ | CH₃ | H | 3-methylbutylthio | H | 1.90^{[a]};5.66^{[b]} |
| I.003 | CH₃ | CH₃ | H | 3,3-dimethylbutyl | H | 1.63^{[a]};4.89^{[b]} |
| I.004 | CH₃ | CH₃ | H | butan-2-ylthio | H | 1.87^{[a]} |
| I.005 | CH₃ | Br | H | 2,2,2-trifluoroethylthio | H | 1.58^{[a]};4.66^{[b]} |
| I.006 | Cl | CH₃ | H | 2,2,2-trifluoroethylthio | H | 1.53^{[a]};4.19^{[b]} |
| I.007 | CH₃ | Br | H | 2-methylpropylthio | H | 1.81^{[a]};5.25^{[b]} |
| I.008 | CH₃ | CH₃ | H | (2-fluorophenyl)thio | H | 1.60^{[a]} |
| I.009 | CH₃ | CH₃ | H | (3-fluorophenyl)thio | H | 1.66^{[a]} |
| I.010 | CH₃ | CH₃ | H | (4-fluorophenyl)thio | H | 1.60^{[a]} |
| I.011 | CH₃ | CH₃ | H | cyclohexylthio | H | 1.86^{[a]} |
| I.012 | CH₃ | CH₃ | H | cyclopentylthio | H | 1.75^{[a]} |
| I.013 | CH₃ | CH₃ | H | 3,3,3-trifluoropropylthio | H | 1.58^{[a]} |
| I.014 | CH₃ | Br | H | isopropylthio | H | 1.60^{[a]};4.78^{[b]} |
| I.015 | CH₃ | Br | H | propylthio | H | 1.66^{[a]};4.86^{[b]} |
| I.016 | CH₃ | Br | H | ethylsulfanyl | H | 1.46^{[a]};4.44^{[b]} |
| I.017 | CH₃ | Br | H | CF₃ | H | 1.32^{[a]};4.08^{[b]} |
| I.018 | CH₃ | Br | tert-butylthio | CH₃ | H | 2.11^{[a]};5.99^{[b]} |
| I.019 | CH₃ | CH₃ | CH₃ | ethylsulfanyl | H | 1.52^{[a]} |
| I.020 | CH₃ | CH₃ | CH₃ | propylthio | H | 1.69^{[a]} |
| I.021 | CH₃ | CH₃ | CH₃ | 2-methylpropylthio | H | 1.84^{[a]} |
| I.022 | CH₃ | CH₃ | CH₃ | isopropylthio | H | 1.69^{[a]} |
| I.023 | CH₃ | CH₃ | tert-butylthio | CH₃ | H | 1.99^{[a]} |
| I.024 | CH₃ | CH₃ | butan-2-ylthio | CH₃ | H | 2.02^{[a]};5.98^{[b]} |
| I.025 | CH₃ | CHF₂ | H | 2-methylpropylthio | H | 1.69^{[a]} |
| I.026 | CH₃ | CH₃ | H | 2-methylpropylthio | H | 1.61^{[a]};5.06^{[b]} |
| I.027 | CH₃ | CH₃ | H | ethylsulfanyl | H | 1.27^{[a]};4.12^{[b]} |
| I.028 | CH₃ | CH₃ | H | isopropylthio | H | 1.45^{[a]};4.54^{[b]} |
| I.029 | CH₃ | CH₃ | H | propylthio | H | 1.46^{[a]};4.61^{[b]} |
| I.030 | CH₃ | CH₃ | H | CF₃ | H | 1.24^{[a]}_{;}3.82^{[b]} |
| I.031 | CH₃ | CH₃ | H | 2,2,2-trifluoroethylthio | H | 1.52^{[a]};4.37^{[b]} |
| I.032 | CH₃ | CH₃ | H | tert-butyl | H | 1.46^{[a]};4.56^{[b]} |
| I.033 | CH₃ | CH₃ | CH₃ | methyl sulfanyl | H | 1.34^{[a]};4.16^{[b]} |
| I.034 | CH₃ | CH₃ | H | (E)-3,3-dimethylbut-1-en-1-yl | H | 1.66^{[a]};4.94^{[b]} |
| I.035 | CH₃ | CF₃ | H | 2-methylpropylthio | H | 1.93^{[a]} |
| I.036 | CH₃ | CHF₂ | H | propylthio | H | 1.55^{[a]} |
| I.037 | CH₃ | Br | H | phenylsulfanyl | H | 1.69^{[a]};4.80^{[b]} |
| I.038 | CH₃ | Br | H | tert-butyl | H | 1.58^{[a]}_{;}4.93^{[b]} |
| I.039 | CH₃ | CH₃ | H | phenylsulfanyl | H | 1.52^{[a]};4.55^{[b]} |
| I.040 | CH₃ | CH₃ | H | cyclopropylmethylthio | H | 1.55^{[a]} |
| I.041 | CH₃ | CH₃ | H | cyclopentyloxy | H | 1.60^{[a]};4.67^{[b]} |
| I.042 | CH₃ | CH₃ | H | benzylsulfanyl | H | 1.63^{[a]}_{;}4.82^{[b]} |
| I.043 | CH₃ | CH₃ | H | 2-trimethylsilylethylthio | H | 2.17^{[a]} |
| I.044 | CH₃ | CN | H | tert-butyl | H | 1.41^{[a]};4.29^{[b]} |
| I.045 | Cl | CH₃ | H | tert-butylthio | H | 1.63^{[a]}_{;}4.50^{[b]} |
| I.046 | Cl | CH₃ | H | isopropylthio | H | 1.52^{[a]}_{;}4.35^{[b]} |
| I.047 | CH₃ | CH₃ | H | pyridin-4-ylthio | H | 1.13^{[a]} |
| I.048 | Cl | CH₃ | H | 2-methylpropylthio | H | 1.72^{[a]};4.77^{[b]} |
| I.049 | CH₃ | CN | H | 2-methylpropylthio | H | 1.60^{[a]};4.50^{[b]} |
| I.050 | CH₃ | CN | H | propylthio | H | 1.44^{[a]};4.16^{[b]} |
| I.051 | CH₃ | CHF₂ | H | 2-methylpropylthio | CH₃ | 1.90^{[a]} |

**Table 3: NMR-peak list for selected compounds according to formula (I):**

| |
|---|
| I.001: ¹H-NMR(400.2 MHz, d₆-DMSO): |
| δ= 8.4639 (1.9); 8.4496 (2.0); 7.1416 (0.4); 7.1077 (1.8); 7.1062 (1.8); 6.8503 (1.8); 6.8360 (1.7); 3.3692 (0.3); 3.3477 (0.3); 3.3247 (47.0); 3.0030 (0.4); 2.9311 (1.0); 2.6750 (0.4); 2.6704 (0.6); 2.6657 (0.4); 2.5239 (1.7); 2.5192 (2.7); 2.5105 (36.8); 2.5060 (75.1); 2.5014 (98.5); 2.4968 (69.6); 2.4922 (32.3); 2.3328 (0.4); 2.3283 (0.6); 2.3236 (0.4); 2.1413 (3.2); 1.4923 (0.9); 1.3975 (1.2); 1.2898 (1.3); 1.2750 (16.0); 1.1565 (0.5); 1.1411 (1.0); 1.1238 (0.6); 0.0079 (0.4); -0.0002 (12.9); -0.0085 (0.4) |
| I.002: ¹H-NMR(400.2 MHz, d₆-DMSO): |
| δ= 8.4368 (3.3); 8.4226 (3.4); 6.8348 (3.1); 6.6983 (1.6); 6.6738 (3.3); 6.6595 (3.2); 3.3509 (0.4); 3.3244 (13.4); 2.9362 (1.4); 2.8815 (1.9); 2.8667 (1.3); 2.8622 (1.9); 2.8575 (1.2); 2.8432 (1.9); 2.5252 (0.5); 2.5205 (0.8); 2.5118 (11.6); 2.5073 (23.7); 2.5027 (31.0); 2.4981 (21.8); 2.4935 (10.2); 2.1330 (8.3); 1.9764 (8.8); 1.4747 (0.6); 1.4579 (0.8); 1.4413 (0.7); 1.4252 (0.5); 1.3976 (1.2); 1.3835 (1.2); 1.3787 (1.6); 1.3736 (1.0); 1.3669 (0.7); 1.3596 (1.6); 1.3427 (0.7); 1.1492 (2.6); 1.1315 (5.5); 1.1138 (2.5); 0.8250 (0.5); 0.8086 (0.6); 0.7981 (16.0); 0.7819 (15.2); 0.0079 (1.1); -0.0002 (37.0); -0.0086 (1.2) |
| I.003: ¹H-NMR(400.2 MHz, d₆-DMSO): |
| δ= 8.5088 (1.1); 8.4944 (1.1); 6.8305 (1.3); 6.7179 (0.6); 6.6183 (0.9); 6.6039 (0.9); 3.3246 (4.6); 2.9336 (0.5); 2.6873 (0.7); 2.6736 (0.5); 2.6666 (0.7); 2.6588 (0.5); 2.6456 (0.7); 2.5114 (4.0); 2.5072 (8.2); 2.5027 (10.8); 2.4982 (8.0); 2.4938 (4.0); 2.1367 (3.3); 1.9747 (3.6); 1.6013 (0.8); 1.5880 (0.5); 1.5800 (0.7); 1.5731 (0.5); 1.5595 (0.8); 1.1514 (1.0); 1.1337 (2.1); 1.1160 (1.0); 0.8820 (16.0); 0.0079 (0.3); -0.0002 (11.4); -0.0085 (0.5) |
| I.004: ¹H-NMR(400.2 MHz, d₆-DMSO): |
| δ= 8.4331 (6.7); 8.4188 (6.9); 8.3983 (0.4); 8.3840 (0.4); 7.6448 (0.4); 6.8379 (5.7); 6.7022 (2.4); 6.6750 (0.4); 6.6449 (6.5); 6.6306 (6.4); 6.5710 (0.4); 6.5568 (0.4); 4.7642 (0.4); 3.4766 (1.1); 3.4596 (2.1); 3.4429 (2.2); 3.4259 (1.3); 3.4089 (0.6); 3.3463 (0.8); 3.3239 (34.9); 3.3074 (0.4); 2.9328 (2.4); 2.8914 (0.4); 2.6760 (0.3); 2.6714 (0.5); 2.6668 (0.4); 2.5250 (1.4); 2.5203 (2.0); 2.5115 (28.9); 2.5070 (59.6); 2.5024 (77.9); 2.4978 (54.5); 2.4932 (25.4); 2.3338 (0.4); 2.3292 (0.5); 2.3245 (0.4); 2.1332 (14.7); 2.0100 (1.0); 1.9805 (16.0); 1.9025 (1.0); 1.6267 (0.5); 1.6109 (0.9); 1.6082 (0.7); 1.5923 (1.8); 1.5763 (1.4); 1.5739 (1.5); 1.5580 (1.4); 1.5404 (1.5); 1.5225 (2.0); 1.5047 (1.7); 1.4880 (1.1); 1.4702 (0.7); 1.3976 (0.3); 1.2395 (1.3); 1.2311 (13.8); 1.2224 (1.5); 1.2138 (13.4); 1.1487 (5.0); 1.1310 (10.9); 1.1133 (4.9); 0.8712 (0.4); 0.8531 (6.9); 0.8348 (13.5); 0.8163 (5.5); 0.0080 (1.8); -0.0002 (57.6); -0.0086 (1.8) |
| I.005: ¹H-NMR(400.2 MHz, d₆-DMSO): |
| δ= 8.5797 (8.9); 8.5654 (9.4); 8.3159 (0.4); 7.7773 (1.5); 7.6655 (0.6); 7.1576 (1.8); 7.1233 (0.8); 7.1055 (8.0); 7.1039 (7.9); 6.9218 (8.4); 6.9075 (8.3); 5.7561 (2.5); 4.0418 (1.4); 4.0380 (1.1); 4.0160 (4.6); 4.0023 (0.6); 3.9900 (4.8); 3.9641 (1.6); 3.5665 (0.4); 3.4595 (0.6); 3.4436 (0.7); 3.3854 (0.8); 3.3691 (1.4); 3.3519 (1.5); 3.3249 (113.9); 3.0060 (1.9); 2.9627 (0.7); 2.9366 (5.0); 2.6802 (0.4); 2.6756 (0.9); 2.6710 (1.2); 2.6664 (0.9); 2.6618 (0.4); 2.5245 (3.8); 2.5198 (5.6); 2.5111 (70.1); 2.5066 (142.9); 2.5020 (187.9); 2.4973 (134.4); 2.4928 (63.2); 2.3381 (0.4); 2.3334 (0.8); 2.3288 (1.2); 2.3242 (0.8); 2.3198 (0.4); 2.1547 (16.0); 1.9888 (2.5); 1.3978 (5.2); 1.1928 (0.8); 1.1750 (1.6); 1.1609 (2.3); 1.1573 (2.7); 1.1438 (4.8); 1.1269 (2.8); -0.0002 (7.0) |
| I.006: ¹H-NMR(400.2 MHz, d₆-DMSO): |
| δ= 8.5662 (5.2); 8.5518 (5.4); 7.7516 (1.4); 7.6369 (0.6); 7.2050 (7.3); 6.9647 (1.6); 6.9382 (0.6); 6.8985 (4.7); 6.8842 (4.6); 5.7563 (6.4); 4.0495 (1.0); 4.0382 (0.5); 4.0236 (3.2); 3.9977 (3.3); 3.9718 (1.1); 3.7301 (0.4); 3.4648 (0.5); 3.4482 (0.5); 3.3853 (0.5); 3.3680 (1.2); 3.3503 (1.2); 3.3260 (26.7); 3.0095 (1.8); 2.9453 (4.7); 2.6717 (0.3); 2.5251 (1.2); 2.5203 (1.8); 2.5117 (21.0); 2.5072 (41.8); 2.5027 (54.8); 2.4980 (39.8); 2.4935 (19.2); 2.3295 (0.3); 1.9939 (16.0); 1.3977 (3.4); 1.1932 (0.4); 1.1753 (1.3); 1.1693 (1.9); 1.1519 (3.9); 1.1347 (2.1); -0.0002 (1.9) |
| I.007: ¹H-NMR(400.2 MHz, d₆-DMSO): |
| δ= 8.4746 (4.3); 8.4604 (4.4); 8.4406 (0.5); 8.4264 (0.5); 8.3158 (0.6); 7.7600 (0.8); 7.1466 (0.9); 7.1142 (0.4); 7.0920 (4.0); 7.0905 (4.0); 6.9127 (0.4); 6.8902 (0.7); 6.8144 (4.2); 6.8002 (4.2); 6.7499 (0.5); 6.7357 (0.5); 5.7556 (2.5); 5.1361 (0.4); 3.5664 (0.4); 3.4363 (0.4); 3.3648 (0.8); 3.3458 (0.9); 3.3232 (120.4); 3.0000 (0.9); 2.9307 (2.4); 2.7413 (0.7); 2.7316 (4.7); 2.7244 (1.0); 2.7144 (4.8); 2.6798 (0.5); 2.6754 (1.0); 2.6708 (1.3); 2.6662 (1.0); 2.6615 (0.4); 2.5243 (3.7); 2.5195 (5.8); 2.5109 (77.5); 2.5064 (156.9); 2.5018 (205.6); 2.4972 (147.0); 2.4927 (69.4); 2.3378 (0.4); 2.3332 (0.9); 2.3286 (1.3); 2.3241 (0.9); 2.3194 (0.4); 2.1562 (8.9); 2.0253 (1.4); 1.9886 (0.5); 1.7042 (0.4); 1.6872 (0.9); 1.6705 (1.1); 1.6537 (0.9); 1.6371 (0.5); 1.3980 (0.5); 1.1525 (1.3); 1.1357 (2.7); 1.1192 (1.5); 0.8211 (1.9); 0.8042 (2.9); 0.7977 (16.0); 0.7811 (15.3); 0.1459 (1.4); 0.0172 (0.4); 0.0164 (0.4); 0.0080 (12.3); -0.0001 (333.7); - 0.0085 (11.6); -0.0161 (0.8); -0.0176 (0.7); -0.0190 (0.6); -0.0205 (0.5); -0.0242 (0.4); -0.0249 (0.3); -0.1496 (1.4) |
| I.008: ¹H-NMR(400.2 MHz, d₆-DMSO): |
| δ= 8.4076 (4.3); 8.3933 (4.4); 7.6673 (0.4); 7.5873 (1.0); 7.5833 (1.1); 7.5681 (1.7); 7.5649 (1.9); 7.5493 (0.9); 7.5457 (1.0); 7.4849 (0.4); 7.4807 (0.5); 7.4719 (0.5); 7.4668 (1.0); 7.4521 (1.0); 7.4466 (1.1); 7.4417 (0.6); 7.4326 (0.6); 7.4285 (0.5); 7.2361 (1.2); 7.2142 (3.9); 7.1946 (3.6); 7.1769 (1.1); 7.1742 (1.0); 6.7327 (4.8); 6.6888 (4.3); 6.6745 (4.3); 6.6328 (2.2); 3.3715 (0.7); 3.3495 (0.6); 3.3261 (37.2); 2.9491 (1.9); 2.6712 (0.3); 2.5246 (0.9); 2.5110 (20.7); 2.5067 (41.7); 2.5023 (54.9); 2.4978 (40.3); 2.4934 (19.9); 2.3291 (0.3); 2.1013 (12.6); 1.9889 (0.6); 1.9146 (13.1); 1.3975 (16.0); 1.1677 (3.8); 1.1500 (8.0); 1.1323 (3.7); 0.1458 (0.3); 0.0077 (2.8); -0.0002 (71.2); -0.0085 (2.7); - 0.1498 (0.3) |
| I.009: ¹H-NMR(400.2 MHz, d₆-DMSO): |
| δ= 8.4362 (6.0); 8.4218 (6.2); 7.6490 (0.4); 7.4183 (0.7); 7.4040 (1.5); 7.3985 (3.3); 7.3949 (1.6); 7.3832 (2.4); 7.3784 (3.0); 7.3716 (1.4); 7.3640 (1.7); 7.3488 (2.1); 7.3454 (3.4); 7.3422 (2.1); 7.3295 (1.2); 7.3258 (1.6); 7.3228 (0.9); 7.2345 (0.8); 7.2317 (0.9); 7.2280 (0.8); 7.2253 (0.7); 7.2120 (1.4); 7.2088 (1.2); 7.2055 (1.2); 7.1923 (0.7); 7.1888 (0.7); 7.1857 (0.6); 7.1826 (0.6); 6.7605 (5.2); 6.7088 (6.0); 6.6945 (6.0); 6.6445 (2.4); 3.3720 (0.7); 3.3251 (33.3); 2.9488 (2.0); 2.6712 (0.4); 2.5248 (1.1); 2.5200 (1.8); 2.5114 (24.0); 2.5069 (49.5); 2.5023 (65.3); 2.4977 (46.5); 2.4931 (21.9); 2.3290 (0.4); 2.1072 (13.4); 1.9889 (0.9); 1.9294 (14.2); 1.3976 (16.0); 1.1752 (0.7); 1.1663 (3.9); 1.1573 (1.0); 1.1486 (8.4); 1.1308 (3.8); 0.1459 (0.4); 0.0080 (3.6); -0.0002 (105.1); -0.0086 (3.5); -0.1496 (0.4) |
| I.010: ¹H-NMR(400.2 MHz, d₆-DMSO): |
| δ= 8.4184 (6.7); 8.4042 (7.0); 7.6579 (0.5); 7.5241 (0.4); 7.5161 (3.5); 7.5107 (1.6); 7.5025 (3.9); 7.4939 (4.2); 7.4858 (1.6); 7.4803 (3.8); 7.4723 (0.4); 7.1724 (0.4); 7.1647 (3.9); 7.1592 (1.3); 7.1479 (1.3); 7.1424 (7.3); 7.1369 (1.4); 7.1255 (1.1); 7.1201 (3.5); 7.1123 (0.3); 6.7338 (5.8); 6.7055 (6.9); 6.6912 (6.8); 6.6329 (3.1); 3.3709 (0.8); 3.3242 (27.5); 2.9491 (2.3); 2.6756 (0.3); 2.6712 (0.4); 2.5247 (1.3); 2.5200 (1.8); 2.5112 (25.2); 2.5068 (52.7); 2.5022 (70.4); 2.4976 (50.6); 2.4930 (24.1); 2.3290 (0.4); 2.1070 (15.1); 1.9891 (0.4); 1.9070 (16.0); 1.3975 (12.0); 1.1670 (4.3); 1.1493 (9.2); 1.1315 (4.1); 0.1459 (0.4); 0.0080 (3.6); -0.0002 (111.6); -0.0086 (4.0); -0.1497 (0.4) |
| I.011: ¹H-NMR(400.2 MHz, d₆-DMSO): |
| δ= 8.4296 (3.1); 8.4153 (3.3); 6.8291 (2.8); 6.7009 (1.6); 6.6872 (3.0); 6.6729 (2.9); 3.3635 (0.5); 3.3548 (0.6); 3.3451 (0.6); 3.3362 (0.7); 3.3235 (9.7); 3.3009 (0.4); 2.9351 (1.6); 2.5246 (0.6); 2.5197 (0.9); 2.5113 (12.5); 2.5068 (24.9); 2.5023 (32.3); 2.4976 (22.8); 2.4931 (10.5); 2.1354 (7.4); 1.9773 (7.9); 1.8790 (0.7); 1.8734 (0.7); 1.8470 (0.8); 1.6037 (0.8); 1.5902 (0.7); 1.5793 (0.6); 1.4772 (0.5); 1.3977 (16.0); 1.3192 (0.7); 1.2911 (0.8); 1.2631 (0.4); 1.1646 (1.1); 1.1442 (3.9); 1.1266 (5.8); 1.1088 (2.7); 0.0080 (1.8); -0.0002 (47.3); -0.0086 (1.5) |
| I.012: ¹H-NMR(400.2 MHz, d₆-DMSO): |
| δ= 8.4308 (5.4); 8.4165 (5.7); 7.6327 (0.4); 6.8400 (5.4); 6.6998 (2.6); 6.6665 (5.1); 6.6523 (5.0); 3.6205 (0.4); 3.6021 (1.3); 3.5838 (1.8); 3.5729 (0.4); 3.5657 (1.1); 3.3258 (58.6); 2.9330 (2.5); 2.6712 (0.4); 2.5247 (1.2); 2.5199 (1.9); 2.5113 (26.3); 2.5069 (53.2); 2.5023 (69.7); 2.4977 (50.0); 2.4933 (23.8); 2.3335 (0.3); 2.3292 (0.4); 2.1324 (14.1); 2.0095 (0.4); 1.9799 (16.0); 1.9654 (1.7); 1.9536 (1.2); 1.9473 (1.4); 1.9351 (1.4); 1.9175 (0.6); 1.9023 (0.4); 1.6357 (0.8); 1.6310 (0.9); 1.6268 (1.1); 1.6194 (1.6); 1.6112 (1.9); 1.5959 (1.5); 1.5893 (0.8); 1.5824 (0.4); 1.5054 (0.4); 1.5005 (0.4); 1.4966 (0.4); 1.4831 (1.2); 1.4710 (1.4); 1.4648 (1.7); 1.4539 (1.9); 1.4402 (2.0); 1.4270 (1.8); 1.4134 (1.5); 1.3977 (13.8); 1.3768 (1.0); 1.3613 (0.4); 1.1467 (4.6); 1.1290 (10.0); 1.1112 (4.5); 0.1458 (0.4); 0.0080 (3.6); -0.0002 (101.4); -0.0085 (3.7); -0.1496 (0.4) |
| I.013: ¹H-NMR(400.2 MHz, d₆-DMSO): |
| δ= 8.4858 (6.5); 8.4715 (6.8); 7.6219 (0.4); 6.8431 (5.8); 6.7277 (6.0); 6.7134 (6.0); 6.6952 (2.9); 3.3260 (33.9); 3.0917 (2.8); 3.0729 (3.2); 3.0680 (2.0); 3.0535 (3.1); 2.9336 (2.8); 2.6717 (0.3); 2.5719 (0.4); 2.5518 (0.6); 2.5441 (1.4); 2.5296 (1.2); 2.5250 (2.2); 2.5204 (2.4); 2.5118 (18.7); 2.5073 (38.2); 2.5027 (50.1); 2.4981 (35.9); 2.4935 (17.1); 2.4780 (1.3); 2.4689 (0.5); 2.4500 (0.4); 2.1270 (15.0); 1.9790 (16.0); 1.3978 (8.2); 1.1477 (4.9); 1.1300 (10.7); 1.1122 (4.8); 0.1459 (0.3); 0.0080 (3.0); -0.0002 (84.6); -0.0086 (2.8) |
| I.014: ¹H-NMR(400.2 MHz, d₆-DMSO): |
| δ= 8.4889 (3.9); 8.4746 (4.1); 7.7795 (0.7); 7.1454 (0.8); 7.1044 (3.8); 7.1029 (3.7); 6.7784 (3.6); 6.7641 (3.6); 5.7586 (16.0); 4.0376 (0.5); 4.0199 (0.5); 3.5591 (0.3); 3.5418 (0.9); 3.5248 (1.2); 3.5078 (0.9); 3.4907 (0.4); 3.3857 (0.3); 3.3707 (0.6); 3.3531 (0.6); 3.3243 (20.1); 3.0055 (0.9); 2.9337 (2.3); 2.6706 (0.4); 2.5243 (1.0); 2.5195 (1.7); 2.5108 (22.9); 2.5063 (46.9); 2.5017 (61.8); 2.4971 (43.9); 2.4926 (20.6); 2.3285 (0.4); 2.1523 (6.9); 1.9891 (2.3); 1.3974 (7.3); 1.2323 (15.0); 1.2152 (14.7); 1.1923 (0.7); 1.1745 (1.3); 1.1567 (1.6); 1.1429 (2.2); 1.1262 (1.3); -0.0002 (9.6) |
| I.015: ¹H-NMR(400.2 MHz, d₆-DMSO): |
| δ= 8.4830 (8.3); 8.4688 (8.6); 7.7757 (1.3); 7.6659 (0.5); 7.1485 (1.5); 7.1112 (0.7); 7.0992 (7.2); 7.0976 (7.0); 6.8053 (8.0); 6.7911 (7.9); 3.4498 (0.6); 3.4367 (0.6); 3.3654 (1.4); 3.3483 (1.5); 3.3244 (182.0); 3.0020 (1.6); 2.9306 (4.2); 2.8067 (3.8); 2.7915 (3.6); 2.7884 (4.4); 2.7847 (3.4); 2.7698 (4.0); 2.6795 (0.8); 2.6750 (1.7); 2.6704 (2.4); 2.6658 (1.8); 2.6612 (0.8); 2.5937 (0.4); 2.5633 (0.4); 2.5240 (7.0); 2.5193 (10.5); 2.5106 (139.9); 2.5060 (287.1); 2.5014 (377.4); 2.4968 (266.8); 2.4922 (124.1); 2.3375 (0.7); 2.3328 (1.6); 2.3283 (2.3); 2.3236 (1.6); 2.3191 (0.7); 2.1557 (14.6); 1.5244 (0.5); 1.5059 (2.0); 1.4874 (3.5); 1.4689 (3.5); 1.4506 (2.1); 1.4325 (0.5); 1.1538 (2.1); 1.1370 (4.3); 1.1192 (2.4); 0.7858 (7.4); 0.7676 (16.0); 0.7492 (6.6); 0.0080 (1.5); -0.0002 (49.3); -0.0085 (1.5) |
| I.016: ¹H-NMR(400.2 MHz, d₆-DMSO): |
| δ= 8.4945 (2.9); 8.4803 (3.0); 7.7868 (0.6); 7.1457 (0.6); 7.1049 (3.0); 6.7795 (2.7); 6.7653 (2.7); 5.7585 (16.0); 4.0374 (0.7); 4.0197 (0.7); 3.3706 (0.5); 3.3528 (0.5); 3.3237 (24.8); 3.0054 (0.7); 2.9330 (1.8); 2.8978 (0.8); 2.8797 (2.7); 2.8616 (2.8); 2.8435 (0.9); 2.6748 (0.4); 2.6705 (0.5); 2.6659 (0.3); 2.5240 (1.2); 2.5192 (1.9); 2.5105 (28.8); 2.5061 (59.1); 2.5015 (77.6); 2.4969 (55.1); 2.4923 (25.8); 2.3329 (0.3); 2.3284 (0.4); 2.3237 (0.3); 2.1546 (5.7); 1.9890 (3.0); 1.3974 (7.7); 1.1922 (0.8); 1.1744 (1.7); 1.1633 (3.8); 1.1567 (1.7); 1.1453 (8.7); 1.1271 (4.1); 0.0080 (0.4); -0.0002 (11.6); -0.0086 (0.3) |
| I.017: ¹H-NMR(400.2 MHz, d₆-DMSO): |
| δ= 8.9223 (5.5); 8.9078 (5.6); 7.8277 (1.9); 7.7140 (0.8); 7.3733 (5.5); 7.3588 (5.4); 7.1851 (9.4); 7.1837 (9.1); 7.1565 (0.9); 5.7569 (5.9); 4.0559 (0.5); 4.0381 (1.7); 4.0203 (1.7); 4.0025 (0.6); 3.4678 (0.7); 3.4491 (0.8); 3.3960 (0.7); 3.3793 (1.6); 3.3615 (1.6); 3.3441 (0.8); 3.3259 (45.2); 3.0145 (2.5); 2.9406 (6.3); 2.6761 (0.5); 2.6716 (0.7); 2.6670 (0.5); 2.5252 (2.3); 2.5204 (3.3); 2.5117 (42.9); 2.5072 (88.1); 2.5026 (115.4); 2.4980 (80.8); 2.4934 (37.3); 2.3340 (0.5); 2.3294 (0.7); 2.3249 (0.6); 2.1678 (16.0); 1.9892 (7.6); 1.4878 (1.0); 1.3978 (2.0); 1.1931 (2.1); 1.1753 (4.7); 1.1667 (2.3); 1.1575 (4.0); 1.1495 (5.0); 1.1329 (3.4); 0.1459 (0.6); 0.0080 (4.7); -0.0002 (149.1); -0.0086 (4.5); - 0.1496 (0.6) |
| I.018: ¹H-NMR(400.2 MHz, d₆-DMSO): |
| δ= 7.7954 (0.3); 7.1239 (0.4); 7.0519 (1.6); 7.0504 (1.6); 6.5326 (2.0); 6.5317 (2.1); 5.7561 (2.6); 3.3260 (24.2); 3.0027 (0.4); 2.9302 (1.0); 2.5247 (0.5); 2.5199 (0.8); 2.5112 (11.0); 2.5067 (22.5); 2.5022 (29.5); 2.4975 (20.9); 2.4930 (9.8); 2.4153 (5.8); 2.1469 (3.3); 1.5734 (16.0); 1.4928 (0.4); 1.3976 (1.1); 1.2903 (0.4); 1.1574 (0.5); 1.1417 (1.0); 1.1253 (0.6); 0.0079 (0.7); -0.0002 (22.6); -0.0086 (0.7) |
| I.019: ¹H-NMR(400.2 MHz, d₆-DMSO): |
| δ= 7.6594 (0.3); 6.8204 (4.5); 6.6966 (2.0); 6.4580 (5.4); 6.4573 (5.5); 3.3480 (0.7); 3.3228 (47.8); 2.9321 (1.8); 2.9213 (2.6); 2.9031 (5.0); 2.8849 (4.9); 2.8668 (1.5); 2.5247 (0.9); 2.5200 (1.3); 2.5112 (16.0); 2.5067 (33.1); 2.5020 (45.7); 2.4974 (34.2); 2.4929 (16.4); 2.3333 (16.0); 2.3137 (0.4); 2.1322 (11.4); 1.9777 (12.2); 1.3977 (7.1); 1.1867 (5.5); 1.1686 (12.3); 1.1502 (8.4); 1.1318 (8.0); 1.1140 (3.6); 0.0080 (1.3); -0.0002 (38.3); -0.0085 (1.1) |
| I.020: ¹H-NMR(400.2 MHz, d₆-DMSO): |
| δ= 7.6366 (0.4); 6.8084 (4.6); 6.6939 (2.4); 6.4940 (5.5); 6.4932 (5.5); 3.3558 (0.6); 3.3466 (0.6); 3.3364 (0.6); 3.3141 (43.5); 2.9338 (2.3); 2.8378 (2.6); 2.8197 (3.2); 2.8167 (2.6); 2.8012 (2.7); 2.6705 (0.4); 2.5240 (0.8); 2.5193 (1.2); 2.5106 (18.6); 2.5060 (39.5); 2.5014 (55.5); 2.4968 (42.4); 2.4923 (21.1); 2.3316 (16.0); 2.3128 (0.4); 2.1327 (11.8); 1.9715 (12.5); 1.5407 (0.3); 1.5223 (1.4); 1.5039 (2.5); 1.4856 (2.6); 1.4674 (1.5); 1.4492 (0.4); 1.3981 (4.3); 1.1460 (4.0); 1.1283 (8.8); 1.1105 (3.9); 0.8254 (0.3); 0.8175 (4.9); 0.7993 (10.3); 0.7809 (4.5); 0.0080 (1.2); -0.0002 (40.9); -0.0085 (1.4) |
| I.021: ¹H-NMR(400.2 MHz, d₆-DMSO): |
| δ= 8.3149 (0.4); 6.8028 (4.0); 6.6934 (2.0); 6.5195 (4.7); 3.3216 (35.0); 3.2981 (0.4); 2.9320 (1.9); 2.7552 (4.7); 2.7382 (4.8); 2.6709 (0.4); 2.5245 (1.0); 2.5197 (1.4); 2.5111 (19.0); 2.5065 (39.8); 2.5019 (55.0); 2.4973 (41.5); 2.4927 (20.3); 2.3323 (13.9); 2.1322 (10.1); 1.9645 (10.7); 1.7070 (0.4); 1.6903 (0.9); 1.6735 (1.1); 1.6568 (0.9); 1.6401 (0.5); 1.3978 (6.2); 1.1437 (3.6); 1.1261 (7.8); 1.1083 (3.4); 0.8165 (16.0); 0.7998 (15.4); 0.0080 (1.5); -0.0002 (45.8); -0.0086 (1.4) |
| I.022: ¹H-NMR(400.2 MHz, d₆-DMSO): |
| δ= 6.8169 (3.8); 6.6927 (1.9); 6.4561 (4.6); 3.6154 (0.4); 3.5984 (1.1); 3.5813 (1.5); 3.5643 (1.1); 3.5471 (0.4); 3.3577 (0.5); 3.3477 (0.5); 3.3381 (0.6); 3.3144 (53.8); 3.2924 (0.3); 2.9360 (1.9); 2.5240 (0.7); 2.5194 (1.0); 2.5106 (12.8); 2.5061 (26.4); 2.5015 (36.6); 2.4969 (27.9); 2.4924 (14.0); 2.3286 (13.3); 2.1299 (9.7); 1.9798 (10.2); 1.3980 (9.9); 1.2579 (0.6); 1.2478 (16.0); 1.2308 (15.9); 1.1502 (3.2); 1.1325 (6.8); 1.1147 (3.1); 0.0081 (0.8); -0.0002 (25.7); - 0.0084 (0.9) |
| I.023: ¹H-NMR(400.2 MHz, d₆-DMSO): |
| δ= 6.7982 (1.7); 6.6938 (0.9); 6.3732 (2.1); 5.7509 (2.4); 3.3134 (23.1); 2.9337 (0.9); 2.5060 (15.0); 2.5016 (19.4); 2.4971 (14.4); 2.4236 (6.0); 2.1286 (4.4); 1.9880 (0.5); 1.9670 (4.6); 1.5781 (0.5); 1.5604 (16.0); 1.3980 (0.9); 1.1493 (1.3); 1.1317 (2.7); 1.1139 (1.3); -0.0002 (8.1); -0.0083 (0.4) |
| I.024: ¹H-NMR(400.2 MHz, d₆-DMSO): |
| δ= 8.3112 (0.4); 7.6628 (0.4); 6.8015 (4.5); 6.6926 (2.5); 6.4501 (5.6); 5.7510 (2.7); 3.8421 (0.8); 3.8253 (1.6); 3.8087 (1.6); 3.7920 (0.8); 3.3410 (0.8); 3.3074 (74.7); 2.9344 (2.4); 2.6738 (1.1); 2.6695 (1.5); 2.6649 (1.1); 2.5049 (180.8); 2.5005 (226.4); 2.4961 (165.2); 2.3897 (16.0); 2.3319 (1.1); 2.3273 (1.4); 2.3229 (1.0); 2.1301 (11.8); 1.9876 (0.6); 1.9693 (12.5); 1.6751 (0.5); 1.6575 (1.6); 1.6379 (2.4); 1.6198 (1.8); 1.6014 (0.6); 1.3982 (1.3); 1.3312 (8.7); 1.3142 (8.6); 1.1492 (3.6); 1.1316 (7.4); 1.1138 (3.4); 0.9655 (4.3); 0.9472 (8.8); 0.9287 (3.9); 0.1458 (0.4); -0.0002 (91.3); -0.0083 (4.6); -0.1498 (0.5) |
| I.025: ¹H-NMR(400.2 MHz, d₆-DMSO): |
| δ= 8.4675 (4.4); 8.4533 (4.6); 7.7408 (0.5); 7.0614 (3.0); 7.0332 (1.9); 6.8964 (2.4); 6.7990 (4.3); 6.7849 (4.3); 6.7596 (1.2); 3.3742 (0.6); 3.3577 (0.6); 3.3223 (19.7); 3.0039 (0.7); 2.9623 (0.6); 2.9415 (1.8); 2.7333 (4.7); 2.7161 (4.9); 2.5243 (0.6); 2.5196 (1.0); 2.5109 (16.6); 2.5064 (34.8); 2.5018 (46.4); 2.4972 (33.3); 2.4926 (15.9); 2.2134 (6.4); 1.6857 (0.4); 1.6690 (0.9); 1.6522 (1.1); 1.6354 (0.9); 1.6186 (0.5); 1.3974 (0.4); 1.1554 (2.6); 1.1377 (5.5); 1.1200 (2.6); 0.8062 (0.5); 0.7978 (0.4); 0.7855 (16.0); 0.7689 (15.4); -0.0002 (1.6) |
| I.026: ¹H-NMR(400.2 MHz, d₆-DMSO): |
| δ= 8.4314 (3.3); 8.4172 (3.3); 7.6354 (0.4); 6.8310 (4.6); 6.7052 (2.4); 6.6830 (3.5); 6.6687 (3.5); 3.3258 (27.2); 2.9327 (2.5); 2.7808 (4.9); 2.7637 (5.0); 2.5027 (37.5); 2.1361 (12.0); 2.0111 (0.4); 1.9719 (12.7); 1.8943 (0.4); 1.7238 (0.5); 1.7072 (1.0); 1.6904 (1.2); 1.6738 (1.0); 1.6574 (0.5); 1.1453 (3.6); 1.1277 (7.5); 1.1100 (3.5); 0.8498 (0.6); 0.8284 (16.0); 0.8118 (15.3); -0.0002 (5.8) |
| I.027: ¹H-NMR(400.2 MHz, d₆-DMSO): |
| δ= 8.4479 (6.3); 8.4337 (6.6); 7.6646 (0.4); 6.8458 (5.5); 6.7066 (2.2); 6.6328 (6.4); 6.6185 (6.4); 3.4126 (0.4); 3.3433 (0.8); 3.3269 (32.5); 2.9441 (3.5); 2.9260 (7.9); 2.9078 (6.7); 2.8897 (2.1); 2.5254 (0.5); 2.5206 (0.7); 2.5120 (12.2); 2.5074 (25.7); 2.5029 (34.4); 2.4982 (24.4); 2.4937 (11.4); 2.1352 (14.1); 1.9833 (15.4); 1.2345 (0.3); 1.1966 (7.2); 1.1785 (16.0); 1.1603 (7.0); 1.1507 (4.7); 1.1331 (9.8); 1.1153 (4.4); -0.0002 (3.1) |
| I.028: ¹H-NMR(400.2 MHz, d₆-DMSO): |
| δ= 8.4432 (3.9); 8.4289 (4.0); 6.8441 (3.5); 6.7042 (1.4); 6.6311 (3.9); 6.6168 (3.9); 3.6361 (0.4); 3.6191 (1.1); 3.6020 (1.5); 3.5849 (1.1); 3.5678 (0.4); 3.3489 (0.5); 3.3428 (0.5); 3.3267 (20.8); 2.9339 (1.4); 2.5255 (0.4); 2.5207 (0.5); 2.5121 (7.5); 2.5075 (15.7); 2.5030 (20.8); 2.4984 (14.6); 2.4938 (6.8); 2.1330 (8.8); 1.9849 (9.6); 1.2667 (0.8); 1.2581 (16.0); 1.2500 (1.2); 1.2410 (15.7); 1.1510 (2.9); 1.1333 (6.1); 1.1156 (2.8); -0.0002 (1.8) |
| I.029: ¹H-NMR(400.2 MHz, d₆-DMSO): |
| δ= 8.4379 (6.5); 8.4236 (6.7); 8.4032 (0.4); 8.3889 (0.4); 7.6492 (0.4); 6.8362 (5.8); 6.7055 (2.5); 6.6728 (0.4); 6.6642 (6.6); 6.6499 (6.6); 6.5918 (0.4); 6.5776 (0.4); 6.5068 (0.3); 4.7647 (0.4); 3.3504 (0.7); 3.3256 (32.4); 2.9330 (2.4); 2.8701 (0.4); 2.8601 (3.5); 2.8520 (0.6); 2.8443 (3.3); 2.8419 (4.1); 2.8387 (3.2); 2.8234 (3.6); 2.5252 (0.6); 2.5205 (1.0); 2.5118 (14.9); 2.5073 (31.1); 2.5027 (41.5); 2.4981 (29.2); 2.4936 (13.5); 2.1360 (14.8); 2.0119 (1.0); 1.9776 (16.0); 1.8999 (1.0); 1.5537 (0.4); 1.5352 (1.8); 1.5265 (0.4); 1.5168 (3.2); 1.4985 (3.3); 1.4802 (2.0); 1.4620 (0.5); 1.1470 (5.0); 1.1294 (10.7); 1.1116 (4.8); 0.8514 (0.5); 0.8329 (1.2); 0.8278 (6.7); 0.8095 (14.0); 0.7911 (6.0); -0.0002 (3.8) |
| I.030: ¹H-NMR(400.2 MHz, d₆-DMSO): |
| δ= 8.8574 (3.6); 8.8428 (3.7); 7.6986 (0.5); 7.2013 (3.7); 7.1867 (3.6); 6.9226 (5.4); 6.7515 (2.2); 4.0380 (0.6); 4.0202 (0.6); 3.8168 (0.3); 3.4057 (0.4); 3.3215 (100.0); 2.9352 (2.0); 2.6754 (0.6); 2.6708 (0.9); 2.6662 (0.6); 2.5243 (2.5); 2.5196 (3.8); 2.5109 (54.5); 2.5064 (110.9); 2.5019 (145.7); 2.4973 (104.0); 2.4928 (49.6); 2.3377 (0.3); 2.3332 (0.7); 2.3288 (0.9); 2.3242 (0.6); 2.1754 (0.6); 2.1453 (14.2); 2.0587 (0.6); 1.9910 (16.0); 1.3979 (1.7); 1.1929 (0.7); 1.1752 (1.5); 1.1547 (4.0); 1.1370 (8.3); 1.1192 (3.8); 0.0080 (2.2); -0.0002 (75.1); -0.0085 (2.5) |
| I.031: ¹H-NMR(400.2 MHz, d₆-DMSO): |
| δ= 8.5305 (7.0); 8.5162 (7.3); 7.6537 (0.4); 6.8501 (5.8); 6.7910 (7.1); 6.7767 (7.1); 6.7170 (2.6); 6.7026 (0.5); 4.0647 (1.2); 4.0388 (3.8); 4.0128 (4.0); 3.9867 (1.4); 3.4247 (0.3); 3.4064 (0.4); 3.3289 (22.4); 2.9366 (2.5); 2.8722 (0.3); 2.7191 (0.4); 2.7178 (0.4); 2.5260 (0.4); 2.5213 (0.7); 2.5126 (11.1); 2.5081 (23.4); 2.5035 (31.2); 2.4989 (22.0); 2.4943 (10.2); 2.1368 (14.6); 2.0125 (0.5); 1.9756 (16.0); 1.8987 (0.5); 1.2335 (0.5); 1.1522 (5.0); 1.1345 (10.6); 1.1167 (4.8); 1.0860 (0.4); -0.0002 (1.6) |
| I.032: ¹H-NMR(400.2 MHz, d₆-DMSO): |
| δ= 8.5554 (1.1); 8.5411 (1.1); 6.8523 (1.0); 6.7165 (0.4); 6.6372 (1.1); 6.6229 (1.1); 3.3249 (6.4); 2.9352 (0.4); 2.5118 (4.0); 2.5074 (8.4); 2.5028 (11.0); 2.4982 (7.9); 2.4937 (3.7); 2.1363 (2.7); 1.9772 (2.9); 1.2725 (0.8); 1.2590 (0.5); 1.2351 (16.0); 1.2114 (0.6); 1.1526 (0.9); 1.1349 (1.9); 1.1171 (0.8) |
| I.033: ¹H-NMR(400.2 MHz, d₆-DMSO): |
| δ= 6.8253 (3.1); 6.7033 (1.3); 6.4196 (3.7); 5.7561 (0.5); 4.0377 (0.5); 4.0200 (0.5); 3.3239 (29.3); 3.2574 (0.4); 2.9317 (1.1); 2.6708 (0.4); 2.5244 (0.9); 2.5197 (1.4); 2.5110 (20.5); 2.5065 (42.4); 2.5019 (55.8); 2.4972 (39.2); 2.4927 (18.0); 2.3654 (16.0); 2.3457 (0.9); 2.3369 (11.5); 2.3242 (0.4); 2.1355 (8.0); 2.1038 (0.5); 2.0675 (0.4); 1.9888 (2.6); 1.9743 (8.7); 1.4249 (1.1); 1.3530 (1.2); 1.3390 (1.7); 1.1927 (0.7); 1.1750 (1.4); 1.1571 (0.8); 1.1491 (2.5); 1.1314 (5.4); 1.1136 (2.4) |
| I.034: ¹H-NMR(400.2 MHz, d₆-DMSO): |
| δ= 8.5340 (1.3); 8.5197 (1.4); 7.0902 (1.2); 7.0505 (1.3); 6.8552 (1.3); 6.7267 (0.6); 6.5858 (1.3); 6.5715 (1.3); 6.2066 (1.2); 6.1670 (1.1); 3.3269 (2.4); 2.9376 (0.5); 2.5126 (3.1); 2.5081 (6.4); 2.5035 (8.4); 2.4989 (6.0); 2.4943 (2.8); 2.1453 (3.3); 1.9880 (3.6); 1.1544 (1.1); 1.1367 (2.3); 1.1189 (1.1); 1.0896 (16.0); 0.0079 (0.4); -0.0002 (10.8); - 0.0086 (0.3) |
| I.035: ¹H-NMR(400.2 MHz, d₆-DMSO): |
| δ= 8.4814 (3.5); 8.4672 (3.6); 7.8032 (0.9); 7.6969 (0.4); 7.1872 (3.1); 7.1541 (1.0); 7.1290 (0.5); 6.8556 (3.5); 6.8414 (3.4); 3.4705 (0.5); 3.4549 (0.5); 3.3988 (0.5); 3.3825 (1.0); 3.3659 (0.9); 3.3490 (0.4); 3.3207 (16.1); 3.0167 (1.4); 2.9476 (3.4); 2.7085 (4.8); 2.6913 (5.0); 2.6757 (0.5); 2.6711 (0.6); 2.5065 (70.9); 2.5022 (90.5); 2.4979 (67.6); 2.3289 (0.6); 2.2410 (8.0); 1.6633 (0.5); 1.6464 (1.0); 1.6297 (1.2); 1.6131 (1.0); 1.5960 (0.5); 1.1606 (2.0); 1.1434 (4.1); 1.1262 (2.1); 0.7695 (16.0); 0.7529 (15.4); -0.0003 (3.7) |
| I.036: ¹H-NMR(400.2 MHz, d₆-DMSO): |
| δ= 8.4723 (6.7); 8.4581 (7.0); 7.7530 (0.9); 7.6494 (0.4); 7.0648 (5.2); 7.0354 (3.3); 6.8987 (4.0); 6.7846 (6.6); 6.7704 (6.5); 6.7619 (2.0); 3.4480 (0.5); 3.3975 (1.0); 3.3756 (1.0); 3.3609 (1.0); 3.3129 (33.1); 3.0048 (1.2); 2.9773 (0.8); 2.9429 (3.0); 2.8907 (0.6); 2.8113 (3.9); 2.7958 (3.8); 2.7931 (4.7); 2.7895 (3.6); 2.7744 (4.1); 2.7311 (0.4); 2.6793 (0.4); 2.6746 (0.7); 2.6700 (1.0); 2.6655 (0.7); 2.6608 (0.4); 2.5404 (1.7); 2.5236 (3.0); 2.5188 (4.7); 2.5101 (59.2); 2.5057 (118.9); 2.5011 (155.2); 2.4965 (111.4); 2.4920 (53.8); 2.3370 (0.3); 2.3325 (0.7); 2.3279 (1.0); 2.3234 (0.7); 2.2129 (11.4); 1.5112 (0.5); 1.4930 (2.1); 1.4745 (3.7); 1.4561 (3.8); 1.4378 (2.2); 1.4195 (0.6); 1.1581 (4.4); 1.1404 (9.2); 1.1226 (4.3); 0.7795 (7.6); 0.7613 (16.0); 0.7428 (6.8); 0.1459 (0.7); 0.0080 (6.0); -0.0001 (172.0); -0.0085 (6.1); -0.1497 (0.7) |
| I.037: ¹H-NMR(400.2 MHz, d₆-DMSO): |
| δ= 8.4587 (8.5); 8.4444 (9.0); 8.3161 (0.9); 7.7954 (1.3); 7.6858 (0.5); 7.4769 (3.8); 7.4725 (4.8); 7.4683 (2.4); 7.4661 (2.1); 7.4637 (2.0); 7.4591 (4.1); 7.4531 (6.2); 7.3591 (0.3); 7.3549 (0.4); 7.3426 (1.3); 7.3383 (2.0); 7.3286 (8.3); 7.3262 (8.7); 7.3204 (3.6); 7.3105 (4.6); 7.3018 (1.1); 7.2975 (1.2); 7.2924 (0.5); 7.2882 (0.8); 7.0868 (1.6); 7.0588 (0.7); 6.9891 (7.1); 6.8099 (8.6); 6.7957 (8.5); 5.7559 (2.1); 3.8299 (1.0); 3.4674 (0.6); 3.3988 (1.3); 3.3808 (1.3); 3.3241 (220.8); 3.0317 (1.6); 2.9549 (4.3); 2.6798 (0.7); 2.6752 (1.6); 2.6706 (2.2); 2.6660 (1.6); 2.6615 (0.8); 2.5242 (6.0); 2.5195 (8.9); 2.5108 (125.7); 2.5063 (261.1); 2.5017 (347.8); 2.4970 (249.5); 2.4925 (118.2); 2.3376 (0.7); 2.3331 (1.6); 2.3285 (2.2); 2.3239 (1.6); 2.3196 (0.7); 2.1335 (1.0); 2.1179 (16.0); 1.9886 (1.2); 1.3978 (13.3); 1.1926 (0.7); 1.1808 (2.5); 1.1749 (2.6); 1.1636 (5.0); 1.1464 (2.7); 0.1458 (2.6); 0.0275 (0.3); 0.0229 (0.5); 0.0080 (21.5); - 0.0002 (629.2); -0.0086 (20.9); -0.0204 (0.9); -0.1497 (2.6) |
| I.038: ¹H-NMR(400.2 MHz, d₆-DMSO): |
| δ= 8.6013 (1.0); 8.5870 (1.1); 7.0922 (1.0); 6.7941 (1.1); 6.7798 (1.1); 3.3238 (11.0); 2.9356 (0.7); 2.5244 (0.6); 2.5197 (1.0); 2.5110 (12.1); 2.5065 (24.4); 2.5020 (31.8); 2.4973 (22.6); 2.4928 (10.6); 2.1557 (2.2); 1.2185 (1.1); 1.2117 (16.0); 1.1614 (0.4); 1.1443 (0.7); 1.1273 (0.4); 0.0080 (0.8); -0.0002 (23.8); -0.0085 (0.7) |
| I.039: ¹H-NMR(400.2 MHz, d₆-DMSO): |
| δ= 8.4009 (7.2); 8.3866 (7.6); 7.6666 (0.4); 7.5224 (0.3); 7.5147 (3.8); 7.5108 (4.4); 7.5068 (1.8); 7.5054 (1.8); 7.5011 (1.5); 7.4961 (4.1); 7.4909 (5.4); 7.4026 (0.4); 7.3984 (0.4); 7.3856 (1.4); 7.3825 (1.8); 7.3755 (1.7); 7.3732 (2.9); 7.3686 (9.6); 7.3633 (4.5); 7.3586 (1.6); 7.3539 (2.2); 7.3500 (4.0); 7.3491 (4.0); 7.3451 (0.8); 7.3423 (0.7); 7.3398 (0.8); 7.3356 (1.3); 7.3320 (0.6); 7.3277 (0.9); 6.7809 (6.0); 6.6629 (2.8); 6.6576 (8.6); 6.6433 (7.6); 5.7562 (2.1); 3.3670 (0.8); 3.3288 (18.6); 2.9505 (2.3); 2.5253 (0.4); 2.5206 (0.6); 2.5118 (8.6); 2.5073 (18.2); 2.5027 (24.4); 2.4981 (17.3); 2.4935 (8.0); 2.1191 (15.4); 1.9634 (0.4); 1.9367 (16.0); 1.1647 (5.1); 1.1470 (10.9); 1.1292 (4.9); -0.0002 (2.8) |
| I.040: ¹H-NMR(400.2 MHz, d₆-DMSO): |
| δ= 8.4383 (6.0); 8.4240 (6.2); 7.6569 (0.5); 6.8408 (5.8); 6.7065 (2.7); 6.6335 (6.0); 6.6193 (5.9); 4.0381 (0.4); 4.0203 (0.4); 3.3484 (0.8); 3.3243 (15.6); 2.9362 (2.5); 2.8971 (7.1); 2.8791 (7.1); 2.5249 (0.7); 2.5203 (1.1); 2.5115 (16.1); 2.5070 (33.3); 2.5024 (44.3); 2.4978 (32.0); 2.4933 (15.3); 2.1333 (14.8); 2.0100 (0.3); 1.9890 (2.6); 1.9811 (16.0); 1.9035 (0.3); 1.3975 (11.2); 1.1932 (0.5); 1.1754 (1.0); 1.1522 (4.8); 1.1346 (10.2); 1.1168 (4.6); 1.0463 (0.4); 1.0345 (0.8); 1.0270 (0.8); 1.0228 (0.6); 1.0150 (1.4); 1.0072 (0.6); 1.0029 (0.8); 0.9953 (0.9); 0.9832 (0.5); 0.9770 (0.3); 0.4662 (1.2); 0.4556 (3.3); 0.4510 (3.5); 0.4464 (1.7); 0.4409 (1.6); 0.4355 (3.4); 0.4310 (3.3); 0.4209 (1.2); 0.1816 (1.3); 0.1712 (3.7); 0.1674 (3.8); 0.1593 (3.4); 0.1554 (3.9); 0.1448 (1.2); 0.0080 (2.0); -0.0002 (64.5); -0.0085 (2.4) |
| I.041: ¹H-NMR(400.2 MHz, d₆-DMSO): |
| δ= 8.3845 (6.5); 8.3703 (6.7); 8.3493 (0.9); 8.3352 (0.9); 7.6633 (0.4); 6.8198 (5.7); 6.6954 (2.5); 6.6590 (0.8); 6.5549 (6.2); 6.5408 (6.2); 6.4999 (0.7); 6.4766 (0.8); 6.4624 (0.8); 5.7548 (7.0); 5.2161 (0.5); 5.2090 (0.9); 5.2010 (1.1); 5.1945 (1.7); 5.1879 (1.2); 5.1799 (1.0); 5.1727 (0.5); 4.7627 (0.8); 4.0381 (0.9); 4.0203 (0.9); 3.3484 (0.8); 3.3202 (31.1); 3.2579 (1.4); 2.9312 (2.4); 2.6754 (0.6); 2.6708 (0.8); 2.6662 (0.6); 2.5243 (2.3); 2.5197 (3.3); 2.5109 (48.2); 2.5064 (100.6); 2.5018 (133.4); 2.4972 (95.6); 2.4926 (45.8); 2.3332 (0.6); 2.3286 (0.8); 2.3240 (0.6); 2.1335 (14.6); 2.0104 (2.3); 1.9886 (4.4); 1.9712 (16.0); 1.8941 (2.5); 1.8813 (0.7); 1.8589 (1.5); 1.8474 (1.6); 1.8316 (1.1); 1.8267 (1.0); 1.8171 (0.9); 1.6807 (1.4); 1.6739 (2.3); 1.6602 (4.4); 1.6550 (3.2); 1.6490 (3.3); 1.6457 (3.2); 1.6300 (1.6); 1.6217 (1.0); 1.5793 (0.4); 1.5605 (1.1); 1.5523 (1.1); 1.5368 (2.2); 1.5057 (0.5); 1.3979 (5.9); 1.2353 (0.3); 1.1931 (1.2); 1.1753 (2.5); 1.1575 (1.4); 1.1493 (4.8); 1.1316 (10.4); 1.1138 (4.6); 0.1459 (0.7); 0.0125 (0.5); 0.0080 (5.7); - 0.0002 (182.8); -0.0085 (6.3); -0.0150 (0.4); -0.1496 (0.7) |
| I.042: ¹H-NMR(400.2 MHz, d₆-DMSO): |
| δ= 8.4609 (7.2); 8.4467 (7.6); 7.6385 (0.5); 7.2456 (0.8); 7.2408 (0.6); 7.2379 (1.0); 7.2338 (0.7); 7.2305 (0.7); 7.2286 (0.8); 7.2241 (3.8); 7.2196 (2.2); 7.2152 (1.6); 7.2106 (4.2); 7.2052 (9.3); 7.2001 (3.5); 7.1910 (1.9); 7.1885 (1.4); 7.1759 (0.5); 7.1713 (0.5); 7.1515 (4.7); 7.1461 (4.0); 7.1407 (1.4); 7.1319 (3.1); 7.1279 (2.4); 6.8881 (6.0); 6.7561 (2.6); 6.7083 (7.0); 6.6940 (7.1); 5.7563 (0.6); 4.1632 (12.4); 3.3832 (0.4); 3.3286 (31.1); 2.9218 (2.9); 2.5251 (0.5); 2.5204 (0.7); 2.5117 (11.3); 2.5072 (23.6); 2.5026 (31.2); 2.4980 (21.9); 2.4934 (10.1); 2.1563 (15.0); 1.9972 (16.0); 1.1346 (6.3); 1.1169 (14.1); 1.0991 (6.1); -0.0002 (3.3) |
| I.043: ¹H-NMR(400.2 MHz, d₆-DMSO): |
| δ= 8.4355 (5.8); 8.4212 (6.0); 7.6393 (0.4); 6.8309 (5.6); 6.6958 (3.0); 6.6553 (5.7); 6.6410 (5.6); 3.3531 (0.9); 3.3265 (102.2); 2.9327 (2.6); 2.9148 (4.2); 2.9017 (2.0); 2.8939 (2.5); 2.8918 (2.7); 2.8840 (1.8); 2.8711 (3.4); 2.6758 (0.5); 2.6712 (0.7); 2.6668 (0.5); 2.5248 (2.1); 2.5201 (3.1); 2.5114 (42.5); 2.5069 (87.0); 2.5023 (113.8); 2.4977 (79.7); 2.4932 (37.0); 2.3337 (0.5); 2.3291 (0.7); 2.3245 (0.5); 2.1326 (15.2); 1.9712 (16.0); 1.2314 (0.4); 1.1473 (4.4); 1.1296 (9.4); 1.1119 (4.3); 0.8435 (3.8); 0.8305 (1.8); 0.8225 (2.5); 0.8205 (2.5); 0.8128 (1.8); 0.7998 (3.6); 0.1039 (0.5); 0.0321 (1.3); 0.0080 (1.0); -0.0002 (33.6); -0.0085 (1.0); -0.0162 (1.1); -0.0347 (3.6); -0.0428 (102.0); -0.0511 (3.7); -0.1929 (0.5) |
| I.044: ¹H-NMR(400.2 MHz, d₆-DMSO): |
| δ= 8.6723 (1.0); 8.6581 (1.0); 8.3150 (0.4); 7.3485 (0.3); 7.2264 (1.0); 7.0139 (1.0); 6.9997 (1.0); 3.3195 (35.2); 3.0190 (0.4); 2.9494 (1.0); 2.6749 (0.9); 2.6704 (1.1); 2.6657 (0.8); 2.6618 (0.4); 2.5238 (3.7); 2.5189 (5.9); 2.5103 (68.9); 2.5059 (135.6); 2.5014 (174.6); 2.4968 (125.3); 2.4923 (60.1); 2.3372 (0.4); 2.3327 (0.8); 2.3282 (1.1); 2.3237 (0.8); 2.3192 (0.4); 2.2589 (1.9); 1.2038 (16.0); 1.1758 (0.3); 1.1580 (0.7); 1.1406 (0.5); -0.0002 (7.6) |
| I.045: ¹H-NMR(400.2 MHz, d₆-DMSO): |
| δ= 8.4562 (1.6); 8.4420 (1.6); 7.7145 (0.5); 7.1920 (2.4); 6.9471 (0.5); 6.8166 (1.5); 6.8024 (1.5); 3.3686 (0.4); 3.3505 (0.4); 3.3232 (9.4); 3.0108 (0.6); 2.9398 (1.4); 2.5200 (0.4); 2.5112 (6.8); 2.5068 (14.3); 2.5023 (19.1); 2.4977 (13.8); 2.4933 (6.7); 2.0024 (5.3); 1.3234 (0.4); 1.2998 (16.0); 1.2336 (0.4); 1.1673 (0.7); 1.1498 (1.4); 1.1324 (0.8); -0.0002 (1.5) |
| I.046: ¹H-NMR(400.2 MHz, d₆-DMSO): |
| δ= 8.4788 (3.9); 8.4646 (4.0); 8.3163 (0.3); 7.7580 (1.0); 7.6410 (0.4); 7.1988 (5.2); 6.9512 (1.0); 6.9231 (0.4); 6.7558 (3.5); 6.7416 (3.4); 3.5895 (0.4); 3.5725 (1.0); 3.5555 (1.4); 3.5384 (1.0); 3.5213 (0.4); 3.4652 (0.4); 3.4474 (0.4); 3.3876 (0.4); 3.3697 (0.8); 3.3524 (0.8); 3.3242 (21.1); 3.0118 (1.6); 2.9830 (0.4); 2.9814 (0.4); 2.9419 (3.2); 2.5249 (0.6); 2.5201 (0.9); 2.5114 (15.4); 2.5069 (32.0); 2.5023 (42.6); 2.4977 (30.5); 2.4932 (14.6); 2.0100 (11.2); 1.2643 (0.3); 1.2463 (16.0); 1.2292 (15.9); 1.2140 (0.4); 1.1821 (0.3); 1.1688 (1.4); 1.1513 (2.7); 1.1341 (1.5); -0.0002 (0.8) |
| I.047: ¹H-NMR(400.2 MHz, d₆-DMSO): |
| δ= 8.7455 (6.9); 8.7312 (7.2); 8.4714 (0.9); 8.4647 (8.8); 8.4604 (2.6); 8.4500 (2.5); 8.4456 (9.4); 8.4389 (0.9); 8.4140 (0.4); 8.3998 (0.4); 7.7057 (0.5); 7.2762 (0.9); 7.2695 (9.4); 7.2652 (2.6); 7.2548 (2.6); 7.2504 (9.4); 7.2438 (0.9); 6.9473 (5.9); 6.9185 (7.1); 6.9042 (7.1); 6.8266 (0.4); 6.7797 (1.9); 6.5527 (0.4); 6.5385 (0.4); 4.0554 (0.9); 4.0376 (2.9); 4.0198 (2.9); 4.0020 (1.0); 3.8096 (1.9); 3.3623 (0.8); 3.3247 (38.5); 3.2459 (0.5); 2.9413 (2.2); 2.6755 (0.5); 2.6709 (0.8); 2.6663 (0.5); 2.5245 (2.0); 2.5197 (3.0); 2.5110 (43.6); 2.5065 (90.9); 2.5019 (120.2); 2.4973 (85.3); 2.4927 (39.8); 2.3334 (0.5); 2.3287 (0.7); 2.3241 (0.6); 2.1637 (14.3); 2.1340 (1.0); 2.0408 (16.0); 1.9892 (13.3); 1.9759 (1.0); 1.3972 (1.2); 1.1924 (3.7); 1.1746 (7.6); 1.1617 (4.2); 1.1568 (4.7); 1.1441 (8.7); 1.1263 (4.0); 1.1141 (0.4); 0.0080 (0.5); -0.0002 (18.9); -0.0085 (0.5) |
| I.048: ¹H-NMR(400.2 MHz, d₆-DMSO): |
| δ= 8.4667 (4.0); 8.4525 (4.2); 7.7388 (1.0); 7.6311 (0.4); 7.1824 (5.7); 6.9547 (1.1); 6.9304 (0.5); 6.7871 (3.6); 6.7729 (3.5); 3.4597 (0.4); 3.4424 (0.4); 3.3808 (0.4); 3.3641 (0.9); 3.3468 (0.9); 3.3254 (10.4); 3.0052 (1.2); 2.9841 (0.4); 2.9405 (3.3); 2.7635 (4.8); 2.7463 (4.9); 2.5256 (0.4); 2.5209 (0.6); 2.5123 (8.8); 2.5078 (18.0); 2.5033 (23.6); 2.4987 (16.9); 2.4942 (8.1); 1.9968 (12.5); 1.7129 (0.4); 1.6961 (0.9); 1.6794 (1.2); 1.6626 (1.0); 1.6459 (0.5); 1.1627 (1.6); 1.1452 (3.2); 1.1279 (1.7); 0.8235 (16.0); 0.8069 (15.2); -0.0002 (0.5) |
| I.049: ¹H-NMR(400.2 MHz, d₆-DMSO): |
| δ= 8.5351 (4.0); 8.5209 (4.1); 7.8302 (1.0); 7.7291 (0.4); 7.3617 (1.2); 7.3168 (0.4); 7.2421 (3.6); 6.9554 (3.7); 6.9413 (3.7); 5.7577 (0.6); 4.0378 (0.8); 4.0200 (0.9); 3.4683 (0.3); 3.4512 (0.4); 3.3877 (0.3); 3.3704 (0.8); 3.3529 (0.8); 3.3330 (0.5); 3.3212 (34.6); 3.0132 (1.3); 2.9451 (3.5); 2.7328 (4.2); 2.7156 (4.4); 2.6751 (0.4); 2.6705 (0.5); 2.6659 (0.4); 2.5240 (1.4); 2.5193 (2.0); 2.5106 (29.0); 2.5061 (60.6); 2.5015 (80.7); 2.4969 (58.0); 2.4923 (27.6); 2.3328 (0.4); 2.3283 (0.5); 2.3237 (0.3); 2.2591 (6.5); 1.9889 (3.9); 1.6926 (0.4); 1.6760 (0.8); 1.6592 (1.0); 1.6424 (0.8); 1.6256 (0.4); 1.3976 (16.0); 1.1924 (1.1); 1.1746 (2.4); 1.1684 (1.0); 1.1567 (1.9); 1.1505 (2.3); 1.1325 (1.7); 1.1132 (0.4); 0.7924 (14.5); 0.7757 (14.0); -0.0002 (1.7) |
| I.050: ¹H-NMR(400.2 MHz, d₆-DMSO): |
| δ= 8.5403 (6.9); 8.5261 (7.0); 8.3137 (0.5); 7.8401 (2.3); 7.7347 (0.9); 7.3552 (2.6); 7.3098 (1.0); 7.2409 (7.8); 6.9473 (7.0); 6.9332 (6.8); 3.4701 (0.8); 3.4547 (0.9); 3.3886 (0.9); 3.3723 (2.0); 3.3546 (2.0); 3.3169 (135.3); 3.0149 (2.9); 2.9463 (7.7); 2.8071 (4.2); 2.7889 (5.2); 2.7702 (4.4); 2.6748 (1.4); 2.6703 (1.8); 2.6658 (1.4); 2.5235 (7.4); 2.5101 (114.0); 2.5058 (219.6); 2.5013 (283.4); 2.4968 (206.3); 2.4925 (101.9); 2.3327 (1.2); 2.3282 (1.7); 2.3236 (1.2); 2.2591 (15.0); 1.5190 (0.5); 1.5009 (2.3); 1.4825 (4.0); 1.4640 (4.1); 1.4457 (2.4); 1.4273 (0.6); 1.2355 (0.4); 1.1707 (2.3); 1.1533 (5.3); 1.1352 (3.9); 0.7818 (7.8); 0.7636 (16.0); 0.7452 (7.0); 0.0078 (1.4); -0.0002 (35.6); -0.0084 (1.4) |
| I.051: ¹H-NMR(400.2 MHz, d₆-DMSO): |
| δ= 8.3298 (4.0); 8.3285 (4.0); 7.7333 (0.6); 7.0578 (3.4); 7.0346 (1.5); 7.0249 (1.2); 6.8982 (2.3); 6.7615 (1.1); 3.4484 (0.4); 3.4242 (0.4); 3.3737 (0.7); 3.3584 (0.7); 3.3214 (40.5); 3.0020 (0.8); 2.9419 (2.0); 2.6750 (0.5); 2.6707 (0.7); 2.6599 (4.9); 2.6427 (4.9); 2.5241 (1.9); 2.5105 (35.1); 2.5063 (69.2); 2.5018 (89.6); 2.4973 (66.0); 2.3331 (0.4); 2.3285 (0.5); 2.3243 (0.4); 2.2172 (7.8); 2.2062 (13.7); 1.6258 (0.4); 1.6090 (0.9); 1.5923 (1.2); 1.5754 (0.9); 1.5587 (0.5); 1.1559 (3.1); 1.1382 (6.6); 1.1205 (3.0); 0.7406 (16.0); 0.7239 (15.3); -0.0002 (3.2) |

### LogP measurement

Measurement of LogP values was performed according to EEC directive 79/831 Annex V.A8 by HPLC (High Performance Liquid Chromatography) on reversed phase columns with the following methods:
^{[a]} LogP value is determined by measurement of LC-UV, in an acidic range, with 0.1% formic acid in water and acetonitrile as eluent (linear gradient from 10% acetonitrile to 95% acetonitrile).
^{[b]} LogP value is determined by measurement of LC-UV, in a neutral range, with 0.001 molar ammonium acetate solution in water and acetonitrile as eluent (linear gradient from 10% acetonitrile to 95% acetonitrile).
^{[c]} LogP value is determined by measurement of LC-UV, in an acidic range, with 0.1% phosphoric acid and acetonitrile as eluent (linear gradient from 10% acetonitrile to 95% acetonitrile).

If more than one LogP value is available within the same method, all the values are given and separated by "+".

Calibration was done with straight-chain alkan2-ones (with 3 to 16 carbon atoms) with known LogP values (measurement of LogP values using retention times with linear interpolation between successive alkanones). Lambda-max-values were determined using UV-spectra from 200 nm to 400 nm and the peak values of the chromatographic signals.

### NMR-Peak Lists

1H-NMR data of selected examples are written in form of 1H-NMR-peak lists. To each signal peak are listed the δ-value in ppm and the signal intensity in round brackets. Between the δ-value - signal intensity pairs are semicolons as delimiters.

The peak list of an example has therefore the form:
δ₁ (intensity₁); δ₂ (intensity₂);........; δᵢ (intensityᵢ);......; δₙ (intensityₙ)

Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown.

For calibrating chemical shift for 1H spectra, we use tetramethylsilane and/or the chemical shift of the solvent used, especially in the case of spectra measured in DMSO. Therefore in NMR peak lists, tetramethylsilane peak can occur but not necessarily.

The 1H-NMR peak lists are similar to classical 1H-NMR prints and contains therefore usually all peaks, which are listed at classical NMR-interpretation.

Additionally they can show like classical 1H-NMR prints signals of solvents, stereoisomers of the target compounds, which are also object of the invention, and/or peaks of impurities.

To show compound signals in the delta-range of solvents and/or water the usual peaks of solvents, for example peaks of DMSO in DMSO-D₆ and the peak of water are shown in our 1H-NMR peak lists and have usually on average a high intensity .

The peaks of stereoisomers of the target compounds and/or peaks of impurities have usually on average a lower intensity than the peaks of target compounds (for example with a purity >90%).

Such stereoisomers and/or impurities can be typical for the specific preparation process. Therefore their peaks can help to recognize the reproduction of our preparation process via "side-products-fingerprints".

An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking at classical 1H-NMR interpretation.

Further details of NMR-data description with peak lists you find in the publication "Citation of NMR Peaklist Data within Patent Applications" of the Research Disclosure Database Number 564025.

The examples (1.001) - (1.051) of the compounds formula (I) can be obtained by the herein described synthesis routes as shown in the following Table 4.

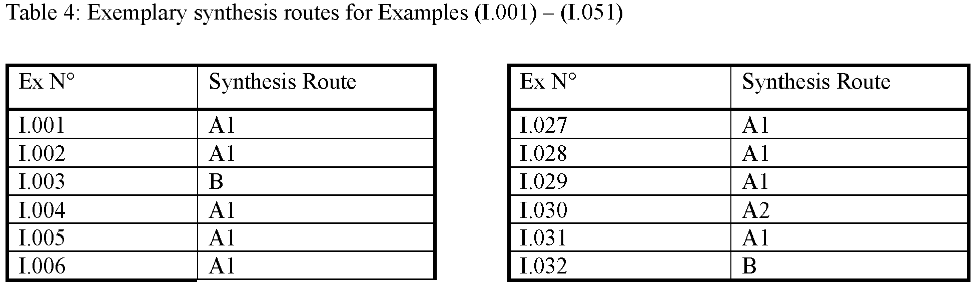

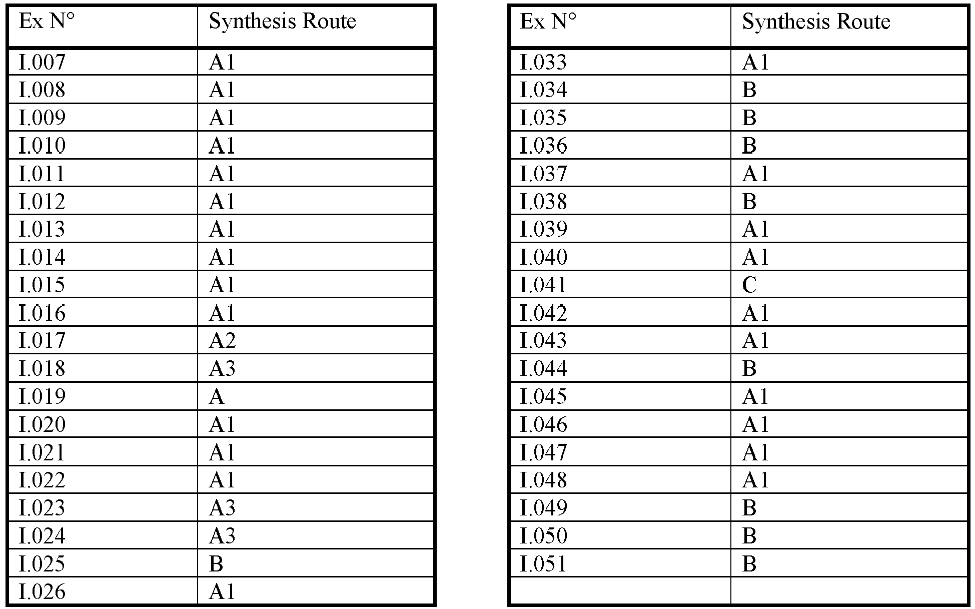

The present invention will be illustrated with the biological examples. However the invention is not limited to the examples.

### Example: in vivo preventive test on Puccinia recondita (brown rust on wheat)

| | | |
|---|---|---|
| Solvent: | 5% | by volume of Dimethyl sulfoxide |
| | 10% | by volume of Acetone |
| Emulsifier: | 1µl | of Tween® 80 per mg of active ingredient |

The active ingredients were made soluble and homogenized in a mixture of Dimethyl sulfoxide/Acetone/ /Tween® 80 and then diluted in water to the desired concentration.

The young plants of wheat were treated by spraying the active ingredient prepared as described above. Control plants were treated only with an aqueous solution of Acetone/Dimethyl sulfoxide/ Tween® 80.

After 24 hours, the plants were contaminated by spraying the leaves with an aqueous suspension of *Puccinia recondita* spores. The contaminated wheat plants were incubated for 24 hours at 20°C and at 100% relative humidity and then for 10 days at 20°C and at 70-80% relative humidity.

The test was evaluated 11 days after the inoculation. 0% means an efficacy which corresponds to that of the control plants while an efficacy of 100% means that no disease was observed.

In this test, the following compounds according to the invention showed efficacy of at least 70% at a concentration of 500 ppm of active ingredient: 1.001; 1.002; 1.003; 1.004; I.005; 1.006; 1.007; 1.008; 1.009; 1.010; 1.011; 1.012; 1.013; 1.014; I.015; 1.016; 1.017; 1.018; 1.019; 1.020; 1.021; 1.022; 1.023; 1.024; 1.025; 1.026; 1.027; 1.028; 1.029; 1.030; 1.031; 1.032; 1.033; 1.034; 1.035; 1.036; 1.037; 1.038; 1.039; 1.040; 1.041; 1.042; 1.043; 1.044; 1.045; 1.046; 1.048; 1.049; I.050.

### Example: in vivo preventive test on Sphaerotheca fuliginea (powdery mildew on cucurbits)

| | | |
|---|---|---|
| Solvent: | 5% | by volume of Dimethyl sulfoxide |
| | 10% | by volume of Acetone |
| Emulsifier: | 1µl | of Tween® 80 per mg of active ingredient |

The active ingredients were made soluble and homogenized in a mixture of Dimethyl sulfoxide/Acetone/ /Tween® 80 and then diluted in water to the desired concentration.

The young plants of gherkin were treated by spraying the active ingredient prepared as described above. Control plants were treated only with an aqueous solution of Acetone/Dimethyl sulfoxide/ 80.

After 24 hours, the plants were contaminated by spraying the leaves with an aqueous suspension of *Sphaerotheca fuliginea* spores. The contaminated gherkin plants were incubated for 8 days at 20°C and at 70-80% relative humidity.

The test was evaluated 8 days after the inoculation. 0% means an efficacy which corresponds to that of the control plants while an efficacy of 100% means that no disease was observed.

In this test, the following compounds according to the invention showed efficacy of at least 70% at a concentration of 500 ppm of active ingredient: I.001; I.002; I.003; I.004; I.005; I.006; I.007; I.008; I.009; I.010; I.011; I.012; I.013; I.014; I.015; I.016; I.017; I.018; I.019; I.020; I.021; I.022; I.023; I.024; I.025; I.027; I.028; I.029; I.030; I.031; I.033; I.034; I.036; I.037; I.039; I.040; I.041; I.042; I.043; I.044; I.045; I.047; I.048; I.049; I.050; I.051

### Example: in vivo preventive test on Phakospora pachyrhizi (soybean rust)

| | | |
|---|---|---|
| Solvent: | 5% | by volume of Dimethyl sulfoxide |
| | 10% | by volume of Acetone |
| Emulsifier: | 1µl | of Tween® 80 per mg of active ingredient |

The active ingredients were made soluble and homogenized in a mixture of Dimethyl sulfoxide/Acetone/ /Tween® 80 and then diluted in water to the desired concentration.

The young plants of soybean were treated by spraying the active ingredient prepared as described above. Control plants were treated only with an aqueous solution of Acetone/Dimethyl sulfoxide/ 80.

After 24 hours, the plants were contaminated by spraying the leaves with an aqueous suspension of *Phakosporapachyrhizi* spores. The contaminated soybean plants were incubated for 24 hours at 24°C and at 100% relative humidity and then for 11 days at 24°C and at 70-80% relative humidity.

The test was evaluated 12 days after the inoculation. 0% means an efficacy which corresponds to that of the control plants while an efficacy of 100% means that no disease was observed.

In this test, the following compounds according to the invention showed efficacy of at least 70% at a concentration of 500 ppm of active ingredient: I.001; I.002; I.003; I.004; I.005; I.006; I.007; I.008; I.009; I.010; I.011; I.012; I.013; I.014; I.015; I.016; I.017; I.018; I.019; I.020; I.021; I.022; I.023; I.024; I.025; I.026; I.027; I.028; I.029; I.030; I.031; I.032; I.033; I.034; I.035; I.036; I.037; I.038; I.039; I.040; I.041; I.042; I.043; I.044; I.045

## Claims

1. Compounds of the formula (I) 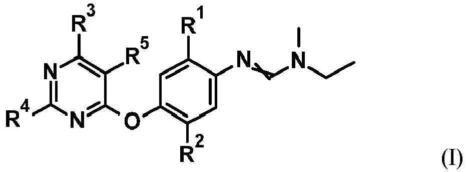 in which
R¹ and R² are independently selected from the group consisting of
cyano, halogen, -Si(R⁶)(R⁷)(R⁸), and
C₁-C₈-Alkyl, C₁-C₈-Alkoxy, C₃-C₈-Cycloalkyl, C₁-C₈-Haloalkyl, C₂-C₈-Alkenyl, C₂-C₈-Alkynyl, C₁-C₈-Alkylsulfanyl, C₁-C₈-Alkylsulfinyl, C₁-C₈-Alkylsulfonyl, -C(O)-C₁-C₈-Alkyl, -C(O)-C₃-C₈-Cycloalkyl, -C(O)NH-C₁-C₈-Alkyl, -C(O)N-di-C₁-C₈-Alkyl,-C(O)O-C₁-C₈-Alkyl, -NH-C₁-C₈-Alkyl, -N-di-C₁-C₈-Alkyl, each of which may be independently non-substituted or substituted with one or more substituent(s) selected from the group consisting of C₁-C₈-Alkoxy and halogen,
R³ and R⁴ are independently selected from the group consisting of
hydrogen,
C₁-C₈-Alkyl, C₂-C₈-Alkenyl, C₁-C₈-Alkoxy, C₃-C₈-Cycloalkyl, C₃-C₈-Cycloalkoxy, each of which may be independently non-substituted or substituted with one or more substituent(s) selected from the group consisting of C₁-C₈-Alkyl, C₁-C₈-Haloalkyl, C₁-C₈-Alkoxy, C₆-C₁₄-Aryl, halogen, and -Si(R⁶)(R⁷)(R⁸), and
-S(O)ₙ-R'
wherein
n represents 0, 1, or 2, and
R' is selected from the group consisting of C₁-C₈-Alkyl, C₃-C₈-Cycloalkyl, C₃-C₈-Cycloalkyl-C₁-C₈-Alkyl, C₃-C₈-Cycloalkoxy, C₆-C₁₄-Aryl, Aryl-Ci-Cs-Alkyl, Heteroaryl, each of which may be independently non-substituted or substituted with one or more substituent(s) selected from the group consisting of C₁-C₈-Alkyl, C₁-C₈-Haloalkyl, C₁-C₈-Alkoxy, C₆-C₁₄-Aryl, halogen, and -Si(R⁶)(R⁷)(R⁸), and
R⁵ is selected from the group consisting of hydrogen, halogen, cyano, C₁-C₈-Alkyl, C₂-C₈-Alkenyl, C₂-C₈-Alkynyl, C₃-C₆-Cycloalkyl, C₃-C₆-Cycloalkoxy, C₁-C₈-Alkylsulfanyl, C₁-C₈-Alkylsulfinyl, C₁-C₈-Alkylsulfonyl, and wherein
R⁶, R⁷ and R⁸ are independently from each other selected from C₁-C₈-Alkyl and phenyl,
or salts, N-oxides, metal complexes, or stereoisomers thereof.

2. Compounds according to claim 1, wherein
R¹ and R² are independently selected from the group consisting of C₁-C₈-Alkyl, C₁-C₈-Alkoxy, C₃-C₈-Cycloalkyl, C₁-C₈-Haloalkyl, C₂-C₈-Alkenyl, C₂-C₈-Alkynyl, C₁-C₈-Alkylsulfanyl, C₁-C₈-Alkylsulfinyl, C₁-C₈-Alkylsulfonyl, cyano, and halogen,
R³ is selected from the group consisting of
hydrogen, C₁-C₈-Alkyl, C₂-C₈-Alkenyl, C₁-C₈-Haloalkyl, C₁-C₈-Alkoxy, C₃-C₈-Cycloalkyl, C₃-C₈-Cycloalkoxy, C₁-C₈-Alkylsulfanyl, C₁-C₈-Alkylsulfinyl, C₁-C₈-Alkylsulfonyl, C₃-C₈-Cycloalkylsulfanyl, C₃-C₈-Cycloalkyl-S(O)-, C₃-C₈-Cycloalkyl-SO₂-, C₃-C₈-Cycloalkyl-C₁-C₈-Alkylsulfanyl, C₆-C₁₄-Aryl-S-, C₆-C₁₄-Aryl-S- having one or more halogen substituents, C₆-C₁₄-Aryl-S(O)-, C₆-C₁₄-Aryl-S(O)- having one or more halogen substituents, C₆-C₁₄-Aryl-SO₂-, C₆-C₁₄-Aryl-SO₂- having one or more halogen substituents, Aryl-C₁-C₈-Alkyl-S-, C₁-C₈-Haloalkylsulfanyl, C₁-C₈-Alkylsulfanyl substituted with -Si(R⁶)(R⁷)(R⁸), C₁-C₈-Alkylsulfinyl substituted with -Si(R⁶)(R⁷)(R⁸), C₁-C₈-Alkylsulfonyl substituted with -Si(R⁶)(R⁷)(R⁸), Heteroaryl-S-, Heteroaryl-S(O)-, Heteroaryl-SO₂-,
R⁴ is selected from the group consisting of
C₁-C₈-Alkyl, C₂-C₈-Alkenyl, C₁-C₈-Haloalkyl, C₃-C₈-Cycloalkyl, C₃-C₈-Cycloalkoxy, C₁-C₈-Alkylsulfanyl, C₁-C₈-Alkylsulfinyl, C₁-C₈-Alkylsulfonyl, C₃-C₈-Cycloalkylsulfanyl, C₃-C₈-Cycloalkyl-S(O)-, C₃-C₈-Cycloalkyl-SO₂-, C₃-C₈-Cycloalkyl-C₁-C₈-Alkylsulfanyl, C₆-C₁₄-Aryl-S-, C₆-C₁₄-Aryl-S- having one or more halogen substituents, C₆-C₁₄-Aryl-S(O)-, C₆-C₁₄-Aryl-S(O)- having one or more halogen substituents, C₆-C₁₄-Aryl-SO₂-, C₆-C₁₄-Aryl-SO₂- having one or more halogen substituents, Aryl-C₁-C₈-Alkyl-S-, C₁-C₈-Haloalkylsulfanyl, C₁-C₈-Alkylsulfanyl substituted with -Si(R⁶)(R⁷)(R⁸), C₁-C₈-Alkylsulfinyl substituted with -Si(R⁶)(R⁷)(R⁸), C₁-C₈-Alkylsulfonyl substituted with -Si(R⁶)(R⁷)(R⁸), Heteroaryl-S-, Heteroaryl-S(O)-, Heteroaryl-SO₂-, wherein R⁶, R⁷ and R⁸ are in each case independently from each other selected from C₁-C₈-Alkyl,
and
R⁵ is selected from the group consisting of hydrogen, halogen, cyano, C₁-C₈-Alkyl, C₂-C₈-Alkenyl, C₂-C₈-Alkynyl, C₃-C₆-Cycloalkyl, C₃-C₆-Cycloalkoxy, C₁-C₈-Alkylsulfanyl, C₁-C₈-Alkylsulfinyl, C₁-C₈-Alkylsulfonyl,
or salts, N-oxides, metal complexes, or stereoisomers thereof.

3. Compounds according to claim 1 or 2, wherein
R¹ and R² are independently selected from the group consisting of C₁-C₆-Alkyl, C₁-C₆-Haloalkyl, cyano, and halogen,
R³ is selected from the group consisting of
hydrogen, C₁-C₆-Alkyl, C₂-C₆-Alkenyl, C₁-C₆-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S- substituted with 1 to 5 halogen, C₆-C₁₄-Aryl-C₁-C₆-Alkyl-S-, C₁-C₃-Haloalkylsulfanyl, C₁-C₆-Alkylsulfanyl substituted with -Si(R⁶)(R⁷)(R⁸), Heteroaryl-S-,
R⁴ is selected from the group consisting of
C₁-C₆-Alkyl, C₂-C₆-Alkenyl, C₁-C₆-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S- substituted with 1 to 5 halogen, C₆-C₁₄-Aryl-C₁-C₆-Alkyl-S-, C₁-C₃-Haloalkylsulfanyl, C₁-C₆-Alkylsulfanyl substituted with -Si(R⁶)(R⁷)(R⁸), Heteroaryl-S-, wherein R⁶, R⁷ and R⁸ are in each case independently from each other selected from C₁-C₆-Alkyl, and
R⁵ is selected from the group consisting of hydrogen and C₁-C₆-Alkyl,
or salts, N-oxides, metal complexes, or stereoisomers thereof.

4. Compounds according to one of the claims 1, 2 or 3, wherein
R¹ is selected from the group consisting of C₁-C₆-Alkyl and halogen,
R² is selected from the group consisting of C₁-C₆-Alkyl, C₁-C₆-Haloalkyl, cyano, and halogen,
R³ is selected from the group consisting of hydrogen, C₁-C₆-Alkyl, and C₁-C₆-Alkylsulfanyl,
R⁴ is selected from the group consisting of
C₁-C₆-Alkyl, C₂-C₆-Alkenyl, C₁-C₆-Haloalkyl, C₃-C₆-Cycloalkoxy, C₁-C₆-Alkylsulfanyl, C₃-C₆-Cycloalkylsulfanyl, C₃-C₆-Cycloalkyl-C₁-C₆-Alkylsulfanyl, C₆-C₁₄-Aryl-S-, C₆-C₁₄-Aryl-S- substituted with 1 to 5 halogen, C₆-C₁₄-Aryl-C₁-C₆-Alkyl-S-, C₁-C₃-Haloalkylsulfanyl, C₁-C₆-Alkylsulfanyl substituted with -Si(R⁶)(R⁷)(R⁸), Heteroaryl-S-,
wherein R⁶, R⁷ and R⁸ are independently from each other selected from C₁-C₆-Alkyl, and
R⁵ is selected from the group consisting of hydrogen and C₁-C₆-Alkyl,
or salts, N-oxides, metal complexes, or stereoisomers thereof.

5. Compounds according to one of the claims 1, 2, 3 or 4, wherein
R¹ is selected from the group consisting of methyl and chlorine,
R² is selected from the group consisting of methyl, difluoromethyl, trifluoromethyl, cyano, and bromine,
R³ is selected from the group consisting of hydrogen, methyl, butan-2-ylthio, and tert-butylthio,
R⁴ is selected from the group consisting of
methyl, 3,3-dimethylbutyl, tert-butyl,
(E)-3,3 -dimethylbut-1-en-1-yl,
trifluoromethyl,
cyclopentyloxy,
methylsulfanyl, ethylsulfanyl, propylthio, isopropylthio, butan-2-ylthio, 2-methylpropylthio, 3-methylbutylthio, tert-butylthio, cyclopropylmethylthio, phenylsulfanyl, benzylsulfinyl, 2,2,2-trifluoroethylthio, 3,3,3-trifluoropropylthio, 2-trimethylsilylethylthio, cyclohexylthio, cyclopentylthio, (2-fluorophenyl)thio, (3-fluorophenyl)thio, (4-fluorophenyl)thio, pyridin-4-ylthio, and
R⁵ is selected from the group consisting of hydrogen and methyl,
or salts, N-oxides, metal complexes, or stereoisomers thereof.

6. Compounds according to one of the claims 1, 2, 3, 4 or 5, wherein compounds are selected from the group consisting of (1.001) N'-[5-bromo-4-(2-tert-butylsulfanylpyrimidin-4-yl)oxy-2-methylphenyl]-N-ethyl-N-methylmethanimidamide, (I.002) N'-[2,5-dimethyl-4-[2-(3-methylbutylsulfanyl)pyrimidin-4-yl]oxyphenyl]-N-ethyl-N-methylmethanimidamide, (I.003) N'-[4-[2-(3,3-dimethylbutyl)pyrimidin-4-yl]oxy-2,5-dimethylphenyl]-N-ethyl-N-methylmethanimidamide, (I.004) N'-[2,5-dimethyl-4-[2-[rac-(2R)-butan-2-yl]sulfanylpyrimidin-4-yl]oxyphenyl]-N-ethyl-N-methylmethanimidamide, (I.005) N'-[5-bromo-2-methyl-4-[2-(2,2,2-trifluoroethylsulfanyl)pyrimidin-4-yl]oxyphenyl]-N-ethyl-N-methylmethanimidamide, (I.006) N'-[2-chloro-5-methyl-4-[2-(2,2,2-trifluoroethylsulfanyl)pyrimidin-4-yl]oxyphenyl]-N-ethyl-N-methylmethanimidamide, (1.007) N'-[5-bromo-2-methyl-4-[2-(2-methylpropylsulfanyl)pyrimidin-4-yl]oxyphenyl]-N-ethyl-N-methylmethanimidamide, (I.008) N-ethyl-N'-[4-[2-(2-fluorophenyl)sulfanylpyrimidin-4-yl]oxy-2,5-dimethylphenyl]-N-methylmethanimidamide, (I.009) N-ethyl-N'-[4-[2-(3-fluorophenyl)sulfanylpyrimidin-4-yl]oxy-2,5-dimethylphenyl]-N-methylmethanimidamide, (I.010) N-ethyl-N'-[4-[2-(4-fluorophenyl)sulfanylpyrimidin-4-yl]oxy-2,5-dimethylphenyl]-N-methylmethanimidamide, (I.011) N'-[4-(2-cyclohexylsulfanylpyrimidin-4-yl)oxy-2,5-dimethylphenyl]-N-ethyl-N-methylmethanimidamide, (I.012) N'-[4-(2-cyclopentylsulfanylpyrimidin-4-yl)oxy-2,5-dimethylphenyl]-N-ethyl-N-methylmethanimidamide, (I.013) N'-[2,5-dimethyl-4-[2-(3,3,3-trifluoropropylsulfanyl)pyrimidin-4-yl]oxyphenyl]-N-ethyl-N-methylmethanimidamide, (I.014) N'-[5-bromo-2-methyl-4-(2-propan-2-ylsulfanylpyrimidin-4-yl)oxyphenyl]-N-ethyl-N-methylmethanimidamide, (I.015) N'-[5-bromo-2-methyl-4-(2-propylsulfanylpyrimidin-4-yl)oxyphenyl]-N-ethyl-N-methylmethanimidamide, (I.016) N'-[5-bromo-4-(2-ethylsulfanylpyrimidin-4-yl)oxy-2-methylphenyl]-N-ethyl-N-methylmethanimidamide, (I.017) N'-[5-bromo-2-methyl-4-[2-(trifluoromethyl)pyrimidin-4-yl]oxyphenyl]-N-ethyl-N-methylmethanimidamide, (I.018) N'-[5-bromo-4-(6-tert-butylsulfanyl-2-methylpyrimidin-4-yl)oxy-2-methylphenyl]-N-ethyl-N-methylmethanimidamide, (I.019) N-ethyl-N'-[4-(2-ethylsulfanyl-6-methylpyrimidin-4-yl)oxy-2,5 -dimethylphenyl] -N-methylmethanimidamide, (I.020) N'-[2,5-dimethyl-4-(6-methyl-2-propylsulfanylpyrimidin-4-yl)oxyphenyl]-N-ethyl-N-methylmethanimidamide, (I.021) N'-[2,5-dimethyl-4-[6-methyl-2-(2-methylpropylsulfanyl)pyrimidin-4-yl]oxyphenyl]-N-ethyl-N-methylmethanimidamide, (I.022) N'-[2,5-dimethyl-4-(6-methyl-2-propan-2-ylsulfanylpyrimidin-4-yl)oxyphenyl]-N-ethyl-N-methylmethanimidamide, (1.023) N'-[4-(6-tert-butylsulfanyl-2-methylpyrimidin-4-yl)oxy-2,5-dimethylphenyl]-N-ethyl-N-methylmethanimidamide, (I.024) N'-[4-(6-butan-2-ylsulfanyl-2-methylpyrimidin-4-yl)oxy-2,5-dimethylphenyl]-N-ethy1-N-methylmethanimidamide, (I.025) N'-[5-(difluoromethyl)-2-methyl-4-[2-(2-methylpropylsulfanyl)pyrimidin-4-yl]oxyphenyl]-N-ethyl-N-methylmethanimidamide, (I.026) N'-[2,5-dimethyl-4-[2-(2-methylpropylsulfanyl)pyrimidin-4-yl]oxyphenyl]-N-ethyl-N-methylmethanimidamide, (I.027) N-ethyl-N'-[4-(2-ethylsulfanylpyrimidin-4-yl)oxy-2,5-dimethylphenyl]-N-methylmethanimidamide, (I.028) N'-[2,5-dimethyl-4-(2-propan-2-ylsulfanylpyrimidin-4-yl)oxyphenyl]-N-ethyl-N-methylmethanimidamide, (I.029) N'-[2,5-dimethyl-4-(2-propylsulfanylpyrimidin-4-yl)oxyphenyl]-N-ethyl-N-methylmethanimidamide, (I.030) N'-[2,5-dimethyl-4-[2-(trifluoromethyl)pyrimidin-4-yl]oxyphenyl]-N-ethyl-N-methylmethanimidamide, (I.031) N'-[2,5-dimethyl-4-[2-(2,2,2-trifluoroethylsulfanyl)pyrimidin-4-yl]oxyphenyl]-N-ethyl-N-methylmethanimidamide, (I.032) N'-[4-(2-tert-butylpyrimidin-4-yl)oxy-2,5-dimethylphenyl]-N-ethyl-N-methylmethanimidamide, (I.033) N'-[2,5-dimethyl-4-(6-methyl-2-methylsulfanylpyrimidin-4-yl)oxyphenyl]-N-ethyl-N-methylmethanimidamide, (I.034) N'-[4-[2-[(E)-3,3-dimethylbut-1-enyl]pyrimidin-4-yl]oxy-2,5-dimethylphenyl]-N-ethyl-N-methylmethanimidamide, (I.035) N-ethyl-N-methyl-N'-[2-methyl-4-[2-(2-methylpropylsulfanyl)pyrimidin-4-yl]oxy-5-(trifluoromethyl)phenyl]methanimidamide, (I.036) N'-[5-(difluoromethyl)-2-methyl-4-(2-propylsulfanylpyrimidin-4-yl)oxyphenyl]-N-ethyl-N-methylmethanimidamide, (I.037) N'-[5-bromo-2-methyl-4-(2-phenylsulfanylpyrimidin-4-yl)oxyphenyl]-N-ethyl-N-methylmethanimidamide, (I.038) N'-[5-bromo-4-(2-tert-butylpyrimidin-4-yl)oxy-2-methylphenyl]-N-ethyl-N-methylmethanimidamide, (I.039) N'-[2,5-dimethyl-4-(2-phenylsulfanylpyrimidin-4-yl)oxyphenyl]-N-ethyl-N-methylmethanimidamide, (I.040) N'-[4-[2-(cyclopropylmethylsulfanyl)pyrimidin-4-yl]oxy-2,5-dimethylphenyl]-N-ethyl-N-methylmethanimidamide, (I.041) N'-[4-(2-cyclopentyloxypyrimidin-4-yl)oxy-2,5-dimethylphenyl]-N-ethyl-N-methylmethanimidamide, (I.042) N'-[4-(2-benzylsulfanylpyrimidin-4-yl)oxy-2,5-dimethylphenyl]-N-ethyl-N-methylmethanimidamide, (I.043) N'-[2,5-dimethyl-4-[2-(2-trimethylsilylethylsulfanyl)pyrimidin-4-yl]oxyphenyl]-N-ethyl-N-methylmethanimidamide, (I.044) N'-[4-(2-tert-butylpyrimidin-4-yl)oxy-5-cyano-2-methylphenyl]-N-ethyl-N-methylmethanimidamide, (I.045) N'-[4-(2-tert-butylsulfanylpyrimidin-4-yl)oxy-2-chloro-5-methylphenyl]-N-ethyl-N-methylmethanimidamide, (I.046) N'-[2-chloro-5-methyl-4-(2-propan-2-ylsulfanylpyrimidin-4-yl)oxyphenyl]-N-ethyl-N-methylmethanimidamide, (I.047) N'-[2,5-dimethyl-4-(2-pyridin-4-ylsulfanylpyrimidin-4-yl)oxyphenyl]-N-ethyl-N-methylmethanimidamide, (I.048) N'-[2-chloro-5-methyl-4-[2-(2-methylpropylsulfanyl)pyrimidin-4-yl]oxyphenyl]-N-ethyl-N-methylmethanimidamide, (I.049) N'-[5-cyano-2-methyl-4-[2-(2-methylpropylsulfanyl)pyrimidin-4-yl]oxyphenyl]-N-ethyl-N-methylmethanimidamide, (I.050) N'-[5-cyano-2-methyl-4-(2-propylsulfanylpyrimidin-4-yl)oxyphenyl]-N-ethyl-N-methylmethanimidamide, and (1.051) N'-[5-(difluoromethyl)-2-methyl-4-[5-methyl-2-(2-methylpropylsulfanyl)pyrimidin-4-yl]oxyphenyl]-N-ethyl-N-methylmethanimidamide.

7. Composition comprising a compound as claimed in one of the claims 1 to 6 and further comprising auxiliaries, solvents, carriers, surfactants or extenders.

8. The use of a compound as claimed in one of the claims 1 to 6 or of a composition according to claim 7 for controlling phytopathogenic fungi.

9. A method for controlling phytopathogenic fungi in crop protection, **characterized in that** a compound as claimed in one of the claims 1 to 6 or a composition according to claim 7 are applied to the phytopathogenic fungi and/or their habitat.

10. Seed coated with a compound as claimed in one of the claims 1 to 6 or a composition according to claim 7.

11. The use of a compound as claimed in one of the claims 1 to 6 or a composition according to claim 7 for treating seed.

12. The use of a compound as claimed in one of the claims 1 to 6 or a composition according to claim 7 for treating transgenic plants.

13. The use of a compound as claimed in one of the claims 1 to 6 or a composition according to claim 7 for treating seed of transgenic plants.

14. A method for protecting seed against phytopathogenic fungi by using seed comprising at least compound as claimed in one of the claims 1 to 6 or a composition according to claim 7.
